Europäisches Patentamt

European Patent Office

Office européen des brevets

(19)

(11) EP 1 071 442 B1

(12) EUROPEAN PATENT SPECIFICATION

(45) Date of publication and mention of the grant of the patent:
06.07.2005 Bulletin 2005/27

(51) Int Cl.7: A61K 38/00, C07K 5/00, C07K 7/00, C07K 16/00, C07K 17/00

(21) Application number: 99912802.8

(22) Date of filing: 22.03.1999

(86) International application number:
PCT/US1999/006230

(87) International publication number:
WO 1999/048513 (30.09.1999 Gazette 1999/39)

(54) **METHODS AND COMPOSITIONS FOR PEPTIDE SYNTHESIS (T-20)**

VERFAHREN UND ZUSAMMENSETZUNGEN ZUR PEPTIDSYNTHESE (T-20)

METHODES ET COMPOSITIONS DE SYNTHESE DE PEPTIDES (T-20)

(84) Designated Contracting States:
AT BE CH DE DK ES FR GB IE IT LI NL

(30) Priority: 23.03.1998 US 45920
01.05.1998 US 71877

(43) Date of publication of application:
31.01.2001 Bulletin 2001/05

(73) Proprietor: TRIMERIS INC.
Durham, NC 27707-3438 (US)

(72) Inventors:
• KANG, Myung-Chol
Chapel Hill, NC 27514 (US)
• BRAY, Brian
Graham, NC 27253 (US)
• LICHTY, Maynard
Durham, NC 27705 (US)
• MADER, Catherine
Durham, NC 27713 (US)
• MERUTKA, Gene
Hillsborough, NC 27278 (US)

(74) Representative: Horner, Martin Grenville et al
Marks & Clerk Scotland
19 Royal Exchange Square
Glasgow G1 3AE Scotland (GB)

(56) References cited:
WO-A-97/29126          WO-A1-94/28929
US-A- 5 464 933

• LAWLESS ET AL: "HIV-1 membrane fusion mechanism: Structural studies of the interactions between biologically-active peptides from gp41" BIOCHEMISTRY, AMERICAN CHEMICAL SOCIETY. EASTON, PA, US, vol. 35, no. 42, 22 October 1996 (1996-10-22), pages 13697-13708, XP002059093 ISSN: 0006-2960

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to a vector.

In particular, the present invention relates to a novel system for packaging and expressing genetic material in a retroviral particle.

More in particular, the present invention relates to a novel system capable of expressing a retroviral particle that is capable of delivering a nucleotide sequence of interest (hereinafter abbreviated as "NOI") - or even a plurality of NOIs - to one or more target sites.

In addition, the present invention relates to *inter alia* a novel retroviral vector useful in gene therapy.

Gene therapy may include any one or more of: the addition, the replacement, the deletion, the supplementation, the manipulation etc. of one or more nucleotide sequences in, for example, one or more targeted sites - such as targeted cells. If the targeted sites are targeted cells, then the cells may be part of a tissue or an organ. General teachings on gene therapy may be found in Molecular Biology (Ed Robert Meyers, Pub VCH, such as pages 556-558).

By way of further example, gene therapy can also provide a means by which any one or more of: a nucleotide sequence, such as a gene, can be applied to replace or supplement a defective gene; a pathogenic nucleotide sequence, such as a gene, or expression product thereof can be eliminated; a nucleotide sequence, such as a gene, or expression product thereof, can be added or introduced in order, for example, to create a more favourable phenotype; a nucleotide sequence, such as a gene, or expression product thereof can be added or introduced, for example, for selection purposes (i.e. to select transformed cells and the like over non-transformed cells); cells can be manipulated at the molecular level to treat, cure or prevent disease conditions - such as cancer (Schmidt-Wolf and Schmidt-Wolf, 1994, Annals of Hematology 69;273-279) or other disease conditions, such as immune, cardiovascular, neurological, inflammatory or infectious disorders; antigens can be manipulated and/or introduced to elicit an immune response, such as genetic vaccination.

In recent years, retroviruses have been proposed for use in gene therapy. Essentially, retroviruses are RNA viruses with a life cycle different to that of lytic viruses. In this regard, a retrovirus is an infectious entity that replicates through a DNA intermediate. When a retrovirus infects a cell, its genome is converted to a DNA form by a reverse transcriptase enzyme. The DNA copy serves as a template for the production of new RNA genomes and virally encoded proteins necessary for the assembly of infectious viral particles.

There are many retroviruses and examples include: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV).

A detailed list of retroviruses may be found in Coffin *et al* ("Retroviruses" 1997 Cold Spring Harbour Laboratory Press Eds: JM Coffin, SM Hughes, HE Varmus pp 758-763).

Details on the genomic structure of some retroviruses may be found in the art. By way of example, details on HIV may be found from the NCBI Genbank (i.e. Genome Accession No. AF033819).

Essentially, all wild type retroviruses contain three major coding domains, *gag, pol, env,* which code for essential virion proteins. Nevertheless, retroviruses may be broadly divided into two categories: namely, "simple" and "complex". These categories are distinguishable by the organisation of their genomes. Simple retroviruses usually carry only elementary information. In contrast, complex retroviruses also code for additional regulatory proteins derived from multiple spliced messages.

Retroviruses may even be further divided into seven groups. Five of these groups represent retroviruses with oncogenic potential. The remaining two groups are the lentiviruses and the spumaviruses. A review of these retroviruses is presented in "Retroviruses" (1997 Cold Spring Harbour Laboratory Press Eds: JM Coffin, SM Hughes, HE Varmus pp 1-25).

All oncogenic members except the human T-cell leukemia virus-bovine leukemia virus group (HTLV-BLV) are simple retroviruses. HTLV, BLV and the lentiviruses and spumaviruses are complex. Some of the best studied oncogenic retroviruses are Rous sarcoma virus (RSV), mouse mammary tumour virus (MMTV) and murine leukemia virus (MLV) and the human T-cell leukemia virus (HTLV).

The lentivirus group can be split even further into "primate" and "non-primate". Examples of primate lentiviruses include the human immunodeficiency virus (HIV), the causative agent of human auto-immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

A distinction between the lentivirus family and other types of retroviruses is that lentiviruses have the capability to infect both dividing and non-dividing cells (Lewis *et al* 1992 EMBO. J 11: 3053-3058; Lewis and Emerman 1994 J.

Virol. 68: 510-516). In contrast, other retroviruses - such as MLV - are unable to infect non-dividing cells such as those that make up, for example, muscle, brain, lung and liver tissue.

During the process of infection, a retrovirus initially attaches to a specific cell surface receptor. On entry into the susceptible host cell, the retroviral RNA genome is then copied to DNA by the virally encoded reverse transcriptase which is carried inside the parent virus. This DNA is transported to the host cell nucleus where it subsequently integrates into the host genome. At this stage, it is typically referred to as the provirus. The provirus is stable in the host chromosome during cell division and is transcribed like other cellular proteins. The provirus encodes the proteins and packaging machinery required to make more virus, which can leave the cell by a process sometimes called "budding".

As already indicated, each retroviral genome comprises genes called *gag, pol* and *env* which code for virion proteins and enzymes. In the provirus, these genes are flanked at both ends by regions called long terminal repeats (LTRs). The LTRs are responsible for proviral integration, and transcription. They also serve as enhancer-promoter sequences. In other words, the LTRs can control the expression of the viral gene. Encapsidation of the retroviral RNAs occurs by virtue of a *psi* sequence located at the 5' end of the viral genome.

The LTRs themselves are identical sequences that can be divided into three elements, which are called U3, R and U5. U3 is derived from the sequence unique to the 3' end of the RNA. R is derived from a sequence repeated at both ends of the RNA and U5 is derived from the sequence unique to the 5' end of the RNA. The sizes of the three elements can vary considerably among different retroviruses.

For ease of understanding, simple, generic structures (not to scale) of the RNA and the DNA forms of the retroviral genome are presented below in which the elementary features of the LTRs and the relative positioning of *gag, pol* and *env* are indicated.

**Virion RNA**

Reverse │ Transcriptase

**Provirus**

Transcription

**Transcript**

As shown in the diagram above, the basic molecular organisation of an infectious retroviral RNA genome is (5') R - U5 - *gag, pol, env* - U3-R (3'). In a defective retroviral vector genome *gag, pol* and *env* may be absent or not functional. The R regions at both ends of the RNA are repeated sequences. U5 and U3 represent unique sequences at the 5' and 3' ends of the RNA genome respectively.

Reverse transcription of the virion RNA into double stranded DNA takes place in the cytoplasm and involves two jumps of the reverse transcriptase from the 5' terminus to the 3' terminus of the template molecule. The result of these jumps is a duplication of sequences located at the 5' and 3' ends of the virion RNA. These sequences then occur fused in tandem on both ends of the viral DNA, forming the long terminal repeats (LTRs) which comprise R U5 and U3 regions. On completion of the reverse transcription, the viral DNA is translocated into the nucleus where the linear copy of the retroviral genome, called a preintegration complex (PIC), is randomly inserted into chromosomal DNA with the aid of the virion integrase to form a stable provirus. The number of possible sites of integration into the host cellular genome is very large and very widely distributed.

The control of proviral transcription remains largely with the noncoding sequences of the viral LTR. The site of transcription initiation is at the boundary between U3 and R in the left hand side LTR (as shown above) and the site of poly (A) addition (termination) is at the boundary between R and U5 in the right hand side LTR (as shown above). U3 contains most of the transcriptional control elements of the provirus, which include the promoter and multiple enhancer sequences responsive to cellular and in some cases, viral transcriptional activator proteins. Some retroviruses have any one or more of the following genes such as *tat, rev, tax* and *rex* that code for proteins that are involved in the regulation of gene expression.

Transcription of proviral DNA recreates the full length viral RNA genomic and subgenomic-sized RNA molecules that are generated by RNA processing. Typically, all RNA products serve as templates for the production of viral proteins. The expression of the RNA products is achieved by a combination of RNA transcript splicing and ribosomal framshifting during translation.

RNA splicing is the process by which intervening or "intronic" RNA sequences are removed and the remaining "exonic" sequences are ligated to provide continuous reading frames for translation. The primary transcript of retroviral DNA is modified in several ways and closely resembles a cellular mRNA. However, unlike most cellular mRNAs, in which all introns are efficiently spliced, newly synthesised retroviral RNA must be diverted into two populations. One population remains unspliced to serve as the genomic RNA and the other population is spliced to provide subgenomic RNA.

The full-length unspliced retroviral RNA transcripts serve two functions: (i) they encode the *gag* and *pol* gene products and (ii) they are packaged into progeny virion particles as genomic RNA. Sub-genomic-sized RNA molecules provide mRNA for the remainder of the viral gene products. All spliced retroviral transcripts bear the first exon, which spans the U5 region of the 5' LTR. The final exon always retains the U3 and R regions encoded by the 3' LTR although it varies in size. The composition of the remainder of the RNA structure depends on the number of splicing events and the choice of alternative splice sites.

In simple retroviruses, one population of newly synthesised retroviral RNA remains unspliced to serve as the genomic RNA and as mRNA for *gag* and *pol*. The other population is spliced, fusing the 5' portion of the genomic RNA to the downstream genes, most commonly *env*. Splicing is achieved with the use of a splice donor positioned upstream of *gag* and a splice acceptor near the 3' terminus of *pol*. The intron between the splice donor and splice acceptor that is removed by splicing contains the *gag* and *pol* genes. This splicing event creates the mRNA for envelope (Env) protein. Typically the splice donor is upstream of *gag* but in some viruses, such as ASLV, the splice donor is positioned a few codons into the *gag* gene resulting in a primary Env translation product that includes a few amino-terminal amino acid residues in common with Gag. The Env protein is synthesised on membrane-bound polyribosomes and transported by the cell's vesicular traffic to the plasma membrane, where it is incorporated into viral particles.

Complex retroviruses generate both singly and multiply spliced transcripts that encode not only the *env* gene products but also the sets of regulatory and accessory proteins unique to these viruses. Compex retroviruses such as the lentiviruses, and especially HIV, provide striking examples of the complexity of alternative splicing that can occur during retroviral infection. For example, it is now known that HIV-1 is capable of producing over 30 different mRNAs by sub-optimal splicing from primary genomic transcripts. This selection process appears to be regulated as mutations that disrupt competing splice acceptors can cause shifts in the splicing patterns and can affect viral infectivity (Purcell and Martin 1993 J Virol 67: 6365-6378).

The relative proportions of full-length RNA and subgenomic-sized RNAs vary in infected cells and modulation of the levels of these transcripts is a potential control step during retroviral gene expression. For retroviral gene expression, both unspliced and spliced RNAs must be transported to the cytoplasm and the proper ratio of spliced and unspliced RNA must be maintained for efficient retroviral gene expression. Different classes of retroviruses have evolved distinct solutions to this problem. The simple retroviruses, which use only full-length and singly spliced RNAs regulate the cytoplasmic ratios of these species either by the use of variably efficient splice sites or by the incorporation of several cis-acting elements, that have been only partially defined, into their genome. The complex retroviruses, which direct the synthesis of both singly and multiply spliced RNA, regulate the transport and possibly splicing of the different genomic and subgenomic-sized RNA species through the interaction of sequences on the RNA with the protein product of one of the accessory genes, such as *rev* in HIV-1 and *rex* in HTLV-1.

With regard to the structural genes *gag, pol* and *env* themselves and in slightly more detail, *gag* encodes the internal structural protein of the virus. Gag is proteolytically processed into the mature proteins MA (matrix), CA (capsid) and NC (nucleocapsid). The *pol* gene encodes the reverse transcriptase (RT), which contains both DNA polymerase, and associated RNase H activities and integrase (IN), which mediates replication of the genome. The *env* gene encodes the surface (SU) glycoprotein and the transmembrane (TM) protein of the virion, which form a complex that interacts specifically with cellular receptor proteins. This interaction leads ultimately to fusion of the viral membrane with the cell membrane.

The Env protein is a viral protein which coats the viral particle and plays an essential role in permitting viral entry into a target cell. The envelope glycoprotein complex of retroviruses includes two polypeptides: an external, glycosylated hydrophilic polypeptide (SU) and a membrane-spanning protein (TM). Together, these form an oligomeric "knob" or "knobbed spike" on the surface of a virion. Both polypeptides are encoded by the *env* gene and are synthesised in the form of a polyprotein precursor that is proteolytically cleaved during its transport to the cell surface. Although uncleaved Env proteins are able to bind to the receptor, the cleavage event itself is necessary to activate the fusion potential of the protein, which is necessary for entry of the virus into the host cell. Typically, both SU and TM proteins are glycosylated at multiple sites. However, in some viruses, exemplified by MLV, TM is not glycosylated.

Although the SU and TM proteins are not always required for the assembly of enveloped virion particles as such, they play an essential role in the entry process. In this regard, the SU domain binds to a receptor molecule, often a specific receptor molecule, on the target cell. It is believed that this binding event activates the membrane fusion-inducing potential of the TM protein after which the viral and cell membranes fuse. In some viruses, notably MLV, a cleavage event, resulting in the removal of a short portion of the cytoplasmic tail of TM, is thought to play a key role in uncovering the full fusion activity of the protein (Brody *et al* 1994 J Virol 68: 4620-4627; Rein *et al* 1994 J Virol 68: 1773-1781). This cytoplasmic "tail", distal to the membrane-spanning segment of TM remains on the internal side of the viral membrane and it varies considerably in length in different retroviruses. Thus, the specificity of the SU/receptor interaction can define the host range and tissue tropism of a retrovirus. In some cases, this specificity may restrict the transduction potential of a recombinant retroviral vector. Here, transduction includes a process of using a viral vector to deliver a non-viral gene to a target cell. For this reason, many gene therapy experiments have used MLV. A particular MLV that has an envelope protein called 4070A is known as an amphotropic virus, and this can also infect human cells because its envelope protein "docks" with a phosphate transport protein that is conserved between man and mouse. This transporter is ubiquitous and so these viruses are capable of infecting many cell types. In some cases however, it may be beneficial, especially from a safety point of view, to specifically target restricted cells. To this end, several groups have engineered a mouse ecotropic retrovirus, which unlike its amphotropic relative normally only infects mouse cells, to specifically infect particular human cells. Replacement of a fragment of an Env protein with an erythropoietin segement produced a recombinant retrovirus which then binds specifically to human cells that express the erythropoietin receptor on their surface, such as red blood cell precursors (Maulik and Patel 1997 "Molecular Biotechnology: Therapeutic Applications and Strategies" 1997 Wiley-Liss Inc. pp 45).

In addition to *gag, pol* and *env*, the complex retroviruses also contain "accessory" genes which code for accessory or auxillary proteins. Accessory or auxillary proteins are defined as those proteins encoded by the accessory genes in addition to those encoded by the usual replicative or structural genes, *gag, pol* and *env*. These accessory proteins are distinct from those involved in the regulation of gene expression, like those encoded by *tat, rev, tax* and *rex*. Examples of accessory genes include one or more of *vif, vpr, vpx, vpu* and *nef*. These accessory genes can be found in, for example, HIV (see, for example pages 802 and 803 of "Retroviruses" Ed. Coffin *et al* Pub. CSHL 1997). Non-essential accessory proteins may function in specialised cell types, providing functions that are at least in part duplicative of a function provided by a cellular protein. Typically, the accessory genes are located between *pol* and *env*, just downstream from *env* including the U3 region of the LTR or overlapping portions of the *env* and each other.

The complex retroviruses have evolved regulatory mechanisms that employ virally encoded transcriptional activators as well as cellular transcriptional factors. These *trans*acting viral proteins serve as activators of RNA transcription directed by the LTRs. The transcriptional *trans*-activators of the lentiviruses are encoded by the viral *tat* genes. Tat binds to a stable, stem-loop, RNA secondary structure, referred to as TAR, one function of which is to apparently optimally position Tat to trans-activate transcription.

As mentioned earlier, retroviruses have been proposed as a delivery system (otherwise expressed as a delivery vehicle or delivery vector) for *inter alia* the transfer of a NOI, or a plurality of NOIs, to one or more sites of interest. The transfer can occur *in vitro, ex vivo, in vivo,* or combinations thereof. When used in this fashion, the retroviruses are typically called retroviral vectors or recombinant retroviral vectors. Retroviral vectors have even been exploited to study various aspects of the retrovirus life cycle, including receptor usage, reverse transcription and RNA packaging (reviewed by Miller, 1992 Curr Top Microbiol Immunol 158:1-24).

In a typical recombinant retroviral vector for use in gene therapy, at least part of one or more of the *gag, pol* and *env* protein coding regions may be removed from the virus. This makes the retroviral vector replication-defective. The removed portions may even be replaced by a NOI in order to generate a virus capable of integrating its genome into a host genome but wherein the modified viral genome is unable to propagate itself due to a lack of structural proteins. When integrated in the host genome, expression of the NOI occurs - resulting in, for example, a therapeutic and/or a diagnostic effect. Thus, the transfer of a NOI into a site of interest is typically achieved by: integrating the NOI into the recombinant viral vector; packaging the modified viral vector into a virion coat; and allowing transduction of a site of interest - such as a targeted cell or a targeted cell population.

It is possible to propagate and isolate quantities of retroviral vectors (e.g. to prepare suitable titres of the retroviral vector) for subsequent transduction of, for example, a site of interest by using a combination of a packaging or helper cell line and a recombinant vector.

In some instances, propagation and isolation may entail isolation of the retroviral *gag, pol* and *env* genes and their separate introduction into a host cell to produce a "packaging cell line". The packaging cell line produces the proteins required for packaging retroviral DNA but it cannot bring about encapsidation due to the lack of a *psi* region. However, when a recombinant vector carrying a NOI and a *psi* region is introduced into the packaging cell line, the helper proteins can package the *psi*-positive recombinant vector to produce the recombinant virus stock. This can be used to transduce cells to introduce the NOI into the genome of the cells. The recombinant virus whose genome lacks all genes required to make viral proteins can tranduce only once and cannot propagate. These viral vectors which are only capable of a single round of transduction of target cells are known as replication defective vectors. Hence, the NOI is introduced into the host/target cell genome without the generation of potentially harmful retrovirus. A summary of the available packaging lines is presented in "Retroviruses" (1997 Cold Spring Harbour Laboratory Press Eds: JM Coffin, SM Hughes, HE Varmus pp 449).

The design of retroviral packaging cell lines has evolved to address the problem of *inter alia* the spontaneous production of helper virus that was frequently encountered with early designs. As recombination is greatly facilitated by homology, reducing or eliminating homology between the genomes of the vector and the helper has reduced the problem of helper virus production. More recently, packaging cells have been developed in which the *gag, pol* and *env* viral coding regions are carried on separate expression plasmids that are independently transfected into a packaging cell line so that three recombinant events are required for wild type viral production. This reduces the potential for production of a replication-competent virus. This strategy is sometimes referred to as the three plasmid transfection method (Soneoka *et al* 1995 Nucl. Acids Res. 23: 628-633).

Transient transfection can also be used to measure vector production when vectors are being developed. In this regard, transient transfection avoids the longer time required to generate stable vector-producing cell lines and is used if the vector or retroviral packaging components are toxic to cells. Components typically used to generate retroviral vectors include a plasmid encoding the Gag/Pol proteins, a plasmid encoding the Env protein and a plasmid containing a NOI. Vector production involves transient transfection of one or more of these components into cells containing the other required components. If the vector encodes toxic genes or genes that interfere with the replication of the host cell, such as inhibitors of the cell cycle or genes that induce apotosis, it may be difficult to generate stable vector-producing cell lines, but transient transfection can be used to produce the vector before the cells die. Also, cell lines have been developed using transient infection that produce vector titre levels that are comparable to the levels obtained from stable vector-producing cell lines (Pear *et al* 1993, Proc Natl Acad Sci 90:8392-8396).

In view of the toxicity of some HIV proteins - which can make it difficult to generate stable HIV-based packaging cells - HIV vectors are usually made by transient transfection of vector and helper virus. Some workers have even replaced the HIV Env protein with that of vesicular stomatis virus (VSV). Insertion of the Env protein of VSV facilitates vector concentration as HIV/VSV-G vectors with titres of $5 \times 10^5$ ($10^8$ after concentration) have been generated by transient transfection (Naldini *et al* 1996 Science 272: 263-267). Thus, transient transfection of HIV vectors may provide a useful strategy for the generation of high titre vectors (Yee *et al* 1994 PNAS. 91: 9564-9568).

With regard to vector titre, the practical uses of retroviral vectors have been limited largely by the titres of transducing particles which can be attained in *in vitro* culture (typically not more than $10^8$ particles/ml) and the sensitivity of many enveloped viruses to traditional biochemical and physicochemical techniques for concentrating and purifying viruses.

By way of example, several methods for concentration of retroviral vectors have been developed, including the use of centrifugation (Fekete and Cepko 1993 Mol Cell Biol 13: 2604-2613), hollow fibre filtration (Paul *et al* 1993 Hum Gene Ther 4: 609-615) and tangential flow filtration (Kotani *et al* 1994 Hum Gene Ther 5: 19-28). Although a 20-fold increase in viral titre can be achieved, the relative fragility of retroviral Env protein limits the ability to concentrate retroviral vectors and concentrating the virus usually results in a poor recovery of infectious virions. While this problem can be overcome by substitution of the retroviral Env protein with the more stable VSV-G protein, as described above, which allows for more effective vector concentration with better yields, it suffers from the drawback that the VSV-G protein is quite toxic to cells. Although helper-virus free vector titres of $10^7$ cfu/ml are obtainable with currently available vectors, experiments can often be done with much lower-titre vector stocks. However, for practical reasons, high-titre virus is desirable, especially when a large number of cells must be infected. In addition, high titres are a requirement for transduction of a large percentage of certain cell types. For example, the frequency of human hematopoietic progenitor cell infection is strongly dependent on vector titre, and useful frequencies of infection occur only with very high-titre stocks (Hock and Miller 1986 Nature 320: 275-277; Hogge and Humphries 1987 Blood 69: 611-617). In these cases, it is not sufficient simply to expose the cells to a larger volume of virus to compensate for a low virus titre. On the contrary, in some cases, the concentration of infectious vector virions may be critical to promote efficient transduction.

Workers are trying to create high titre vectors for use in gene delivery. By way of example, a comparison of different vector designs has proved useful in helping to define the essential elements required for high-titre viral production. Early work on different retroviral vector design showed that almost all of the internal protein-encoding regions of MLVs could be deleted without abolishing the infectivity of the vector (Miller *et al* 1983 Proc Natl Acad Sci 80: 4709-4713). These early vectors retained only a small portion of the 3' end of the *env*-coding region. Subsequent work has shown that all of the *env*-gene-coding sequences can be removed without further reduction in vector titre (Miller and Rosman 1989 Biotechnique 7: 980-990; Morgenstern and Land 1990 Nucleic Acids Res 18: 3587-3596). Only the viral LTRs and short regions adjoining the LTRs, including the segments needed for plus- and minus-strand DNA priming and a region required for selective packaging of viral RNA into virions (the *psi* site; Mann *et al* 1983 Cell 33: 153-159) were deemed necessary for vector transmission. Nevertheless, viral titres obtained with these early vectors were still about tenfold lower than the parental helper virus titre.

Additional experiments indicated that retention of sequences at the 5' end of the *gag* gene significantly raised viral vector titres and that this was due to an increase in the packaging efficiency of viral RNA into virions (Armentano *et al* 1987 J Virol 61: 1647-1650; Bender *et al* 1987 J Virol 61: 1639-1646; Adam and Miller 1988 J Virol 62: 3802-3806). This effect was not due to viral protein synthesis from the *gag* region of the vector because disruption of the *gag* reading frame or mutating the *gag* codon to a stop codon had no effect on vector titre (Bender *et al* 1987 *ibid*). These experiments demonstrated that the sequences required for efficient packaging of genomic RNA in MLV were larger than the *psi* signal previously defined by deletion analysis (Mann *et al* 1983 *ibid*). In order to obtain high titres ($10^6$ to > $10^7$), it was shown to be important that this larger signal, called *psi* plus, be included in retroviral vectors. It has now been demonstrated that this signal spans from upstream of the splice donor to downstream of the *gag* start codon (Bender *et al* 1987 *ibid*). Because of this position, in spliced *env* expressing transcripts this signal is deleted. This ensures that only full length transcripts containing all three essential genes for viral life cycle are packaged.

In addition to manipulating the retroviral vector with a view to increasing vector titre, retroviral vectors have also been designed to induce the production of a specific NOI (usually a marker protein) in transduced cells. As already mentioned, the most common retroviral vector design involves the replacement of retroviral sequences with one or more NOIs to create replication-defective vectors. The simplest approach has been to use the promoter in the retroviral 5' LTR to control the expresssion of a cDNA encoding an NOI or to alter the enhancer/promoter of the LTR to provide tissue-specific expression or inducibility. Alternatively, a single coding region has been expressed by using an internal promoter which permits more flexibility in promoter selection.

These strategies for expression of a gene of interest have been most easily implemented when the NOI is a selectable marker, as in the case of hypoxanthine-guanine phosphoribosyl transferase (*hprt*) (Miller *et al* 1983 Proc Natl Acad Sci 80: 4709-4713) which facilitates the selection of vector transduced cells. If the vector contains an NOI that is not a selectable marker, the vector can be introduced into packaging cells by co-transfection with a selectable marker present on a separate plasmid. This strategy has an appealing advantage for gene therapy in that a single protein is expressed in the ultimate target cells and possible toxicity or antigenicity of a selectable marker is avoided. However, when the inserted gene is not selectable, this approach has the disadvantage that it is more difficult to generate cells that produce a high titre vector stock. In addition it is usually more difficult to determine the titre of the vector.

The current methodologies used to design retroviral vectors that express two or more proteins have relied on three general strategies. These include: (i) the expression of different proteins from alternatively spliced mRNAs transcribed from one promoter; (ii) the use of the promoter in the 5' LTR and internal promoters to drive transcription of different cDNAs and (iii) the use of internal ribosomal entry site (IRES) elements to allow translation of multiple coding regions from either a single mRNA or from fusion proteins that can then be expressed from an open reading frame.

Vectors containing internal promoters have been widely used to express multiple genes. An internal promoter makes it possible to exploit the promoter/enhancer combinations other than the viral LTR for driving gene expression. Multiple internal promoters can be included in a retroviral vector and it has proved possible to express at least three different cDNAs each from its own promoter (Overell *et al* 1988 Mol Cell Biol 8: 1803-1808).

While there now exist many such modified retroviral vectors which may be used for the expression of NOIs in a variety of mammalian cells, most of these retroviral vectors are derived from simple retroviruses such as murine oncoretroviruses that are incapable of transducing non-dividing cells.

By way of example, a widely used vector that employs alternative splicing to express genes from the viral LTR SV(X) (Cepko *et al* 1984 Cell 37: 1053-1062) contains the neomycin phosphotransferase gene as a selectable marker. The model for this type of vector is the parental virus, MO-MLV, in which the Gag and Gag-Pol proteins are translated from the full-length viral mRNA and the Env protein is made from the spliced mRNA. One of the proteins encoded by the vector is translated from the full-length RNA whereas splicing that links the splice donor near the 5'LTR to a splice acceptor just upstream of the second gene produces an RNA from which the second gene product can be translated. One drawback of this strategy is that foreign sequences are inserted into the intron of the spliced gene. This can affect the ratio of spliced to unspliced RNAs or provide alternative splice acceptors that interfere with production of the spliced RNA encoding the second gene product (Korman *et al* 1987 Proc Natl Acad Sci 84: 2150-2154). Because these effects are unpredictable, they can affect the production of the encoded genes.
Other modified retroviral vectors can be divided into two classes with regards to splicing capabilities.

The first class of modified retroviral vector, typified by the pBABE vectors (Morgenstern *et al* 1990 Nucleic Acid Research 18: 3587-3596), contain mutations within the splice donor (GT to GC) that inhibit splicing of viral transcripts. Such splicing inhibition is beneficial for two reasons: Firstly, it ensures all viral transcripts contain a packaging signal and thus all can be packaged in the producer cell. Secondly, it prevents potential aberrant splicing between viral splice donors and possible cryptic splice acceptors of inserted genes.

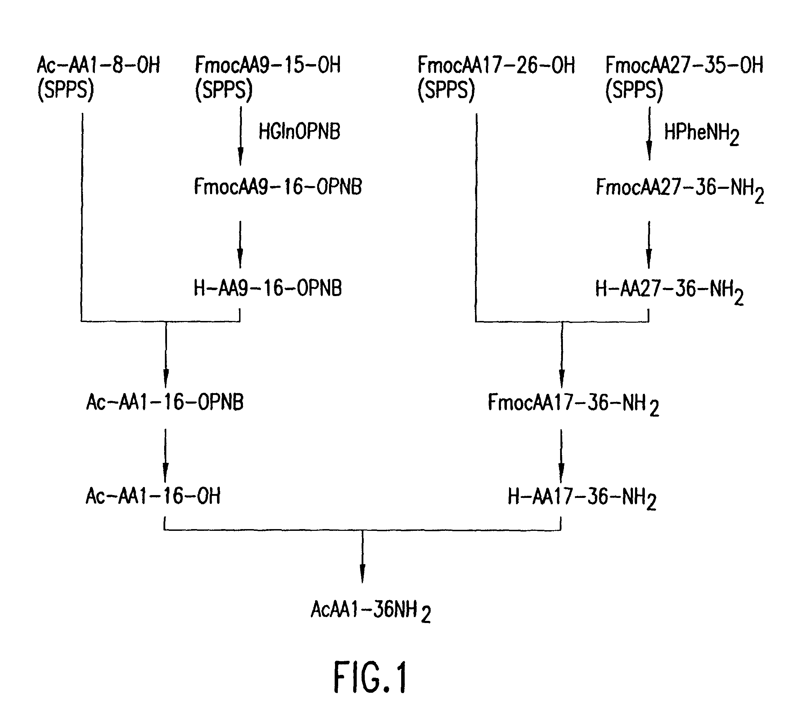

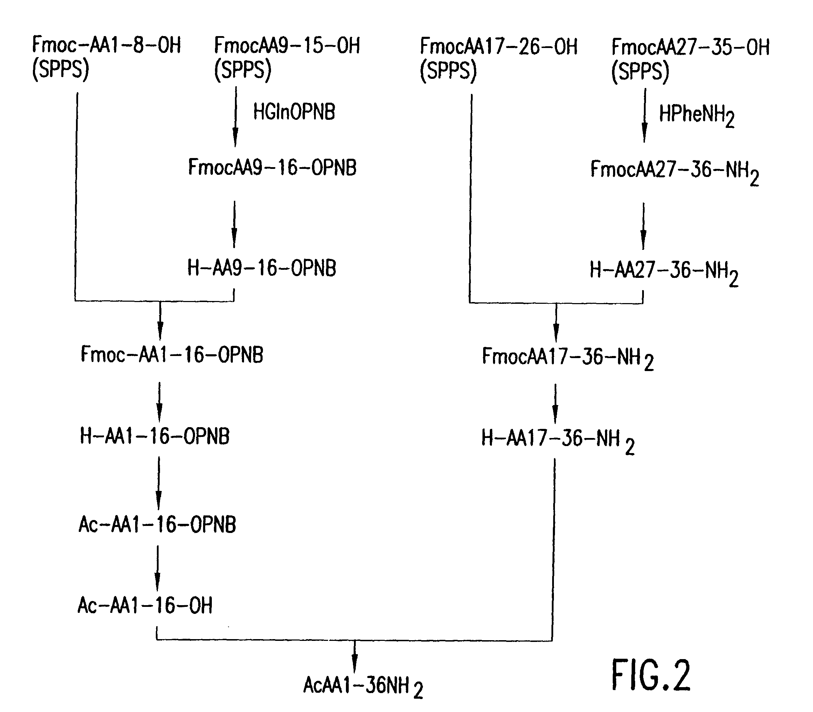

The second class of modified retroviral vector, typified by both N2 (Miller *et al* 1989 Biotechniques 7: 980-990) and the more recent MFG (Dranoff *et al* 1993 Proc Natl Acad Sci 19: 3979-3986), contain functional introns. Both of these vectors use the normal splice donor found within the packaging signal. However, their respective splice acceptors (SAs) differ. For N2, the SA is found within the "extended" packaging signal (Bender *er al* 1987 *ibid*). For MFG, the natural SA (found within *pol,* see Figure 1 thereof) is used. For both these vectors, it has been demonstrated that splicing greatly enhances gene expression in transduced cells (Miller *et al* 1989 *ibid;* Krall *et al* 1996 Gene Therapy 3: 37-48). Such observations support previous findings that, in general, splicing can enhance mRNA translation (Lee *et al* 1981 Nature 294: 228-232; Lewis *et al* 1986 Mol Cell Biol 6: 1998-2010; Chapman *et al* 1991 Nucleic Acids Res 19: 3979-3986). One likely reason for this is that the same machinery involved in transcript splicing may also aid in transcript export from the nucleus.

Unlike the modified retroviral vectors described above, there has been very little work on alternative splicing in the retroviral lentiviral systems which are capable of infecting non-dividing cells (Naldini *et al* 1996 Science 272: 263-267). To date the only published lentiviral vectors are those derived from HIV-1 (Kim *et al* 1997 J Virol 72: 811-816) and FIV (Poeschla *et al* 1998 Nat Med 4: 354-357). These vectors still contain virally derived splice donor and acceptor sequences (Naldini *et al* 1996 *ibid*).

Some alternative approaches to developing high titre vectors for gene delivery have included the use of: (i) defective viral vectors such as adenoviruses, adeno-associated virus (AAV), herpes viruses, and pox viruses and (ii) modified retroviral vector designs.

The adenovirus is a double-stranded, linear DNA virus that does not go through an RNA intermediate. There are over 50 different human serotypes of adenovirus divided into 6 subgroups based on the genetic sequence homology. The natural target of adenovirus is the respiratory and gastrointestinal epithelia, generally giving rise to only mild symptoms. Serotypes 2 and 5 (with 95% sequence homology) are most commonly used in adenoviral vector systems and are normally associated with upper respiratory tract infections in the young.

Adenoviruses are nonenveloped, regular icosohedrons. A typical adenovirus comprises a 140nm encapsidated DNA virus. The icosahedral symmetry of the virus is composed of 152 capsomeres: 240 hexons and 12 pentons. The core of the particle contains the 36kb linear duplex DNA which is covalently associated at the 5' ends with the Terminal Protein (TP) which acts as a primer for DNA replication. The DNA has inverted terminal repeats (ITR) and the length of these varies with the serotype.

Entry of adenovirus into cells involves a series of distinct events. Attachment of the virus to the cell occurs via an interaction between the viral fibre (37nm) and the fibre receptors on the cell. This receptor has recently been identified for Ad2/5 serotypes and designated as CAR (Coxsackie and Adeno Receptor, Tomko *et al* (1997 Proc Natl Acad Sci 94: 3352-2258). Internalisation of the virus into the endosome via the cellular $\alpha v\beta 3$ and $\alpha v\beta 5$ integrins is mediated by and viral RGD sequence in the penton-base capsid protein (Wickham *et al*., 1993 Cell 73: 309-319). Following internalisation, the endosome is disrupted by a process known as endosomolysis, an event which is believed to be preferentially promoted by the cellular $\alpha v\beta 5$ integrin (Wickham *et al*., 1994 J Cell Biol 127: 257-264). In addition, there is recent evidence that the Ad5 fibre knob binds with high affinity to the MHC class 1 $\alpha 2$ domain at the surface of certain cell types including human epithelial and B lymphoblast cells (Hong *et al*., 1997 EMBO 16: 2294-2306).

Subsequently the virus is translocated to the nucleus where activation of the early regions occurs and is shortly followed by DNA replication and activation of the late regions. Transcription, replication and packaging of the adenoviral DNA requires both host and viral functional protein machinery.

Viral gene expression can be divided into early (E) and late (L) phases. The late phase is defined by the onset of viral DNA replication. Adenovirus structural proteins are generally synthesised during the late phase. Following adenovirus infection, host cellular mRNA and protein synthesis is inhibited in cells infected with most serotypes. The adenovirus lytic cycle with adenovirus 2 and adenovirus 5 is very efficient and results in approximately 10, 000 vinous per infected cell along with the synthesis of excess viral protein and DNA that is not incorporated into the virion. Early adenovirus transcription is a complicated sequence of interrelated biochemical events but it entails essentially the synthesis of viral RNAs prior to the onset of DNA replication.

The Schematic diagram below is of the adenovirus genome showing the relative direction and position of early and late gene transcription:

The organisation of the adenovirus genome is similiar in all of the adenovirus groups and specific functions are generally positioned at identical locations for each serotype studied. Early cytoplasmic messenger RNAs are complementary to four defined, noncontiguous regions on the viral DNA. These regions are designated E1-E4. The early transcripts have been classified into an array of intermediate early (E1a), delayed early (E1b, E2a, E2b, E3 and E4), and intermediate regions.

The early genes are expressed about 6-8 hours after infection and are driven from 7 promoters in gene blocks E1-4.

The E1a region is involved in transcriptional transactivation of viral and cellular genes as well as transcriptional repression of other sequences. The E1a gene exerts an important control function on all of the other early adenovirus messenger RNAs. In normal tisssues, in order to transcribe regions E1b, E2a, E2b, E3 or E4 efficiently, active E1a product is required. However, the E1a function may be bypassed. Cells may be manipulated to provide E1a-like functions or may naturally contain such functions. The virus may also be manipulated to bypass the E1a function. The viral packaging signal overlaps with the E1a enhancer (194-358 nt).

The E1b region influences viral and cellular metabolism and host protein shut-off. It also includes the gene encoding the pIX protein (3525-4088 nt) which is required for packaging of the full length viral DNA and is important for the thermostability of the virus. The E1b region is required for the normal progression of viral events late in infection. The E1b product acts in the host nucleus. Mutants generated within the E1b sequences exhibit diminished late viral mRNA accumulation as well as impairment in the inhibition of host cellular transport normally observed late in adenovirus infection. E1b is required for altering functions of the host cell such that processing and transport are shifted in favour of viral late gene products. These products then result in viral packaging and release of virions. E1b produces a 19 kD protein that prevents apoptosis. E1b also produces a 55 kD protein that binds to p53. For a review on adenoviruses and their replication, see WO 96/17053.

The E2 region is essential as it encodes the 72 kDa DNA binding protein, DNA polymerase and the 80 kDa precurser of the 55 kDa Terminal Protein (TP) needed for protein priming to initiate DNA synthesis.

A 19 kDa protein (gp19K) is encoded within the E3 region and has been implicated in modulating the host immune response to the virus. Expression of this protein is upregulated in response to TNF alpha during the first phase of the infection and this then binds and prevents migration of the MHC class I antigens to the epithelial surface, thereby dampening the recognition of the adenoviral infected cells by the cytotoxic T lymphocytes. The E3 region is dispensible in *in vitro* studies and can be removed by deletion of a 1.9 kb *Xba*I fragment.

The E4 region is concerned with decreasing the host protein synthesis and increasing the DNA replication of the virus.

There are 5 families of late genes and all are initiated from the major late promoter. The expression of the late genes includes a very complex post-transcriptional control mechanism involving RNA splicing. The fibre protein is encoded within the L5 region. The adenoviral genome is flanked by the inverted terminal repeat which in Ad5 is 103 bp and is essential for DNA replication. 30-40 hours post infection viral production is complete.

Adenoviruses may be converted for use as vectors for gene transfer by deleting the E1 gene, which is important for the induction of the E2, E3 and E4 promoters. The E1-replication defective virus may be propagated in a cell line that provides the E1 polypeptides in trans, such as the human embryonic kidney cell line 293. A therapeutic gene or genes can be inserted by recombination in place of the E1 gene. Expression of the gene is driven from either the E1 promoter or a heterologous promoter.

Even more attenuated adenoviral vectors have been developed by deleting some or all of the E4 open reading frames (ORFs). However, certain second generation vectors appear not to give longer-term gene expression, even though the DNA seems to be maintained. Thus, it appears that the function of one or more of the E4 ORFs may be to enhance gene expression from at least certain viral promoters carried by the virus.

An alternative approach to making a more defective virus has been to "gut" the virus completely maintaining only the terminal repeats required for viral replication. The "gutted" or "gutless" viruses can be grown to high titres with a first generation helper virus in the 293 cell line but it has been difficult to separate the "gutted" vector from the helper virus.

Replication-competent adenoviruses can also be used for gene therapy. For example, the E1A gene can be inserted into a first generation virus under the regulation of a tumour-specific promoter. In thoery, following injection of the virus into a tumour, it could replicate specifically in the tumour but not in the surrounding normal cells. This type of vector could be used either to kill tumour cells directly by lysis or to deliver a "suicide gene" such as the herpes-simplex-virus thymidine-kinase gene (HSV *tk*) which can kill infected and bystander cells following treatment with ganciclovir. Alternatively, an adenovirus defective only for E1b has been used specifically for antitumour treatment in phase-1 clinical trials. The polypeptides encoded by E1b are able to block p53-mediated apoptosis, preventing the cell from killing itself in response to viral infection. Thus, in normal nontumour cells, in the absence of E1b, the virus is unable to block apoptosis and is thus unable to produce infectious virus and spread. In tumour cells deficient in p53, the E1b defective virus can grow and spread to adjacent p53-defective tumour cells but not to normal cells. Again, this type of vector could also be used to deliver a therapeutic gene such as HSV *tk*.

The adenovirus provides advantages as a vector for gene delivery over other gene therapy vector systems for the following reasons:

It is a double stranded DNA nonenveloped virus that is capable of *in vivo* and *in vitro* transduction of a broad range of cell types of human and non-human origin. These cells include respiratory airway epithelial cells, hepatocytes, muscle cells, cardiac myocytes, synoviocytes, primary mammary epithelial cess and post-mitotically terminally differentiated cells such as neurons (with perhaps the important exception of some lymphoid cells including monocytes). Adenoviral vectors are also capable of transducing non dividing cells. This is very important for diseases, such as cystic fibrosis, in which the affected cells in the lung epithelium, have a slow turnover rate. In fact, several trials are underway utilising adenovirus-mediated transfer of cystic fibrosis transporter (CFTR) into the lungs of afflicted adult cystic fibrosis patients.

Adenoviruses have been used as vectors for gene therapy and for expression of heterologous genes. The large (36 kilobase) genome can accommodate up to 8kb of foreign insert DNA and is able to replicate efficiently in complementing cell lines to produce very high titres of up to $10^{12}$. Adenovirus is thus one of the best systems to study the expression of genes in primary non-replicative cells.

The expression of viral or foreign genes from the adenovirus genome does not require a replicating cell. Adenoviral vectors enter cells by receptor mediated endocytosis. Once inside the cell, adenovirus vectors rarely integrate into the host chromosome. Instead, it functions episomally (independently from the host genome) as a linear genome in the host nucleus. Hence the use of recombinant adenovirus alleviates the problems associated with random integration into the host genome.

There is no association of human malignancy with adenovirus infection. Attenuated adenoviral strains have been developed and have been used in humans as live vaccines.

However, current adenoviral vectors suffer from some major limitations for *in vivo* therapeutic use. These include: (i) transient gene expression- the adenoviral vector generally remains episomal and does not replicate so that it is not passed onto subsequent progeny (ii) because of its inability to replicate, target cell proliferation can lead to dilution of the vector (iii) an immunological response raised against the adenoviral proteins so that cells expressing adenoviral proteins, even at a low level, are destroyed and (iv) an inability to achieve an effective therapeutic index since *in vivo* delivery leads to an uptake of the vector and expression of the delivered genes in only a proportion of target cells.

If the features of adenoviruses can be combined with the genetic stability of retro/lentiviruses then essentially the adenovirus can be used to transduce target cells to become transient retroviral producer cells that can stably infect neighbouring cells.

The present invention seeks to provide a novel retroviral vector.

In particular, the present invention seeks to provide a novel retroviral vector capable of providing efficient expression of a NOI - or even a plurality of NOIs - at one or more desired target sites.

The present invention also seeks to provide a novel system for preparing high titres of vector virion which incorporates safety features for *in vivo* use and which is capable of providing efficient expression of a NOI - or even a plurality of NOIs - at one or more desired target sites.

According to a first aspect of the present invention, there is provided a retroviral vector comprising a functional splice donor site and a functional splice acceptor site; wherein the functional splice donor site and the functional splice acceptor site flank a first nucleotide sequence of interest ("NOI"); wherein the functional splice donor site is upstream of the functional splice acceptor site; wherein the retroviral vector is derived from a retroviral pro-vector; wherein the retroviral pro-vector comprises a first nucleotide sequence (NS) capable of yielding the functional splice donor site and a second NS capable of yielding the functional splice acceptor site; wherein the first NS is downstream of the second NS; such that the retroviral vector is formed as a result of reverse transcription of the retroviral pro-vector.

According to a second aspect of the present invention, there is provided a retroviral vector wherein the retroviral pro-vector comprises a retroviral packaging signal; and wherein the second NS is located downstream of the retroviral packaging signal such that splicing is preventable at a primary target site.

According to a third aspect of the present invention, there is provided a retroviral vector wherein the second NS is placed downstream of the first NOI such that the first NOI is capable of being expressed at a primary target site.

According to a fourth aspect of the present invention, there is provided a retroviral vector wherein the second NS is placed upstream of a multiple cloning site such that one or more additional NOIs may be inserted.

According to a fifth aspect of the present invention, there is provided a retroviral vector wherein the second NS is a nucleotide sequence coding for an immunological molecule or a part thereof.

According to a sixth aspect of the present invention, there is provided a retroviral vector wherein the immunological molecule is an immunoglobulin.

According to a seventh aspect of the present invention, there is provided a retroviral vector wherein the second NS is a nucleotide sequence coding for an immunoglobulin heavy chain variable region.

According to an eighth aspect of the present invention, there is provided a retroviral vector wherein the vector additionally comprises a functional intron.

According to a ninth aspect of the present invention, there is provided a retroviral vector wherein the functional intron is positioned so that it is capable of restricting expression of at least one of the NOIs in a desired target site.

According to a tenth aspect of the present invention, there is provided a retroviral vector wherein the target site is a cell.

According to an eleventh aspect of the present invention, there is provided a retroviral vector wherein the vector or pro-vector is derivable from a murine oncoretrovirus or a lentivirus.

According to a twelfth aspect of the present invention, there is provided a retroviral vector wherein the vector is derivable from MMLV, MSV, MMTV, HIV-1 or EIAV.

According to a thirteenth aspect of the present invention, there is provided a retroviral vector wherein the retroviral vector is an integrated provirus.

According to a fourteenth aspect of the present invention, there is provided a retroviral particle obtainable from a retroviral vector.

According to a fifteenth aspect of the present invention, there is provided a cell transfected or transduced with a retroviral vector.

According to a sixteenth aspect of the present invention there is provided a retroviral vector or a viral particle or a cell for use in medicine.

According to a seventeenth aspect of the present invention there is provided a delivery system for a retroviral vector or a viral particle or a cell wherein the delivery system comprises one or more non-retroviral expression vector(s), adenovirus(es), or plasmid(s) or combinations thereof for delivery of an NOT or a plurality of NOIs to a first target cell and a retroviral vector for delivery of an NOT or a plurality of NOIs to a second target cell.

According to an eighteenth aspect of the present invention there is provided a retroviral pro-vector.

According to a nineteenth aspect of the present invention there is provided a hybrid viral vector system for *in vivo* gene delivery, which system comprises one or more primary viral vectors which encode a secondary viral vector, the primary vector or vectors capable of infecting a first target cell and of expressing therein the secondary viral vector, which secondary vector is capable of transducing a secondary target cell.

According to a twentieth aspect of the present invention there is provided a hybrid viral vector system wherein the primary vector is obtainable from or is based on an adenoviral vector and/or the secondary viral vector is obtainable from or is based on a retroviral vector preferably a lentiviral vector.

According to a twenty first aspect of the present invention there is provided a hybrid viral vector system wherein the lentiviral vector comprises or is capable of delivering a split-intron configuration.

According to a twenty second aspect of the present invention there is provided a lentiviral vector system wherein the lentiviral vector comprises or is capable of delivering a split-intron configuration.

According to a twenty third aspect of the present invention there is provided an adenoviral vector system wherein the adenoviral vector comprises or is capable of delivering a split-intron configuration.

According to a twenty fourth aspect of the present invention there are provided vectors or plasmids based on or obtained from any one or more of the entities presented as pE1sp1A, pCI-Neo, pE1RevE, pE1HORSE3.1, pE1PEGASUS4, pCI-Rab, pE1Rab.

According to a twenty fifth aspect of the present invention there is provided a retroviral vector capable of differential expression of NOIs in target cells.

Another aspect of the present invention includes a hybrid viral vector system for *in vivo* gene delivery, which system comprises a primary viral vector which encodes a secondary viral vector, the primary vector capable of infecting a first target cell and of expressing therein the secondary viral vector, which secondary vector is capable of transducing a secondary target cell, wherein the primary vector is obtainable from or is based on an adenoviral vector and the secondary viral vector is obtainable from or is based on a retroviral vector preferably a lentiviral vector.

Another aspect of the present invention includes a hybrid viral vector system for *in vivo* gene delivery, which system comprises a primary viral vector which encodes a secondary viral vector, the primary vector capable of infecting a first target cell and of expressing therein the secondary viral vector, which secondary vector is capable of transducing a secondary target cell, wherein the primary vector is obtainable from or is based on an adenoviral vector and the secondary viral vector is obtainable from or is based on a retroviral vector preferably a lentiviral vector; wherein the viral vector system comprises a functional splice donor site and a functional splice acceptor site; wherein the functional splice donor site and the functional splice acceptor site flank a first nucleotide sequence of interest ("NOI"); wherein the functional splice donor site is upstream of the functional splice acceptor site; wherein the retroviral vector is derived from a retroviral pro-vector; wherein the retroviral pro-vector comprises a first nucleotide sequence ("NS") capable of yielding the functional splice donor site and a second NS capable of yielding the functional splice acceptor site; wherein the first NS is downstream of the second NS; such that the retroviral vector is formed as a result of reverse transcription of the retroviral pro-vector.

Preferably the retroviral pro-vector comprises a third NS that is upstream of the second nucleotide sequence; wherein the third NS is capable of yielding a non-functional splice donor site.

Preferably the retroviral vector further comprises a second NOI; wherein the second NOI is downstream of the functional splice acceptor site.

Preferably the retroviral pro-vector comprises the second NOI; wherein the second NOI is downstream of the second nucleotide sequence.

Preferably the second NOI, or the expression product thereof, is or comprises a therapeutic agent or a diagnostic agent.

Preferably the first NOI, or the expression product thereof, is or comprises any one or more of an agent conferring selectablity (e.g. a marker element), a viral essential element, or a part thereof, or combinations thereof.

Preferably the first NS is at or near to the 3' end of a retroviral pro-vector; preferably wherein the 3' end comprises a U3 region and an R region; and preferably wherein the first NS is located between the U3 region and the R region.

Preferably the U3 region and/or the first NS of the retroviral pro-vector comprises an NS that is a third NOI; wherein the NOI is any one or more of a transcriptional control element, a coding sequence or a part thereof.

Preferably the first NS is obtainable from a virus.

Preferably the first NS is an intron or a part thereof.

Preferably the intron is obtainable from the small t-intron of SV40 virus.

Preferably the vector components are regulated. In one preferred aspect of the invention, the vector components are regulated by hypoxia.

In another preferred aspect of the invention, the vector components are regulated by tetracycline on/off system.

Thus, the present invention provides a delivery system which utilises a retroviral vector.

The retroviral vector of the delivery system of the present invention comprises a functional splice donor site ("FSDS") and a functional splice acceptor site ("FSAS") which flank a first NOI. The retroviral vector is formed as a result of reverse transcription of a retroviral pro-vector which may comprise a plurality of NOIs.

When the FSDS is positioned upstream of the FSAS, any intervening sequence(s) are capable of being spliced. Typically, splicing removes intervening or "intronic" RNA sequences and the remaining "exonic" sequences are ligated to provide continuous sequences for translation.

The splicing process can be pictorially represented as:

Unspliced Form

X           Y           Z

5'  ▬ ▬ ▬ ▬ FSDS - - - - - - - - - - - - FSAS ─ ─ ─ ─ ─ 3'─

Splicing ↓

Spliced Form

X           Z

5'  ▬ ▬ ▬ ▬ ─ ─ ─ ─ ─ ─ ─ 3'─

In this pictorial representation, Y represents the intervening sequence that is removed as a result of splicing.

The natural splicing configuration for retroviral vectors is shown in Figure 27a. The splicing configuration of known vectors is shown in Figure 27b. The Splicing configuration according to the present invention is shown in Figure 27c.

In accordance with the present invention, if the FSDS is downstream of the FSAS, then splicing cannot occur.

Likewise, if the FSDS is a non-functional splice donor site (NFSDS) and/or the FSAS is a non-functional acceptor acceptor site (NFAS), then splicing cannot occur.

An example of a NFSDS is a mutated FSDS such that the FSDS can no longer be recognised by the splicing mechanism.

In accordance with the present invention, each NS can be any suitable nucleotide sequence. For example, each sequence can be independently DNA or RNA - which may be synthetically prepared or may be prepared by use of recombinant DNA techniques or may be isolated from natural sources or may be combinations thereof. The sequence may be a sense sequence or an antisense sequence. There may be a plurality of sequences, which may be directly or indirectly joined to each other, or combinations thereof.

In accordance with the present invention, each NOI can be any suitable nucleotide sequence. For example, each sequence can be independently DNA or RNA - which may be synthetically prepared or may be prepared by use of recombinant DNA techniques or may be isolated from natural sources or may be combinations thereof. The sequence may be a sense sequence or an antisense sequence. There may be a plurality of sequences, which may be directly or indirectly joined to each other, or combinations thereof.

The first NOI may include any one or more of the following selectable markers which have been successfully in retroviral vectors: the bacterial neomycin and hygromycin phosphotransferase genes which confer resistance to G418 and hygromycin respectively (Palmer *et al* 1987 Proc Natl Acad Sci 84: 1055-1059; Yang *et al* 1987 Mol Cell Biol 7: 3923-3928); a mutant mouse dihydrofolate reductase gene (*dhfr*) which confers resistance to methotrexate (Miller *et al* 1985 Mol Cell Biol 5: 431-437); the bacterial gpt gene which allows cells to grow in medium containing mycophenolic acid, xanthine and aminopterin (Mann *et al* 1983 Cell 33: 153-159); the bacterial hisD gene which allows cells to grow in medium without histidine but containing histidinol (Danos and Mulligan 1988 Proc Natl Acad Sci 85: 6460-6464); the multidrug resistance gene (*mdr*) which confers resistance to a variety of drugs (Guild *et al* 1988 Proc Natl Acad Sci 85: 1595-1599; Pastan *et al* 1988 Proc Natl Acad Sci 85: 4486-4490) and the bacterial genes which confer resistance to puromycin or phleomycin (Morgenstern and Land 1990 Nucleic Acid Res 18: 3587-3596).

All of these markers are dominant selectable markers and allow chemical selection of most cells expressing these genes. β-galactosidase can also be considered a dominant marker; cells expressing β-galactosidase can be selected by using the fluorescence-activated cell sorter. In fact, any cell surface protein can provide a selectable marker for cells not already making the protein. Cells expressing the protein can be selected by using a fluorescent antibody to the protein and a cell sorter. Other selectable markers that have been included in vectors include the *hprt* and HSV thymidine kinase which allows cells to grow in medium containing hypoxanthine, amethopterin and thymidine.

The first NOI could contain non-coding sequences, for example the retroviral packaging site or non-sense sequences that render the second NOI non-functional in the pro-vector but when they are removed by the splicing the second NOI is revealed for functional expression.

The first NOI may also encode a viral essential element such as *env* encoding the Env protein which can reduce the complexity of production systems. By way of example, in an adenoviral vector, this allows the retroviral vector genome and the envelope to be configured in a single adenoviral vector under the same promoter control thus providing a simpler system and leaving more capacity in the adenoviral vector for additional sequences. In one aspect, those additional sequences could be the gag-pol cassette itself. Thus in one adenoviral vector one can produce a retroviral vector particle. Previous studies (Feng et al 1997 Nature Biotechnology 15: 866) have required the use of multiple adenoviral vectors.

If the retroviral component includes an *env* nucleotide sequence, then all or part of that sequence can be optionally replaced with all or part of another *env* nucleotide sequence such as, by way of example, the amphotropic Env protein designated 4070A or the influenza haemagglutinin (HA) or the vesicular stomatitis virus G (VSV-G) protein. Replacement of the *env* gene with a heterologous *env* gene is an example of a technique or strategy called pseudotyping. Pseudotyping is not a new phenomenon and examples may be found in WO-A-98/05759, WO-A-98/05754, WO-A-97/17457, WO-A-96/09400, WO-A-91/00047 and Mebatsion *et al* 1997 Cell 90, 841-847.

In one preferred aspect, the retroviral vector of the present invention has been pseudotyped. In this regard, pseudotyping can confer one or more advantages. For example, with the lentiviral vectors, the *env* gene product of the HTV based vectors would restrict these vectors to infecting only cells that express a protein called CD4. But if the *env* gene in these vectors has been substituted with *env* sequences from other RNA viruses, then they may have a broader infectious spectrum (Verma and Somia 1997 Nature 389:239-242). By way of example, workers have pseudotyped an HIV based vector with the glycoprotein from VSV (Verma and Somia 1997 *ibid*).

In another alternative, the Env protein may be a modified Env protein such as a mutant or engineered Env protein. Modifications may be made or selected to introduce targeting ability or to reduce toxicity or for another purpose (Valsesia-Wittman *et al* 1996 J Viral 70: 2056-64; Nilson *et al* 1996 Gene Therapy 3: 280-6; Fielding *et al* 1998 Blood 9: 1802 and references cited therein).

Suitable second NOI coding sequences include those that are of therapeutic and/or diagnostic application such as, but not limited to: sequences encoding cytokines, chemokines, hormones, antibodies, engineered immunoglobulin-like molecules, a single chain antibody, fusion proteins, enzymes, immune co-stimulatory molecules, immunomodulatory molecules, anti-sense RNA, a transdominant negative mutant of a target protein, a toxin, a conditional toxin, an antigen, a tumour suppressor protein and growth factors, membrane proteins, vasoactive proteins and peptides, anti-viral proteins and ribozymes, and derivatives therof (such as with an associated reporter group). When included, such coding sequences may be typically operatively linked to a suitable promoter, which may be a promoter driving expression of a ribozyme(s), or a different promoter or promoters.

The second NOI coding sequence may encode a fusion protein or a segment of a coding sequence.

The retroviral vector of the present invention may be used to deliver a second NOI such as a pro-drug activating enzyme to a tumour site for the treatment of a cancer. In each case, a suitable pro-drug is used in the treatment of the individual (such as a patient) in combination with the appropriate pro-drug activating enzyme. An appropriate pro-drug is administered in conjunction with the vector. Examples of pro-drugs include: etoposide phosphate (with alkaline phosphatase, Senter *et al* 1988 Proc Natl Acad Sci 85: 4842-4846); 5-fluorocytosine (with cytosine deaminase, Mullen *et al* 1994 Cancer Res 54: 1503-1506); Doxorubicin-N-p-hydroxyphenoxyacetamide (with Penicillin-V-Amidase, Kerr *et al* 1990 Cancer Immunol Immunother 31: 202-206); Para-N-bis(2-chloroethyl) aminobenzoyl glutamate (with carboxypeptidase G2); Cephalosporin nitrogen mustard carbamates (with β -lactamase); SR4233 (with P450 Reducase); Ganciclovir (with HSV thymidine kinase, Borrelli *et al* 1988 Proc Natl Acad Sci 85: 7572-7576); mustard pro-drugs with nitroreductase (Friedlos *et al* 1997 J Med Chem 40: 1270-1275) and Cyclophosphamide (with P450 Chen *et al* 1996 Cancer Res 56: 1331-1340).

The vector of the present invention may be a delivered to a target site by a viral or a non-viral vector.

As it is well known in the art, a vector is a tool that allows or faciliates the transfer of an entity from one environment to another. By way of example, some vectors used in recombinant DNA techniques allow entities, such as a segment of DNA (such as a heterologous DNA segment), to be transferred into a target cell. Optionally, once within the target cell, the vector may then serve to maintain the heterologous DNA within the cell or may act as a unit of DNA replication. Examples of vectors used in recombinant DNA techniques include plasmids, chromosomes, artificial chromosomes or viruses.

Non-viral delivery systems include but are not limted to DNA transfection methods. Here, transfection includes a process using a non-viral vector to deliver a gene to a target mammalian cell.

Typical transfection methods include electroporation, DNA biolistics, lipid-mediated transfection, compacted DNA-mediated transfection, liposomes, immunoliposomes, lipofectin, cationic agent-mediated, cationic facial amphiphiles (CFAs) (Nature Biotechnology 1996 14; 556), and combinations thereof.

Viral delivery systems include but are not limited to adenovirus vector, an adeno-associated viral (AAV) vector, a herpes viral vector, retroviral vector, lentiviral vector, baculoviral vector. Other examples of vectors include *ex vivo* delivery systems, which include but are not limited to DNA transfection methods such as electroporation, DNA biolistics, lipid-mediated transfection, compacted DNA-mediated transfection.

The vector delivery system of the present invention may consist of a primary vector manufactured *in vitro* which encodes the genes necessary to produce a secondary vector *in vivo.*

The primary viral vector or vectors may be a variety of different viral vectors, such as retroviral, adenoviral, herpes virus or pox virus vectors, or in the case of multiple primary viral vectors, they may be a mixture of vectors of different viral origin. In whichever case, the primary viral vectors are preferably defective in that they are incapable of independent replication. Thus, they are capable of entering a target cell and delivering the secondary vector sequences, but not of replicating so as to go on to infect further target cells.

In the case where the hybrid viral vector system comprises more than one primary vector to encode the secondary vector, both or all three primary vectors will be used to transfect or transduce a primary target cell population, usually simultaneously.

Preferably, there is a single primary viral vector which encodes all components of the secondary viral vector.

The preferred single or multiple primary viral vectors are adenoviral vectors.

Adenoviral vectors for use in the invention may be derived from a human adenovirus or an adenovirus which does not normally infect humans. Preferably the vectors are derived from adenovirus type 2 or adenovirus type 5 (Ad2 or Ad5) or a mouse adenovirus or an avian adenovirus such as CELO virus (Cotton *et al* 1993 J Viral 67:3777-3785). The vectors may be replication competent adenoviral vectors but are more preferably defective adenoviral vectors. Adenoviral vectors may be rendered defective by deletion of one or more components necessary for replication of the virus. Typically, each adenoviral vector contains at least a deletion in the E1 region. For production of infectious adenoviral vector particles, this deletion may be complemented by passage of the virus in a human embryo fibroblast cell line such as human 293 cell line, containing an integrated copy of the left portion of Ad5, including the E1 gene. The capacity for insertion of heterologous DNA into such vectors can be up to approximately 7kb. Thus such vectors are useful for construction of a system according to the invention comprising three separate recombinant vectors each containing one of the essential transcription units for construction of the retroviral secondary vector.

Alternative adenoviral vectors are known in the art which contain further deletions in other adenoviral genes and these vectors are also suitable for use in the invention. Several of these second generation adenoviral vectors show reduced immunogenicity (eg E1 + E2 deletions Gorziglia *et al* 1996 J Virol 70: 4173-4178; E1 + E4 deletions Yeh *et al* 1996 J Virol 70: 559-565). Extended deletions serve to provide additional cloning capacity for the introduction of multiple genes in the vector. For example a 25 kb deletion has been described (Lieber *et al* 1996 J Virol 70: 8944-8960) and a cloning vector deleted of all viral genes has been reported (Fisher *et al* 1996 Virolology 217: 11-22) which permit the introduction of more than 35 kb of heterologous DNA. Such vectors may be used to generate an adenoviral primary vector according to the invention encoding two or three transcription units for construction of the retroviral secondary vector.

The secondary viral vector is preferably a retroviral vector. The secondary vector is produced by expression of essential genes for assembly and packaging of a defective viral vector particle, within the primary target cells. It is defective in that it is incapable of independent replication. Thus, once the secondary retroviral vector has transduced a secondary target cell, it is incapable of spreading by replication to any further target cells.

The term "retroviral vector" typically includes a retroviral nucleic acid which is capable of infection, but which is not capable, by itself, of replication. Thus it is replication defective. A retroviral vector typically comprises one or more NOI(s), preferably of non-retroviral origin, for delivery to target cells. A retroviral vector may also comprise a functional splice donor site (FSDS) and a functional splice acceptor site (FSAS) so that when the FSDS is upstream of the FSAS, any intervening sequence(s) are capable of being spliced. A retroviral vector may comprise further non-retroviral sequences, such as non-retroviral control sequences in the U3 region which may influence expression of an NOI(s) once the retroviral vector is integrated as a provirus into a target cell. The retroviral vector need not contain elements from only a single retrovirus. Thus, in accordance with the present invention, it is possible to have elements derivable from two of more different retroviruses or other sources

The term "retroviral pro-vector" typically includes a retroviral vector genome as described above but which comprises a first nucleotide sequence (NS) capable of yielding a functional splice donor site (FSDs) and a second NS capable of yielding a functional splice acceptor site (FSAS) such that the first NS is downstream of the second NS so that splicing associated with the first NS and the second NS cannot occur. Upon reverse transcription of the retroviral pro-vector, a retroviral vector is formed.

The term "retroviral vector particle" refers to the packaged retroviral vector, that is preferably capable of binding to and entering target cells. The components of the particle, as already discussed for the vector, may be modified with respect to the wild type retrovirus. For example, the Env proteins in the proteinaceous coat of the particle may be genetically modified in order to alter their targeting specificity or achieve some other desired function.

The retroviral vector of this aspect of the invention may be derivable from a murine oncoretrovirus such as MMLV, MSV or MMTV; or may be derivable from a lentivirus such as HIV-1, EIAV; or may be derivable from another retrovirus.

The retroviral vector of the invention can be modified to render the natural splice donor site of the retrovirus non-functional.

The term "modification" includes the silencing or removal of the natural splice donor. Vectors, such as MLV based vectors, which have the splice donor site removed are known in the art. An example of such a vector is pBABE (Morgenstern *et al* 1990 *ibid*).

The secondary vector may be produced from expression of essential genes for retroviral vector production encoded in the DNA of the primary vector. Such genes may include a *gag-pol* gene from a retrovirus, an *env* gene from an enveloped virus and a defective retroviral vector containing one or more therapeutic or diagnostic NOI(s). The defective retroviral vector contains in general terms sequences to enable reverse transcription, at least part of a 5' long terminal repeat (LTR), at least part of a 3'LTR and a packaging signal.

If it is desired to render the secondary vector replication defective, that secondary vector may be encoded by a plurality of transcription units, which may be located in a single or in two or more adenoviral or other primary vectors. Thus, there may be a transcription unit encoding the secondary vector genome, a transcription unit encoding *gag-pol* and a transcription unit encoding *env*. Alternatively, two or more of these may be combined. For example, nucleic acid sequences encoding *gag-pol* and *env,* or *env* and the genome, may be combined in a single transcription unit. Ways of achieving this are known in the art.

Transcription units as described herein are regions of nucleic acid containing coding sequences and the signals for achieving expression of those coding sequences independently of any other coding sequences. Thus, each transcription unit generally comprises at least a promoter, an enhancer and a polyadenylation signal.

The term "promoter" is used in the normal sense of the art, e.g. an RNA polymerase binding site in the Jacob-Monod theory of gene expression.

The term "enhancer" includes a DNA sequence which binds to other protein components of the transcription initiation complex and thus facilitates the initiation of transcription directed by its associated promoter.

The promoter and enhancer of the transcription units encoding the secondary vector are preferably strongly active, or capable of being strongly induced, in the primary target cells under conditions for production of the secondary viral vector. The promoter and/or enhancer may be constitutively efficient, or may be tissue or temporally restricted in their activity. Examples of suitable tissue restricted promoters/enhancers are those which are highly active in tumour cells such as a promoter/enhancer from a MUC1 gene, a CEA gene or a 5T4 antigen gene. Examples of temporally restricted promoters/enhancers are those which are responsive to ischaemia and/or hypoxia, such as hypoxia response elements or the promoter/enhancer of a grp78 or a grp94 gene. One preferred promoter-enhancer combination is a human cytomegalovirus (hCMV) major immediate early (MIE) promoter/enhancer combination.

Other preferred additional components include entities enabling efficient expression of an NOI or a plurality of NOIs.

In one preferred aspect of the present invention, there is hypoxia or ischaemia regulatable expression of the secondary vector components. In this regard, hypoxia is a powerful regulator of gene expression in a wide range of different cell types and acts by the induction of the activity of hypoxia-inducible transcription factors such as hypoxia inducible factor-1 (HIF-1; Wang & Semenza 1993 Proc Natl Acad Sci 90:430), which bind to cognate DNA recognition sites, the hypoxia-responsive elements (HREs) on various gene promoters. Dachs *et al* (1997 Nature Med 5: 515) have used a multimeric form of the HRE from the mouse phosphoglycerate kinase-1 (PGK-1) gene (Firth *et al* 1994 Proc Natl Acad Sci 91:6496-6500) to control expression of both marker and therapeutic genes by human fibrosarcoma cells in response to hypoxia *in vitro* and within solid tumours *in vivo* (Dachs *et al ibid*). Alternatively, the fact that marked glucose deprivation is also present in ischaemic areas of tumours can be used to activate heterologous gene expression specifically in tumours. A truncated 632 base pair sequence of the grp 78 gene promoter, known to be activated specifically by glucose deprivation, has also been shown to be capable of driving high level expression of a reporter gene in murine tumours *in vivo* (Gazit *et al* 1995 Cancer Res 55:1660).

**Notes:**

1 In process control, HPLC
Phenomenex Jupiter, C5, 5μ, 300A
0.75 mL/min, 260 nm
A $H_2O$/0.05% TFA
B 50% IPA/MeOH/0.05% TFA
80-100%B over 10 min, 100%B for 5 min.
Retention time: HAA9-16OPNB, 7.2 minutes, FmocAA1-8OH, 7.9 minutes
Retention time: FmocAA1-16OPNB, 14.5 minutes

**8.5 Preparation of Fragment H-AA1-16OPNB**

**Structure:**

H-Tyr(tBu)-Thr(tBu)-Ser(tBu)-Leu-Ile-His(trt)-Ser(tBu)-Leu-
Ile-Glu(tBu)-Glu(tBu)-Ser(tBu)-Gln(trt)-Asn(trt)-Gln(trt)-
Gln-OPNB (SEQ ID NO:4)

| $C_{192}H_{243}N_{23}O_{32}$ MW 3384.41 | | | | | |
|---|---|---|---|---|---|
| **Materials:** | MW | eq | mmoles | grams | mL |
| FmocAA1-16OPNB | 3607.49 | 1 | 0.28 | 1.0 | -- |
| piperidine | -- | -- | -- | -- | 0.6 |
| dichloromethane | -- | -- | -- | -- | 11.4 |
| hexane | -- | -- | -- | -- | 45 |
| **Theoretical Yield:** 0.94 g | **Expected Yield:** 90-105% | | | | |

**Procedure:**

A 50 mL round bottom flask containing a magnetic stir bar was charged with FmocAA1-16OPNB (as synthesized in Section 8.4, above), dichloromethane (11.4 mL) and piperidine (0.6 mL). The solution was stirred at room temperature under an atmosphere of nitrogen for 90 minutes (note 1). Hexane (45 mL) was added to the reaction mixture and the solvent volume was reduced to 25 mL by vacuum distillation. The resulting solids were collected by vacuum filtration and dried to give 0.96g of HAA1-16OPNB in 102% yield. HPLC analysis of the solid indicated 72A% HAA1-16OPNB and 18A% fulvene and piperidine-fulvene adduct.

**Notes:**

1 Phenomenex Jupiter, C5, 5μ, 300A
0.8 mL/min, 260 nm
A $H_2O$/0-05% TFA
B 50% IPA/MeOH/0.05% TFA
80-100%B over 10 min, 100%B for 5 min

Retention time FmocAA1-16OPNB, 14.1 min
Retention time HAA1-16OPNB, 11.6 min
Retention time fulvene and piperdine-fulvene adduct, 5.5 and 4.8 min.

## 8.6 Preparation of Fragment Ac-AA1-16OPNB by Acetylation of N-terminus of HAA1-16OPNB

**Structure:**

Ac-Tyr(tBu)-Thr(tBu)-Ser(tBu)-Leu-Ile-His(trt)-Ser(tBu)-Leu-
Ile-Glu(tBu)-Glu(tBu)-Ser(tBu)-Gln(trt)-Asn(trt)-Gln(trt)-
Gln-OPNB (SEQ ID NO:4)

| $C_{194}H_{246}N_{23}O_{34}$ MW 3427.43 | | | | | |
|---|---|---|---|---|---|
| **Materials:** | MW | eq | mmoles | grams | mL |
| HAA1-16OPNB | 3384.41 | 1 | 0.28 | 0.95 | -- |
| acetic anhydride (d=1.08) | 102.09 | 3 | 0.84 | 0.086 | 0.080 |
| pyridine (d=0.978) | 79.1 | 3 | 0.84 | 0.67 | 0.068 |
| DMF | -- | -- | -- | -- | 10 |
| water | -- | -- | -- | -- | 30 |
| MTBE | -- | -- | -- | -- | 10 |
| hexane | -- | -- | -- | -- | 10 |
| **Theoretical Yield**: 0.96 g | **Expected Yield:** 80-100% | | | | |

**Procedure:**

A 50 mL round bottom flask containing a magnetic stir bar was charged with HAA1-16OPNB (as synthesized in Section 8.5, above), DMF (10 mL), acetic anhydride and pyridine. The reaction mixture was stirred at room temperature under an atmosphere of nitrogen for 60 min (note 1). Water (20 mL) was added to precipitate the peptide. The solids were collected by vacuum filtration, washed with water (10 mL) and dried to give 0.87g of AcAA1-16OPNB. To remove residual fulvene and piperidine-fulvene adduct, the solid was triturated with 1:1 MTBE/hexane (20 mL) for 4.5 hours at room temperature. The solids were collected by vacuum filtration and dried to give 0.82 g of AcAA1-16OPNB in 85% yield and over 90A% purity by HPLC (note 1).

**Notes:**

1 In process control, HPLC
Phenomenex Jupiter, C5, 5μ, 300A
0.8 mL/min, 260 nm
A H$_2$O/0.05% TFA
B 50% IPA/MeOH/0.05% TFA
80-100%B over 10 min, 100%B for 15 min
Retention time HAA1-16OPNB, 11.6 min
Retention time AcAA1-16OPNB, 12.1 min
TLC, 10% ethanol in dichloromethane
UV, Iodine detection
Rf AcAA1-16OPNB, 0.69

**8.7 Preparation of Fragment AcAA1-16OH by Selective Removal of a Paranitrobenzyl Protecting Group From AcAA1-16OPNB in the Presence of His(trt).**

**Structure:**

```
Ac-Tyr(tBu)-Thr(tBu)-Ser(tBu)-Leu-Ile-His(trt)-Ser(tBu)-Leu-
Ile-Glu(OtBu)-Glu(OtBu)-Ser(tBu)-Gln(trt)-Asn(trt)-Gln(trt)-
Gln-OH (SEQ ID NO:4)
```

| $C_{187}H_{241}N_{22}O_{32}$ MW 3276.4 | | | | | |
|---|---|---|---|---|---|
| **Materials:** | MW | eq | mmoles | grams | mL |
| AcAA1-16OPNB | 3424.86 | 1 | 0.23 | 0.80 | -- |
| 10% Pd/C, Degussa, 50% water | -- | -- | -- | 0.30 | -- |
| ammonium formate | 63.06 | 15 | 3.5 | 0.22 | -- |
| DMF | -- | -- | -- | -- | 15 |
| Water | -- | -- | -- | -- | 120 |
| Ethyl acetate | -- | -- | -- | -- | 100 |
| Hexane | -- | -- | -- | -- | 44 |
| methanol | -- | -- | -- | -- | 10 |
| saturated aq.NaCl | -- | -- | -- | -- | 5 |
| MTBE | -- | -- | -- | -- | 4 |
| **Theoretical Yield**: 0.76 g | **Expected Yield**: 70-85% | | | | |

**Procedure**:

A 25 mL round bottom flask containing a magnetic stir bar was charged with AcAA1-16OPNB (as synthesized in Section 8.6, above) sand DMF (10 mL). To this solution was added a solution of ammonium formate in water (0.5 mL), then wet palladium on carbon (Degussa, 10%, 50% water). The slurry was stirred under an atmosphere of nitrogen at room temperature for 120 minutes (note 1). The slurry was filtered through a tightly packed bed of celite into 90 mL of water. The filter cake was washed with DMF (5 mL). The aqueous suspension was washed with ethyl acetate (100 mL). The ethyl acetate was then concentrated to a volume of 20 mL (note 2). Hexane (40 mL) was added to complete the precipitation and the solvent was decanted from the solids. The solids were dissolved in methanol (10 mL) and 4: 1 water/saturated aqueous sodium chloride (25 mL) was added to precipitate the peptide. The solids were collected by vacuum filtration, washed with water (10 mL) and dried to give 0-62g of AcAA1-16OH. The solids were triturated with 50% MTBE/hexane (8 mL) at room temperature for 15 hours, collected and dried to give 0.59g of AcAA1-16OH in 77% yield and 90A% purity by HPLC (note 3).

**Notes:**

1 In process control, TLC
80/20 dichloromethane/ethanol
UV, iodine detection
Rf: AcAA1-16OPNB, 0.90
Rf: Ac1-16OH, 69

2 The AcAA1-16OH may begin to precipitate as the solvent volume is reduced.

3 Phenomenex Jupiter, C5, 5μ, 300A
0.8 mL/min, 260 nm
A H$_2$O/0.05% TFA
B 50% IPA/MeOH/0.05% TFA
80-100%B over 10 minutes, 100%B for 5 minutes
Retention time: AcAA1-16OH, 10.73 minutes.

### 8.8 Preparation of Fragment FmocAA27-36NH$_2$ by Solution-Phase Coupling of FmocAA27-35OH With HPheNH$_2$

**Structure:**

```
Fmoc-Asp(tBu)-Lys(Boc)-Trp(Boc)-Ala-Ser(tBu)-Leu-Trp(Boc)-
Asn(trt)-Trp(Boc)-Phe-NH₂ (SEQ ID NO:18)
```

| C$_{130}$H$_{159}$N$_{16}$O$_{24}$ MW 2329.64 | | | | | |
|---|---|---|---|---|---|
| **Materials:** | MW | eq | mmoles | grams | mL |
| FmocAA27-35OH | 2182.61 | 1 | 18.4 | 40.2 | -- |
| HPheNH$_2$ | 162.21 | 1.2 | 22.1 | 3.6 | |
| HBTU | 379.25 | 1.2 | 22.1 | 8.4 | |
| HOAT | 136.1 | 1.2 | 22.1 | 3.0 | |
| EtPr$_2$N        (d=0.755) | 129.25 | 2.1 | 38.7 | 5.0 | 6.6 |
| DMF | | | | | 500 |
| Water | | | | | 600 |
| **Theoretical Yield**        42.8g | **Expected Yield:**        90-105% | | | | |

**Procedure:**

A 2L round bottom flask containing a magnetic stir bar was charged with FmocAA27-350H (as synthesized in Section 7.6, above), HOAT, HpheNH$_2$ and DMF (500 mL). EtPr$_2$N was added and the solution was cooled to 0-5°C then HBTU was added. The reaction mixture was stirred for 15 minutes at 0-5°C then warmed to room temperature and stirred an additional 70 minutes (note 1). The solution was cooled to 0-5°C and water (500 mL) was added to precipitate the peptide. The solids were collected by vacuum filtration, washed with water (100 mL) and dried to give 43g of FmocAA27-36NH$_2$ in 100% yield and 93A% purity by HPLC (note 2).

**Notes:**

1 In process control, TLC
88/12 dichloromethane/methanol
UV, iodine detection
Rf: FmocAA27-35OH, 0.49
Rf: FmocAA27-36NH$_2$, 0.63
2 Phenomenex Jupiter, C18, 5μ, 300A
1.0 mL/min, 260 nm
A H$_2$O/0.1 % TFA
B ACN
75-99%B over 20 min, 99%B for 5 min. Retention time: 29.4 minutes

**8.9 Preparation of Fragment H-AA(27-36)-NH$_2$**

**Structure:**

```
H-Asp(tBu)-Lys(Boc)-Trp(Boc)-Ala-Ser(tBu)-Leu-Trp(Boc)-
Asn(trt)-Trp(Boc)-NH₂ (SEQ ID NO:17)
```

| $C_{115}H_{148}N_{16}O_{22}$ MW 2106.56 | | | | | |
|---|---|---|---|---|---|
| **Materials:** | MW | eq | mmoles | grams | mL |
| FmocAA(27-36)-NH$_2$ | 2328.8 | 1.0 | 9.3 | 21.7 | - |
| Piperidine | 85.15 | 5.0 | 46.6 | 4.0 | 4.6 |
| Methylene chloride (DCM) | - | - | - | - | 100 |
| Water | - | - | - | - | 2 X 100 |
| Methyl t-butyl ether (MTBE) | - | - | - | - | 100 + 30 |
| **Theoretical Yield** 19.6g | **Expected Yield:** 85-95% | | | | |

**Procedure:**

To a 250 mL round bottom flask equipped with a magnetic stirrer and nitrogen blanket was charged the Fmoc-fragment synthesized in Section 8.8, above, the methylene chloride (-5 vol), and the piperidine. A solution was obtained and stirred at room temperature for 1.5 hours (Note 1).

The solution was washed with 2 X 100 mL of water. The layers were separated and the organic layer was concentrated under vacuum to approximately one half the original volume. MTBE was added portionwise, 2 X 50 mL, while the concentration was continued to remove DCM to a point of heavy precipitation and a final pot volume of approximately 150 mL.

The product slurry was stirred and chilled in an ice bath at 0-5°C for approximately one hour. The solids were isolated by vacuum filtration and washed with 2 X 15 mL of MTBE. The product was air dried to give 17.6 g (89.6%) of H-AA(27-36)NH$_2$ of 95% HPLC purity (Note 2).

**Notes:**

1) Reaction completion is monitored by HPLC:
Column: Phenomenox Jupiter C18; 300Å; 5µ
Flow rate: 1 mL/min
Detection: UV at 260 nm
Mobile phase: A: 0.1% aqueous TFA
         B: 0.1% TFA in acetonitrile gradient from 75% B to 99% B in 20 minutes
Retention time: approximately 18 minutes

2) Both of the Fmoc by-products, the dibenzofulvene and its piperidine adduct, are effectively removed in the workup; however, a use-test should be performed before carrying the material forward. If the material fails the use test, the workup procedure is repeated by dissolving the solid in DCM (5 vol), then continuing with the process described above.

**8.10 Preparation of Fragment FmocAA17-36NH$_2$ by Solution-phase coupling of FmocAA17-26OH with HAA27-36NH$_2$**

Structure:

```
Fmoc-Glu(OtBu)-Lys(Boc)-Asn(trt)-Glu(OtBu)-Gln(trt)-
Glu(OtBu)-Leu-Leu-Glu(OtBu)-Leu-Asp(tBu)-Lys(Boc)-Trp(Boc)-
Ala-Ser(tBu)-Leu-Trp(Boc)-Asn(trt)-Trp(Boc)-Phe-NH₂
(SEQ ID NO:12)
```

| $C_{242}H_{313}N_{29}O_{46}$ MW 4364.38 | | | | | |
|---|---|---|---|---|---|
| **Materials:** | MW | eq | mmoles | grams | mL |
| FmocAA17-26OH | 2275.82 | 1 | 9.5 | 21.6 | |
| HAA27-36NH$_2$ | 2106.56 | 1 | 9.5 | 20 | |
| HOAT | 136.1 | 1.2 | 11.4 | 1.55 | |
| HBTU | 379.25 | 1.2 | 11.4 | 4.33 | |
| EtPr$_2$N        (d=0.755) | 129.25 | 2 | 19.0 | 2.46 | 3.25 |
| DMF | | | | | 400 |
| Water | | | | | 600 |
| 2-propanol | | | | | 1100 |
| **Theoretical Yield**        41.5g | **Expected Yield:**        80-85% | | | | |

Procedure:

A 2L round bottom flask containing a magnetic stir bar was charged with FmocAA17-26OH (as synthesized in Section 7.5, above), HAA27-36NH$_2$ (as synthesized in Section 8.9, above), HOAT and DMF (400 mL). EtPr$_2$N was added, the stirred solution was cooled to 0-5°C under an atmosphere of nitrogen and HBTU was added. The reaction mixture was stirred at 0-5°C for 15 minutes, then warmed to room temperature and stirred an additional 2.5 hours (note 1). Water (500 mL) vas added to the reaction mixture to precipitate the peptide (note 2). The resulting slurry was stirred for 45 minutes, the solids were collected by vacuum filtration, washed with water (100 mL) and dried. The solids were returned to the 2L round bottom flask containing a magnetic stir bar and 60°C 2-propanol (1.1 L) was added. The slurry was stirred under an atmosphere of nitrogen as it cooled to room temperature (overnight). The solids were collected by vacuum filtration and dried to give 37.5g of FmocAA17-36NH$_2$ in 90% yield and 95.5A% purity by HPLC (note 1).

Notes:

1 In process control, HPLC
Phenomenex Jupiter, C5, 5µ, 300A
0.75 mL/min, 260 nm
A H$_2$O/0.05% TFA
B 50% IPA/MeOH/0.05% TFA
80-100%B over 10 min, 100%B for 25 min.
Retention time: FmocAA17-26OH, 8.2 minutes, HAA26-36NH$_2$, 8.4 minutes
Retention time: FmocAA17-36NH$_2$, 13.3 minutes

2 The reaction mixture warmed to 38°C on addition of the water.

**8.11 Preparation of Fragment H-AA(17-36)-NH$_2$**

**Structure:**

```
H-Glu(OtBu)-Lys(Boc)-Asn(trt)-Glu(OtBu)-Gln(trt)-Glu(OtBu)-
Leu-Leu-Glu(OtBu)-Leu-Asp(tBu)-Lys(Boc)-Trp(Boc)-Ala-
Ser(tBu)-Leu-Trp(Boc)-Asn(trt)-Trp(Boc)-NH₂ (SEQ ID NO:19)
```

| C$_{227}$H$_{303}$N$_{29}$O$_{44}$ HCl | | | | | |
|---|---|---|---|---|---|
| **Materials:** | MW | eq | mmoles | grams | mL |
| FmocAA17-(17-36)-NH$_2$ | 4364.36 | 1.0 | 5.2 | 22.5 | - |
| 5 N NaOH(ag) | - | - | - | - | 55 |
| Tetrahydrofuran (THF) | - | - | - | - | 170 |
| 1 N HCl | - | - | - | - | 13 |
| Saturated NaCL(aq) | - | - | - | - | 2 X 55 |
| Heptane | - | - | - | - | 3 X 25 + 50, 200 + 50 |
| **Theoretical Yield**    21.6g | **Expected Yield:**    95-100% | | | | |

**Procedure:**

To a 250 mL round bottom flask equipped with an air stirrer and nitrogen blanket was charged with the Fmoc - fragment synthesized in Section 8.10, above, the THF (approximately 7.5 vol), and the 5 N NaOH(-2.5 vol). A two-phase solution was obtained and stirred at room temperature for 10-15 minutes (Note 1).

The layers were separated and the organic phase was adjusted to pH 2-3 with 1 N HCl. The solution was then washed 2 X 55 mL (2.5 vol) with a saturated brine solution (Note 2). The layers were separated and the organic layer was concentrated under vacuum at 15-20°C to about ½ original volume. Heptane was added portionwise, 3 X 25 mL, while the concentration was continued to remove THF to a point of heavy precipitation and a final pot volume of approximately 100 mL (Note 2).

The product slurry was stirred at room temperature for about 2 hrs. The solids were isolated by vacuum filtration and washed with about 50 mL of heptane. The product was air dried to give 21.0g (97.5%) of H-AA(17-36)NH$_2$.

A rework may be performed to remove residual dibenzofulvene byproduct. The product was slurried at room temperature in 200 mL of heptane for 3 hrs. The material was filtered, washed with about 50 mL of heptane, and air dried yielding 20.8 g (96.6%) of product of over 95% HPLC purity.

**Notes:**

1) Reaction completion is monitored by HPLC:
Column: Zorbax LP C8; 1 OOA; 2011 Flow rate: 1 mL/min
Detection: UV at 260 nm
Mobile phase: A: 0.1% aqueous TFA
B: 0.05% TFA in 1:1 ACN:IPA
gradient from 80% B to 99% B in 20 minutes
Retention time: approximately 18 minutes

2) The solids precipitate initially as a waxy gum which remains stirrable and triturates with further concentration to a filterable solid.

## 9. EXAMPLE: Synthesis of Full Length T-20 peptides

Presented herein, in Sections 9.1 - 9.5, below, are examples of the utilization of the peptide intermediate fragments to produce full length T-20 peptides.

The Example presented in this Section demonstrates the successful coupling of solid phase and solution phase synthesis techniques to produce a full-length T-20 peptide from peptide intermediate fragments.

### 9.1 Preparation of Fragment AcAA1-36NH$_2$ by Solution-phase coupling of AcAA1-16OH with HAA17-36NH$_2$

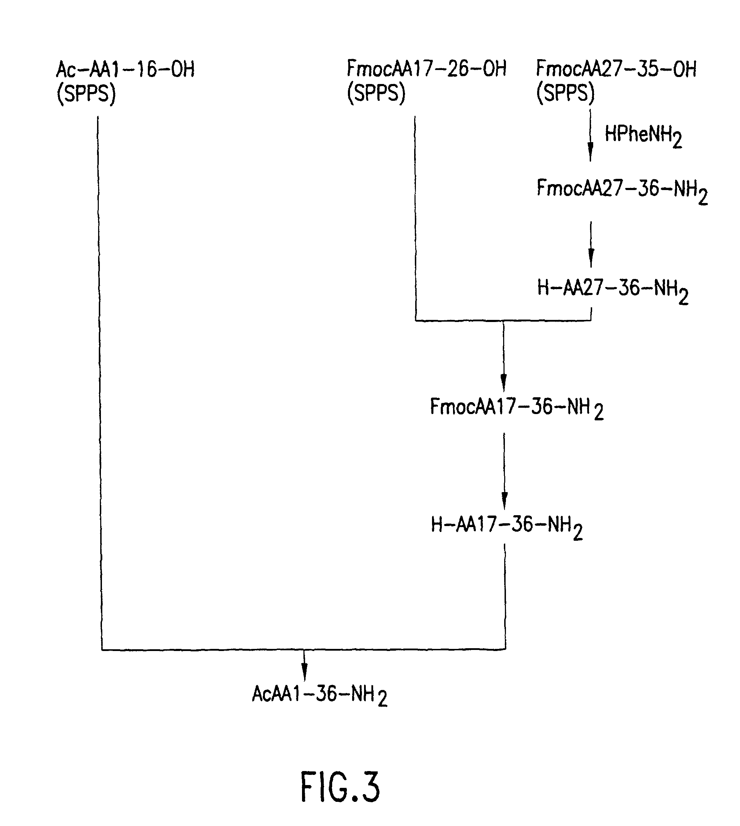

The synthesis route described here represents the culmination of the T-20 four fragment approaches schematically depicted in FIGS. 1 and 3. In instances in which AcAA1-16OH is synthesized via solid phase techniques, the approach in FIG. 1 is followed, and in instances in which AcAA1-16 OH is synthesized via solution phase techniques, the approach in FIG. 3 is followed. It is noted that the T-20 full length peptide synthesized here has the amino acid sequence of SEQ ID N0:1, with an acetyl modification at its amino terminus (i.e., "X") and an amido modification at its carboxyl terminus (i.e., "Z").

### Structure:

```
Ac-Tyr(tBu)-Thr(tBu)-Ser(tBu)-Leu-Ile-His(trt)-Ser(tBu)-Leu-
Ile-Glu(OtBu)-Glu(OtBu)-Ser(tBu)-Gln(trt)-Asn(trt)-Gln(trt)-
Gln-Glu(OtBu)-Lys(Boc)-Asn(trt)-Glu(OtBu)-Gln(trt)-Glu(OtBu)-
Leu-Leu-Glu(OtBu)-Leu-Asp(tBu)-Lys(Boc)-Trp(Boc)-Ala-
Ser(tBu)-Leu-Trp(Boc)-Asn(trt)-Trp(Boc)-Phe-NH₂ (SEQ ID NO:1)
```

| C$_{414}$H$_{543}$N$_{51}$O$_{74}$ MW 7411.95 | | | | | |
|---|---|---|---|---|---|
| **Materials:** AcAA1-1OH | MW | eq | mmoles | grams | mL |
| | 3276.4 | 1 | 0.24 | 0.79 | |
| HClHAA17-36NH$_2$ | 4178.56 | 1 | 0.24 | 1.0 | |
| HOAT | 136.1 | 1.1 | 0.26 | 0.036 | |
| HBTU | 379.25 | 1.0 | 0.95 | 0.25 | |
| EtPr$_2$N     (d=0.755) | 129.25 | 2.8 | 0.67 | 0.087 | 0.115 |
| DMF | | | | | 20 |
| water saturated NaCl | | | | | 25 5 |
| MTBE hexane | | | | | 10 10 |
| **Theoretical Yield**     1.77g | **Expected Yield:**     85-100% | | | | |

### Procedure:

A 100 mL round bottom flask containing a magnetic stir bar was charged with AcAAI-16OH (as synthesized in either Section 7.4, above, via solid phase techniques or in Section 8.7 above, via solution phase techniques), HOAT, DMF (20 mL) then EtPr$_2$N (0.074 mL). The solution was cooled to 0-5°C under an atmosphere of nitrogen and HBTU was added. The solution was stirred for 15 minutes at 0-5°C and HClHAA17-36NH$_2$ (as synthesized in Section 8.11, above) was added, followed an additional 0.041 mL of EtPr$_2$N. The cooling bath was removed and the reaction mixture was stirred for 2 hours (note 1). To precipitate the peptide, water (25 mL) and saturated aqueous sodium chloride (5 mL) was added. The solids were collected by vacuum filtration, washed with water (10 mL) and dried to give 1.74g of crude AcAA1-36NH$_2$ (note 2). The solids were triturated with 50% MTBE/hexane at room temperature for 2.5 hours, collected by vacuum filtration and dried to give 1.70g in 96% yield and 92A% purity by HPLC.

**Notes:**

   1) In process control, HPLC
      Phenomenex Jupiter, C5, 5μ 300A
      0.8 mL/min, 260 nm
      A H$_2$O/0.05% TFA
      B 50% IPA/MeOH/0.05% TFA
      80-100%B over 10 minutes, 100%B for 15 minutes
      Retention time: AcAAI-160H, 11.8 minutes.
      Retention time: HCl HAA17-36NH$_2$, 12.7 minutes.
      Retention time: AcAA1-36NH$_2$, 22.9 minutes.

   2) The water dropout produces a very fine precipitate. A double filtration may be required.

**9.2 Preparation of Fragment FmocAA1-36NH$_2$ (T-20) by Solution-phase coupling of FmocAA1-16OH with HAA17-36NH2**

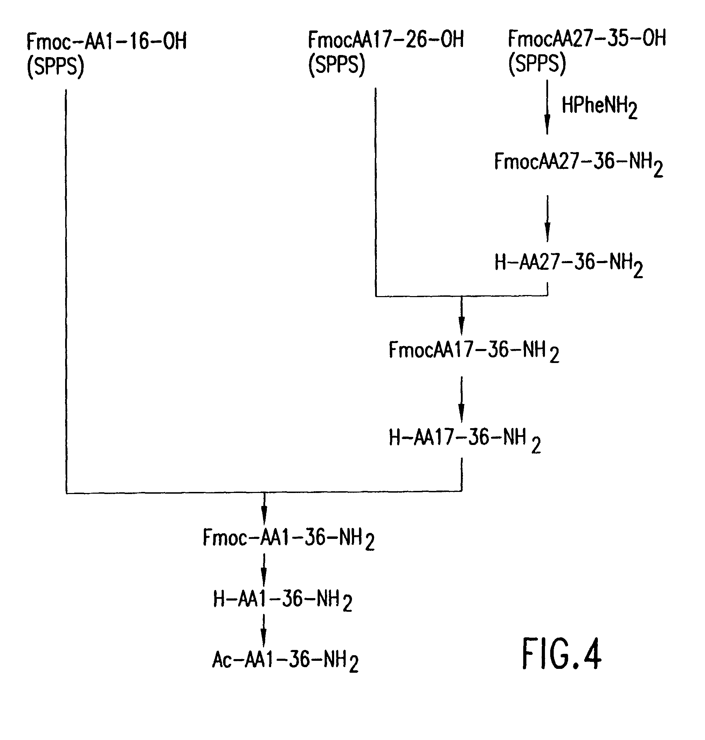

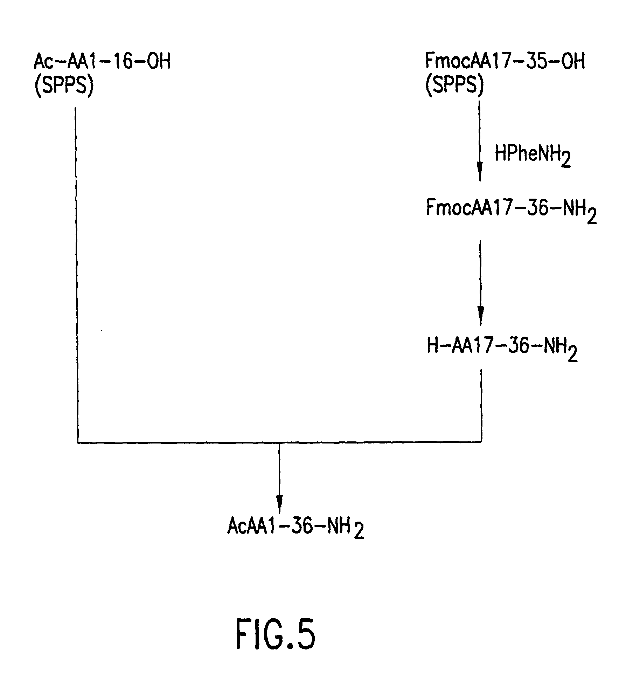

The Example presented in this Section demonstrates the successful coupling of solid and liquid phase synthesis techniques to produce a T-20 peptide from peptide intermediate fragments. In particular, the synthesis route described here represents the T-20 three fragment approach schematically depicted in FIG. 4 to the point in the figure at which FmocAA1-36NH$_2$ is synthesized.

**Structure:**

```
Fmoc-Tyr(tBu)-Thr(tBu)-Ser(tBu)-Leu-Ile-His(trt)-Ser(tBu)-
Leu-Ile-Glu(OtBu)-Glu(OtBu)-Ser(tBu)-Gln(trt)-Asn(trt)-
Gln(trt)-Gln-Glu(OtBu)-Lys(Boc)-Asn(trt)-Glu(OtBu)-Gln(trt)-
```

```
Glu(OtBu)-Leu-Leu-Glu(OtBu)-Leu-Asp(tBu)-Lys(Boc)-Trp(Boc)-
Ala-Ser(tBu)-Leu-Trp(Boc)-Asn(trt)-Trp(Boc)-Phe-NH2
(SEQ ID NO:1)
```

| C$_{427}$H$_{551}$N$_{51}$O$_{75}$ MW 7593.01 | | | | | |
|---|---|---|---|---|---|
| **Materials:** | MW | eq | mmoles | grams | mL |
| FmocAA1-16OH | 3453.89 | 1 | 0.12 | 0.41 | |
| HCl HAA17-36NH$_2$ | 4174.88 | 1 | 0.12 | 0.50 | |
| HOAT | 136.1 | 1.25 | 0.15 | 0.020 | |
| HBTU | 379.25 | 1.25 | 0.15 | 0.057 | |
| EtPr$_2$N   (d=0.755) | 129.25 | 2.5 | 0.30 | 0.039 | 0.051 |
| DMF | | | | | 10 |

(continued)

| C$_{427}$H$_{551}$N$_{51}$O$_{75}$ MW 7593.01 | | | | | |
|---|---|---|---|---|---|
| **Materials:** | MW | eq | mmoles | grams | mL |
| water | | | | | 18 |
| 2-propanol | | | | | 14 |
| **Theoretical Yield** 0.91g | **Expected Yield:** 85-100% | | | | |

**Procedure:**

A 25 mL round bottom flask containing a magnetic stir bar was charged with FmocAA1-16OH (as synthesized in Section 7.3, above), HCl HAA17-36NH$_2$ (as synthesized in Section 8.11, above), HOAT, DMF (10 mL) the EtPr$_2$N was added. The solution was cooled to 0-5°C under an atmosphere of nitrogen and HBTU was added. The reaction mixture was stirred at 0-5°C for 15 minutes, then warmed to room temperature and stirred for 1.5 hours (note 1). Water was added to precipitate the peptide and the solids were collected by vacuum filtration and dried. The solids were triturated with 2 propanol (14 mL) for 15 hours at room temperature then water (3 mL) was added to drive the desired product from solution. The solids were collected by vacuum filtration and dried to give 0.80g of FmocAA1-36NH$_2$ in 88% yield and 85A% HPLC purity.

**Notes:**

1) In process control, HPLC
Phenomenex Jupiter, C5, 5μ, 300A
0.8 mL/min, 260 nm
A H$_2$O/0.05% TFA
B 50% IPA/MeOH/0.05% TFA
80-100%B over 10 minutes, 100%B for 15 minutes
Retention time: FmocAA1-16OH, 11.4 minutes.
Retention tim: HCl HAA17-36NH$_2$, 12. minutes.
Retention time: FmocAA1-36NH$_2$, 20.4 minutes.
TLC, 9/1 dichloromethane/ethanol
UV, Iodine detection
Rft: FmocAA1-36NH$_2$, 0.71.

**9.3 Preparation of Fragment HAA1-36NH$_2$ (T-20)**

The Example presented in this Section demonstrates the successful coupling of solid and liquid phase synthesis techniques to produce a T-20 peptide from peptide intermediate fragments. In particular, the synthesis route described here represents the T-20 three fragment approach schematically depicted in FIG. 4 to the point in the figure at which H-AA1-36-NH$_2$ is synthesized.

**Structure:**

```
H-Tyr(tBu)-Thr(tBu)-Ser(tBu)-Leu-Ile-His(trt)-Ser(tBu)-Leu-
Ile-Glu(OtBu)-Glu(OtBu)-Ser(tBu)-Gln(trt)-Asn(trt)-Gln(trt)-
Gln-Glu(OtBu)-Lys(Boc)-Asn(trt)-Glu(OtBu)-Gln(trt)-Glu(OtBu)-
Leu-Leu-Glu(OtBu)-Leu-Asp(tBu)-Lys(Boc)-Trp(Boc)-Ala-
Ser(tBu)-Leu-Trp(Boc)-Asn(trt)-Trp(Boc)-Phe-NH₂ (SEQ ID NO:1)
```

| $C_{412}H_{541}N_{51}O_{73}$ MW 7370.94 | | | | | |
|---|---|---|---|---|---|
| **Materials:** | MW | eq | mmoles | grams | mL |
| FmocAA1-36NH$_2$ | 7593.01 | 1 | 0.105 | 0.80 | |
| piperidine | | | | | 0.5 |
| DMF | | | | | 9.5 |
| water | | | | | 20 |
| saturated aqueous NaCl | | | | | 5 |
| MTBE | | | | | 5 |
| hexane | | | | | 5 |
| **Theoretical Yield** . 77g | **Expected Yield:** 85-95% | | | | |

**Procedure:**

A 25 mL round bottom flask containing a magnetic stir bar was charged with FmocAA1-36NH$_2$ (as synthesized in Section 9.2, above), DMF (9.5 mL) and piperidine (0.5 mL). The solution was stirred at room temperature under an atmosphere of nitrogen for 2 hours (note 1, 2). Water (20 mL) and saturated aqueous sodium chloride (5 mL) was added to precipitate the protected peptide. The solids were collected by vacuum filtration and dried to give 0.77g of HAA1-36NH$_2$ contaminated with fulvene and the piperidine-fulvene adduct. The solids were triturated with 50% MTBE/ hexane at room temperature for 15 hours to remove the fulvene and the piperidine-fulvene adduct. The solids were collected by vacuum filtration and dried to give 0.73g of HAA1-36NH$_2$ in 95% yield and 90A% purity by HPLC.

**Notes:**

```
1) In process control, HPLC
        Phenomenex Jupiter, C5, 5μ, 300A
        0.8 mL/min, 260 nm
        A H₂O/0.05% TFA
        B 50% IPA/MeOH/0.05% TFA
        80-100%B over 10 minutes, 100%B for 15 minutes
        Retention time: FmocAA1-36OH, 20.4 minutes.
        Retention time: HCl HAA1-36NH₂, 19.9 minutes
        Retention time: fulvene and piperidine-fulvene adduct, 5 minutes.
        TLC, 9/1 dichloromethane/ethanol
        UV, Iodine detection
        Rt: FmocAA1-36NH₂, 0.71.
```

2) The product and starting material do not separate well on TLC or reverse phase HPLC. Product formation was followed by observing the fulvene and piperidiine-fulvene adduct.

### 9.4 Preparation of Fragment AcAA1-36NH$_2$ (T-20) by N-terminal acetylation of HAA1-36NH$_2$

The Example presented in this Section demonstrates the successful coupling of solid and liquid phase synthesis techniques to produce a T-20 peptide from peptide intermediate fragments. In particular, the synthesis route described here represents the T-20 three fragment approach schematically depicted in FIG. 4 to the point in the figure at which Ac-AA1-36-NH$_2$ is synthesized.

### Structure:

```
Ac-Tyr(tBu)-Thr(tBu)-Ser(tBu)-Leu-Ile-His(trt)-Ser(tBu)-Leu-
Ile-Glu(OtBu)-Glu(OtBu)-Ser(tBu)-Gln(trt)-
Asn(trt)-Gln(trt)-Gln-Glu(OtBu)-Lys(Boc)-Asn(trt)-Glu(OtBu)-
Gln(trt)-Glu(OtBu)-Leu-Leu-Glu(OtBu)-Leu-
Asp(tBu)-Lys(Boc)-Trp(Boc)-Ala-Ser(tBu)-Leu-Trp(Boc)-
Asn(trt)-Trp(Boc)-Phe-NH₂ (SEQ ID NO:1)
```

| C$_{414}$H$_{543}$N$_{51}$O$_{74}$ MW 7411.95 | | | | | |
|---|---|---|---|---|---|
| **Materials:** | MW | eq | mmoles | grams | mL |
| HAA1-36NH$_2$ | 7370.94 | 1 | 0.096 | 0.71 | |
| acetic anhydride (d=1.08) | 102.09 | 3 | 0.29 | 0.029 | 0.027 |
| pyridine        (d=0.978) | 79.1 | 6 | 0.58 | 0.046 | 0.046 |
| DMF | | | | | 10 |
| water | | | | | 17.5 |
| saturated aqueous NaCl | | | | | 7.5 |
| **Theoretical Yield**   0.71g | **Expected Yield:**   85-100% | | | | |

### Procedure:

A 25 mL round bottom flask containing a magnetic stir bar was charged with HAA1-36NH$_2$ (as synthesized in section 9.3, above), DMF (10 mL), pyridine (0.046 mL) and acetic anhydride (0.027 mL) (note 1). The solution was stirred at room temperature under an atmosphere of nitrogen for 4 hours (note 2). Water (7.5 mL) and saturated aqueous sodium chloride (7.5 mL) was added to precipitate the protected peptide. The solids were collected by vacuum filtration, washed with water (10 mL) and dried to give 0.65g of AcAA1-36NH$_2$ in 91% yield and 90A% purity by HPLC (note 3).

### Notes:

1) Dichloromethane may be also utilized as the solvent for this reaction.

2) In process control, HPLC
       Phenomenex Jupiter, C5, 5µ, 300A
       0.8 mL/min, 260 nm

A H$_2$O/0.05% TFA
B 50% IPA/MeOH/0.05% TFA
80-100%B over 10 minutes, 100%B for 15 minutes
Retention time: HAA1-360H, 23.3 minutes.
Retention time: AcAA1-36NH$_2$, 22.7 minutes.

3) The product and starting material do not separate well on TLC or reverse phase HPLC.

## 9.5 Preparation of T-20 Side by Side-chain deprotection of AcAA1-36NH$_2$

The Example presented in this Section demonstrates the successful coupling of solid and liquid phase synthesis techniques to produce a T-20 peptide from peptide intermediate fragments. In particular, the synthesis route described here represents the T-20 three fragment approach shown in FIG. 4.

## Structure:

```
Ac-Tyr-Thr-Ser-Leu-Ile-His-Ser-Leu-Ile-Glu-Glu-Ser-Gln-Asn-
Gln-Gln-Glu-Lys-Asn-Glu-Gln-Glu-Leu-LeuGlu-Leu-Asp-Lys-Trp-
Ala-Ser-Leu-Trp-Asn-Trp-Phe-NH₂ (SEQ ID NO:1)
```

| $C_{414}H_{543}N_{51}O_{74}$ MW 4492.1 | | | | | |
|---|---|---|---|---|---|
| **Materials:** | MW | eq | mmoles | grams | mL |
| AcAA1-36NH$_2$ | 7411.95 | 1 | 0.035 | 0.26 | |
| Trifluoroacetic acid | | | | | 2.25 |
| Water | | | | | 15.1 |
| Dithiothreitol | | | | 0.12 | |
| MTBE | | | | | 170 |
| Acetonitrile | | | | | 15 |
| **Theoretical Yield**    157mg | **Expected Yield**:     25-50% | | | | |

## Procedure:

A solution of 90:5:5 (v/v/wt %) trifluoroacetic acid/water/dithiothreitol was degassed with nitrogen and cooled to 0-5°C. To the cooled solution was added AcAA1-36NH$_2$ (as synthesized in Section 9.4, above). The slurry was stirred at 0-5°C until the solids dissolved (- 5 minutes) then warmed to room temperature and stirred for 2.5 hours. The solution was added to 0-5°C MTBE (70 mL) to precipitate the peptide. The slurry was spun in a centrifuge for five minutes at 2200 rpm and the MTBE decanted from the solids. The solids were again suspended in MTBE (50 mL), spun in a centrifuge for five minutes at rpm and the MTBE was decanted. This process was repeated once more then the solids were dissolved in 1:1 water/acetonitrile (30 mL) containing 1 vol% acetic acid and stored at room temperature for 24 hours (note 1). The solution was frozen then freeze dried using a lyopholyzer to give 155 mg of crude T-20. Purification by preparative HPLC provides 55 mg of the full-length T-20 peptide in 95A% purity by HPLC (note 2).

**Notes:**

1) The tBu side-chain of Trp(Boc) is removed quickly leaving TrpCOOH. Decarboxylation of the TrpCOOH requires a minimum of 24 hours at ambient temperature in aqueous acetic acid.

2) Preparative HPLC
     2" YMC, 120A, 10μ, C18
     220 nm, 50 mL/min
     A H$_2$O/0-1 % TFA
     B ACN/0. 1 % TFA
     39-49% B/40 minutes

## 10. EXAMPLE: PURIFICATION OF T-20 PEPTIDE

The Example presented herein describes methods by which T-20 peptides can be purified under conditions which greatly increase peptide purification throughout.

## MATERIALS

Column used: 20 x 30 cm packed with Amberchrom CG-300S (Tosohaus; Montgomeryville, PA) 35μm particles.

## PREPARATION OF BUFFERS

Buffer A = 100 mM Ammonium acetate adjusted to pH 8.5 with NH$_4$OH.
Buffer B = acetonitrile.

1. Column with approximately 6 column volumes of 20% B.
2. T-20 is dissolved in 50-100 mL of 85%A/15%B per gram of peptide. The pH is adjusted to 8-10 with 2M K$_2$CO$_3$. The acetonitrile concentration in the T-20 sample does not exceed 15-20%.
3. T-20 solution is loaded at 500 mL/min, with column pressure monitoring.
5. Column eluate is monitored at 303 mn, and eluate is collected during entire loading process. The wavelength or attenuation is adjusted during the run to keep peak on scale. Absorbance is a function of wavelength and cell path length.
6. Gradient operation is begun as below (1.6% change in B/hour), with pressure being monitored during entire run.

| Time (min) | %B | Flow (mL/min) |
|---|---|---|
| 0 | 15 | 330 |
| 788 | 36 | 330 |

7. 10 min fractions are collected (3.3L) when main peak begins to elute. All of T-20 should be eluted by 35%B. On average, 35-40 fractions are collected. Fractions are stored at 0-5° C until a determination of which fraction to pool is made.
8. Fractions are collected until detector absorbance is less than 0.1 AU or until a major inflection point is reached after the main peak elutes.
9. The purity of each fraction is monitored by analytical reversed-phased HPLC.

## RESULTS

T-20 peptide is much more soluble at pH ranges greater than 7. Column supports commonly used to purify peptides are silica-based and can, therefore, only be used at pH ranges because silica support tends to dissolve at higher pH ranges.

The method described herein utilizes a polystyrene-based resin support that is stable at a broad pH range (pH 1-14). This method greatly increases the capacity of the column in that T-20 throughput increased from 10g up to 250-450g (for an 8" diameter x 30 cm column).

## 11. EXAMPLE: LARGE SCALE SYNTHESIS AND PURIFICATION OF T-20 PEPTIDE RESIN LOADING

FmocTrp(Boc)OH can be loaded on very active 2-CTC resin at levels of 1.2 mmole./g of starting resin or higher. At levels over 1.1 mmole of FmocTrp(Boc)OH per gram of starting resin (over 0.72 mmol/g on loaded resin), it is difficult to obtain negative ninhydrin tests for the last three amino acids of the fragment. Likewise, it becomes difficult to build FmocAA17-26O-resin when the FmocLeuO-resin is loaded greater than 0.85 mmole/g and AcAA1-16O-resin when FmocGlnOH-resin is loaded greater then 0.75 mmole/g.

Upon receipt of the resin from the vendor, a use test with about 1 g of 2-CTC resin is conducted with each amino acid to be loaded. The purpose of the use test is to determine the quantity of amino acid to be used during loading to obtain the desired loading range. Use 0.8, 1.0 and 1.5 equivalents of FmocGlnOH, 1.0, 1.2 and 1.5 equivalents of FmocTrp(Boc)OH and 0.8, 1.0 and 1.5 equivalents of FmocLeuOH with 0.5 equivalent excess of DIEA for each (relative to the reported activity of the resin). If 1 mole of FmocLeuOH cannot be loaded onto 1 Kg of 2-CTC resin, the 2-CTC should not be accepted from the vendor. Below is a description of the targeted resin load for FmocLeuOH, FmocGlnOH and FmocTrp(Boc)OH.

From 1.0 to 1.1 moles of FmocLeuOH can be loaded onto each Kg of 2-CTC resin. Taking into account loss of HCl, the dry weight of the resin after loading FmocLeuOH should be 1.32 to 1.35 times the weight of starting resin and the measured loading should be 0.75 to 0.81 mmole/g.

From 0.8 to 1.0 moles of FmocGlnOH can be loaded onto each Kg of 2-CTC resin. Taking into account loss of HCL, the dry weight of the resin after loading FmocGlnOH should be 1.27 to 1.33 times the weight of starting resin and the measured loading should be 0.63 to 0.75.

From 0.9 to 1.1 moles of FmocTrp(Boc)OH can be loaded onto each Kg of 2-CTC resin. Taking into account loss of HCL, the dry weight of the resin after loading FmocTrp(Boc)OH should be 1.44 to 1.54 times the weight of starting resin and the measured loading should be 0.63 to 0.72.

For accurate weight gain analysis, the loss on drying (LOD) of the starting resin must be determined. It should not exceed 1%. If residual solvent (or DIEA/HCl) is not completely removed from the resin during drying (after the loading), then the isolated mass will be higher and the measured loading lower. However, the total moles loaded (mass times measured loading) should fall within the above mentioned ranges. If the high-end loading level is exceeded (or total moles loaded per Kg of starting resin) for any of the amino acids mentioned, by over 10%, run a use test using less amino acid. This is particularly important for FmocGlnOH.

### 11.1 Large Scale Preparation of Fmoc-AA(Boc)-2-Chlorotrityl Resin

The purpose of the following section is to perform a large scale loading of resins suitable for preparation of high quantities of peptides for solid phase peptide synthesis (SPPS). The resin is charged with each of the following: FmocTrp(Boc)OH, and FmocGln(Boc)OH, and FmocLeu(Boc)OH. Each starting residue was charged onto approximately 4 Kg of 2-CTC resin.

| AA Loaded | 2-CTC (Kg) | Loaded Mass (Kg) | Substitution (mmol/g)[1] | Total Moles Loaded[1] |
|---|---|---|---|---|
| FmocTrp(Boc)OH | 3.7 | 4.493 | 0.56 (0.56) | 2.52 (2.52) |
| FmocLeu(Boc)OH | 2.2 | 2.436 | 1.07 (0.647) | 2.6 (1.58) |
| FmocGln(Boc)OH | 3.65 | 3.856 | 0.55 (0.52) | 2.12 (2.0) |

1. The first number was calculated from a weight based HPLC assay against an Fmoc-AA standard. The value in parentheses was calculated from weight gain taking into account a 12% LOD on the starting 2-CTC resin.

Starting 2-CTC resin was purchased from Colorado BioTech with a reported loading of 1.4 meq/g. The standard loading procedure, 1.5 eq of FmocTrp(Boc)OH, 1.7 eq of DIEA in DCM (5 vol), ambient temperature for 2 hours, was used starting with 3.7 Kg of 2-CTC. This provided 4.493 Kg of FmocTrp(Boc)O-resin that had a loading calculated at 0.56 mmol/g (2.52 moles total) by weight based HPLC assay of FmocTrp(Boc)OH after cleavage from the resin. By weight gain of the resin, the loading is calculated to be 0.36 mmol/g for a total of 1.62 moles prepared. However, if the 12% LOD on the starting 2-CTC resin is taken into account, the loading calculated by weight gain is the same at 0.56 mmole/g for a total of 2.52 moles, well short of the targeted loading of 4 moles FmocTrp(Boc)OH per 4 Kg of resin.

A total of 4.59 Kg of FmocLeuO-resin was prepared from 3.9 Kg of 2-CTC in two runs using standard loading procedures.

However, a substantially lower loading than was anticipated was obtained. The first batch had a calculated loading of 1.07 mmol/g by weight based HPLC analysis of FmocLeuOH after clevage from the resin. This is clearly impossible as the loading calculated by weight gain taking into account the 12% LOD on the starting 2-CTC resin, is (500 g) 0.647 mmole/g. The actual loading is likely close to 0.647 mmole/g for a total of 1.577 moles loaded.

A second batch of FmocLeu-O-resin prepared (2.154 Kg) from 1.7 Kg of 2-CTC had a calculated loading of 0.68 mmole/g versus a weight gain loading of 0.66 mmole/g.

A total of 3.856 Kg of FmocGlnO-resin was prepared in a single batch starting with 3.65 Kg of 2-CTC using 0.8 eq of FmocGlnOH and 1 eq of DIEA in 7 volumes of 5/2 DMF/DCM. The substitution calculated by weight based HPLC assay was 0.55 mmol/g for a total of 2.12 moles of FmocGlnO-resin. The loading calculated by weight gain taking into account the 12% LOD of the starting 2-CTC resin is 0.52 mmol/g for a total of 2.0 moles FmocGlnO-resin.

Generally, the loading by weight gain should be greater than or equal to the loading calculated by a weight based HPLC assay against the AA loaded or fulvene removed. The starting 2-CTC should have less than 1% LOD and the isolated FmocAAOresin will likely have a small amount of NMP, salt and/or residual FmocAAOH.

#### 11.1.1 Preferred Method for Large Scale Preparation of Fmoc-AA(Boc)-2-Chlorotrityl Resin

#### FmocTrp(Boc)OH and FmocLeuOH

The air sensitive 2-Chlorotritylchloride resin (1 eq, 3.7 Kg, 5.18 mole) is added to a SPPS chamber and washed with 5 volumes of DCM. The solvent is drained and a solution of either FmocTrp(Boc)OH or FmocLeu(Boc)OH (1.5 eq) and DIEA (1.7 eq) in DCM (5 vol) is added (note 1). The slurry is agitated with stirring for 2 hours. The solvent is drained and the remaining active sites on the resin are end-capped with 9:1 MeOH:DIEA (5 vol) for 30 minutes. The solvent is drained and the resin is washed with 6 x 5 volumes of DCM. The resin is dried to constant weight, then sampled and analyzed for loading (note 2). The same procedure is used for loading FmocLeuOH.

#### FmocGluOH

The air-sensitive 2-CTC (3.65 Kg, 1.4 mmole/g) resin is charged into a 40L SPPS chamber under an atmosphere of nitrogen and washed with DCM (5 vol). A 20L reactor is charged with FmocGlnOH (1.506 Kg, 0.8 eq), DMF (5 vol) and DIEA (1.0 eq). The SPPS chamber is drained and the DMF solution is added. The reactor is rinsed with DCM (2 vol) and the rinse added to the SPPS chamber. The resin is stirred in the SPPS chamber under a nitrogen atmosphere for 2 hours and drained. Any remaining active sites on the resin are capped with 9:1 Methanol:DIEA (5 vol) for 30 minutes. The resin is drained, washed with DCM (6 x 5 vol) and dried to constant weight (3.856 Kg). The resin is sampled and tested for loading level (note 2).

#### Notes :

1. The 2-Chlorotritylchloride resin is very sensitive to moister. Water caps the resin and yields HCl. As long as the solvent is dry, there is little difference between DCE and DCM.
2. Resin loading is determined by cleaving the Fmoc amino acid from the resin and assaying against a standard with a quantitative wt/wt HPLC analysis.
Phenomenex Jupiter C18, 300A, Sm
1 mL/min, 260 nm
A: 0.1% aqueous TFA
B: 0.1% TFA in acetonitrile
65%B, isocratic
Retention time: approximately 8 minutes

Determination of the load by weight gain of the resin can be inaccurate as the number of washes used prior to drying the resin may not be sufficient to completely remove NMP from the resin. The calculation is:

(Mass Fmoc-AA-OH-resin - Mass of stating 2-CTC)/Mass Fmoc-AA-

OH-resin (MW of AA loaded - MW HCl)

It was observed that storing the peptide fragments of the present invention for prolonged periods of time (days) in DCM results in substantial cleavage from the resin. For example, during the course of building FmocAA17-26OH, the partially built fragment was stored over the weekend twice in DCM. An 8% yield of FmocAA17-26OH was obtained after completion of the synthesis and removal from the resin. It is suspected that trace HCl in the DCM slowly cleaved the partially built fragment from the resin during the weekend storage.

Samples of FmocAA17-26O-resin, AcAA1-16O-resin and FrmoAA27-35O-resin were allowed to stand in DCM and IPA at room temperature for 1 week. The supernatant for each was analyzed by HPLC. Although quantitative results were not obtained, all the fragments were cleaved from the resin to a significant extent in DCM while cleavage in IPA was not observed. If partially built fragments need to be stored for a few days (over the weekend), it is better to rinse the solids with NMP, drain the bed and let it stand saturated with NMP under a nitrogen atmosphere.

DCM was replaced with IPA for the final washes of FmocLeuO-resin, FmocGlnO-resin, FmocTrp(Boc)O-resin, FmocAA17-26O-resin, AcAA1-16O-resin and FrmoAA27-35O-resin. The IPA saturated FmocLeuO-resin, FmocGlnO-resin and FmocTrp(Boc)O-resin were dried at 40°C. FmocAA17-26O-resin, AcAA1-16O-resin, and FrmoAA27-35O-resin were built from the loaded resins that were dried at 4°C. At completion of the synthesis, the resin bound fragments were washed clean with DOM followed by IPA. The IPA saturated, resin bound fragments were divided and dried at ambient temperature and 40°C. The fragments were cleaved from the resin and analyzed by HPLC. There were no observed differences in the fragment purity. Drying IPA saturated, resin bound fragments at 40°C does not impact the quality or quantity of the fragment isolated.

SPPS of the fragments in DMF as opposed to NMP/DCM was examined (1 g of resin swells to 5 mL in both solvent systems). Fragment FmocAA27-35OH was synthesized using DMF as a solvent and TBTU vs HBTU as a coupling reagent. FmocAA27-35OH was isolated in 77% yield and 89.5A% purity.

The work up protocol for fragments FmocAA17-36NH$_2$ and AcAA1-36NH$_2$ removes the uncoupled fragments. Acetylating (endcapping) these fragments at deletion sites during the solid phase synthesis will ensure that the deletion fragments do not couple in the solution phase couplings. The uncoupled fragments in the synthesis of both FmocAA17-36NH$_2$ and AcAA1-36NH2 should be removed in the IPA and ACN work ups.

### 11.2 Large Scale Preparation of Fragment Ac-AA(1-16)-OH(Fragment 3c)

Large scale preparation by SPPS of fragment AcAA1-16OH was conducted in two runs yielding a total of 7.941 Kg of resin bound fragment from 3.53 Kg of FmocGlnOH-resin. The loading value for the starting FmocGlnOH-resin in both runs was the somewhat overestimated 0.55 mmol/g value obtained from a weight based HPLC assay rather then the more accurate 0.52 mmol/g obtained by weight gain taking into account 12% LOD on the starting 2-CTC. In addition to having the resin loading lower then desired, a 6% excess of each amino acid was used during the SPPS.

The SPPS used 2.5 equivalents of the first FmocGln(trt)OH onto the resin, and 1.7 equivalent of each of the subsequent amino acids in the fragment (relative to the 0.55 mmol/g loading). The coupling reactions were conducted in 8 volumes of 3:1 NMP:DCM and all the but two of the 32 coupling reactions (acetylation included) went to completion within 2 hours. All washes were done with 5 volumes of NMP.

The resin-bound fragment is washed with 5 to 6 times in 3.4 volumes (relative to the weight of the dry AcAA1-16O-resin) of 1% TFA/DCM at 0-5°C, 5 minutes per wash. Each wash is collected onto 1.36 volumes of 0.33 M aq NaHCO$_3$ to neutralize the TFA. The triphasic washes are combined and water (2 vol) is added. The DCM is removed by distillation leaving a filterable precipitate in the aqueous sodium bicarbonate. During the course of the distillation, the multi-phasic slurry becomes viscous and cream-like before collapsing into a filterable slurry as the last of the DCM is removed. The slurry is cooled to 0-5 °C and the pH is adjusted to 3.0 with 0.1 N HCl. The slurry was stirred at 0-5 °C for 1-2 hours, collected by filtration, washed and dried. In the remaining runs the solids were collected, returned to the reactor and triturated with water for 1-2 hour, then collected and dried.

Cleavage of AcAA1-16oH from the resin was accomplished in four runs using 1% TFA in DCM. A total of 4.814 Kg of AcAA1-16OH was produced from 3.53 Kg of FmocGlnO-resin in 93-96A% purity. The overall yield based on the 0.52 mmol/g loading determined by weight gain taking into account the 12% LOD of the starting 2-CTC (5.83 Kg theoretical) is 83%.

The sodium salt of AcAA1-16OH is insoluble in DMF and NMP, therefore, the pH adjustment is needed to protonate the carboxyl terminus. The water wash ensures removal of the sodium trifluoroacetate from the product. A very small amount of sodium trifluoroacetate on a wt/wt basis will interfere with the solution-phase coupling with HAA17-36NH$_2$.

This fragment should be dried at ambient temperature. A stability study looking at drying wet AcAA1-16OH at 40 °C showed about 4% degradation over 3 days. Interestingly, the dry solid is stable at 80°C for 24 hours.

**11.2.1 Preferred Method of Large Scale Preparation of Fragment Ac-AA(1-16)-OH(Fragment 3c)**

To a 40 L peptide chamber is added the resin-bound FmocGlnOH (1.53 Kg, 0.55 mmol/g, 0.84 mole). The resin is conditioned with DCM (5 vol) under nitrogen with agitation for 15 minutes, then drained. A 20% piperidine in NMP solution (5 vol) is added and the suspension is stirred under nitrogen for 20 minutes. The solution is drained and the process repeated. The resin is washed 5 times with 5 volumes of NMP to remove dibenzofulvene and piperidine as determined by a chloranil test (note 1).

While the resin is being washed clean of piperidine, the subsequent amino acid in the sequence (1.5 eq), HOBT (1.5 eq) and DIEA (1.5 eq) are combined in NMP (6-7 vol) and cooled to 0°C. To the cool solution is added HBTU (1.5 eq) and the solution is stirred for 10-15 minutes to dissolve the HBTU. The cooled solution of activated amino acid is added to the resin followed by a DCM rinse (2.5 vol) (note 2). The suspension is stirred under nitrogen purge for 2 hours, then a sample of the resin is removed for a qualitative ninhydrin test (note 3). If the ninhydrin test is negative, the vessel is drained, washed with 3 times 5 volumes of NMP (note 4) and the cycle is repeated with the next amino acid in the sequence. If the test is positive, the suspension is agitated for an additional hour and retested. If the ninhydrin is negative, proceed to the next cycle. If the ninhydrin remains positive, recouple with one eq of the amino acid and reagents. If the ninhydrin is positive after one hour, endcap with 5eq of acetic anhydride and 5eq of pyridine in NMP (10 vol) for one hour.

At completion of the fragment synthesis, the Fmoc is removed from the last amino acid as described and the resin is stirred in a solution of acetic anhydride and pyridine (5 eq each) in 3:1 NMP:DCM (10 vol) for 20-30 minutes or until a negative ninhydrin test is obtained. The resin is drained, washed with 2 X 5 volumes of NMP, 5 times with 5 volumes of DCM and dried yielding 3.49 Kg of AcAAl-160-resin.

The 40 L SPPS chamber is charged with the dried, resin-bound AcAA1-16 (3.49 Kg). The resin-bound peptide is cleaved from the resin using 6 X 3.4 volumes of 1% TFA/DCM (note 7). Each cleavage wash is collected onto 1.36 volumes of 0.33 M aq. sodium bicarbonate. The biphasic fractions are combined and diluted with 2 volumes of water. The biphasic mixture is concentrated under reduced pressure (15 in Hg, 20 °C) to remove DCM. Additional water (2 volumes) is added during the distillation as needed to maintain stirring (note 6). When the DCM is removed (several hours), the suspension is cooled to 0 °C and the pH is adjusted to 3 with IN aq HCl. The slurry is stirred at 0 °C for 1 hr and collected. The still damp solid is returned to the reaction vessel and triturated with water (7 volumes) to remove residual TFA and sodium trifluoroacetate. The solids are collected by vacuum filtration and dried to a constant weight (1.12 Kg, 95A%). The yield AcAA1-16OH based on 0.52 mmol/g load is 86% (note 7).

**Notes:**

1. To approximately 1 mL of acetone is added 1 drop of a saturated solution if chloranil in toluene, followed by one drop of effluent. A blue or violet color is a positive indication for the presence of a piperidine. As the bed height increases, larger volumes of NMP will be needed to wash out the piperidine.

2. DCM is added to ensure adequate swelling of the resin.

3. Quantitative ninhydrin testing is adequate for monitoring the coupling efficiency. A 2-20 mg sample of the resin is withdrawn and washed clean with methanol. To the sample is added 3 drops of 76% phenol in ethanol, 4 drops of 0.2 mM KCN in pyridine and 3 drops of 0.28 M ninhydrin in ethanol. The solution is diluted to 0.5-1 mL with ethanol and placed in a heat block at 75°C for 5-10 minutes. A blue or violet color indicates free amines (positive). Clear or faint blue is a negative result. Protocol for Quantitative Ninhydrin Test References: Sarin, V. K., Kent, S. B. H., Tam, J. P., & Merrifield, R. B. (1981) Analytical Biochem. iii, 147-157.

4. If the resin is to be stored overnight, wash with 2 times 5 volumes of NMP, drain and store under nitrogen. Do not store under DCM. This can result in cleavage of the peptide from the resin and substantial mass loss.

5. Each fraction is tested for product content by TLC visualization at 254 nm. The majority of the product is removed in the first 5 washes.

6. As the DCM is removed, the reaction takes on the consistency of marshmallow cream. As the distillation continues, the suspension gradually breaks into a solid slurry. It is important to remove the DCM slowly to avoid product oil-out.

7. Vydac C8, 5 μ, 300A
1 mL/min, 30°C, 230 nm
A 1000:1 water/TFA

B 800:200:1 IPA:ACN:TFA
60-95%B/30 min.
Retention time: 13.1 minutes

### 11.3 Large Scale Preparation of Fragment FmocAA(17-26)-OH(Fragment 10b)

A total of 5.3 Kg of FmocAA17-260-resin was prepared from 2.4 Kg of FmocLeuO-resin in two, equal size runs. The 5.3 Kg of FmocLeuO-resin was cleaved from the resin in four equal size runs yielding 3.184 Kg of FmocAA17-26OH averaging 94.4A% purity and 90% yield.

The reactions were run using 1.5 equivalent of each amino acid relative to the loading factor, resulting in the use of 65% excess of amino acid for each coupling cycle. All of the coupling reactions were complete (negative ninhydrin test) within 2 hours.

The Fmoc protecting group was removed with 20% piperidine in NMP (5 volumes) for 20 minutes and repeated. The piperidine was washed out of the resin with 5 x 5 volume NMP washes. The coupling reactions were run in 8 volumes of 3:1 NMP:DCM. The coupling solution was drained and the solids were cleaned with three, 5 volume NMP washes. The cycle was repeated with the next amino acid in the sequence. At completion of the fragment, the resin bed was washed with 2 x 5 volumes of NMP, 5 x 5 volumes of DCM and dried to constant weight.

Several 1% TFA in DCM washes are used to remove the fragment from the resin. Cooling the 1% TFA in DCM solution is not necessary as the stability of this fragment in 1% TFA/DCM (1%/day) is much greater then AcAA1-16OH. As the product containing, acidic DCM washes are collected, they are neutralized with pyridine. The combined washes are distilled to remove DCM and ethanol is added to maintain a solution as well as to drive the remaining DCM out during the distillation. After the DCM has been removed (determined by an increase in the temperature of the distillate being removed) water was added to precipitate the fragment. The solid was collected by vacuum filtration (less than 60 minutes). The still damp solid was transferred back to the reactor and triturated with 80/20 ethanol/water (5 vol) at 0-5 °C for 60 minutes. The precipitated FmocAA17-26OH was collected by vacuum filtration, ashed with a minimal amount of 80/20 ethanol/water and dried (vacuum, no heat, 10 days).

### 11.3.1 Preferred Method for Large Scale Preparation of Fragment FmocAA(17-26)-OH(Fragment 10b)

A 40L SPPS chamber is charged with FmocLeuO-resin (1.2 Kg, 0.776 mole) followed by DCM (5 vol) to swell the resin. The DCM is needed to ensure complete swelling of the dried starting resin. The suspension is stirred for 20-30 minutes and drained. A 20% piperidine in NMP solution is added (5 vol) and the solution is stirred for 20 minutes. The solvent is drained and the process is repeated. The solvent is drained and the resin bed is washed with 5 x 5 volumes of NMP to remove piperidine (note 1).

While deprotecting, a 20 L round bottom flask with mechanical stirred is charged with the next amino acid in the sequence (1.95 eq), HOBT (1.95 eq), DIEA (1.95 eq) and NMP (6 vol). The solution is stirred until the solids dissolve, then cooled to 0-5 °C and HBTU (1.95 eq) is added. The solution is stirred until the HBTU dissolves or the resin if free of piperidine (whichever is first), then added to the resin. The reactor is washed with DCM (2 vol) and this is transferred to the SPPS reactor. Note: The stoichiometry of amino acid and reagents should be 1.5 equivalents.

The resin is suspended in coupling solution with gentle stirring. After two hours, a sample of resin is removed from the SPPS chamber and a qualitative ninhydrin test is performed (note 2). If the ninhydrin test is negative, the vessel is drained, washed with 3 times 5 volumes of NMP and the cycle is repeated with the next amino acid in the sequence (the DCM swelling is used only for the first amino acid loaded).

If the test is positive, the suspension is agitated for an additional hour and retested. If the ninhydrin is negative, proceed to the next cycle. If the ninhydrin remains positive, recouple with one eq of the amino acid and reagents. If the ninhydrin is positive after one hour, endcap with 5 eq of acetic anhydride and 5 eq of pyridine in NMP (10 vol) for one hour.

At completion of the fragment synthesis, the resin is drained, washed with 2 X 5 volumes of NMP, 5 X 5 volumes of DCM and dried yielding 2.67 Kg of FmocAA17-260-resin.

FmocAA17-26OH is cleaved from the resin (1.33 Kg) using 5-6 x 1.7 volumes of 1% TFA in DCM, 5 minutes each. The 1% TFA/DCM washes are collected in a flask containing pyridine (1:1 volume ratio with the TFA in the wash). The product containing washes are combined (approximately 14 L) and the DCM is removed by distillation to a minimum pot volume (approximately one third of the original volume). The vacuum is adjusted to maintain a pot temperature of 15-25 °C. Ethanol (6.5 vol) is added and the distillation is continued until the DCM is removed (as determined by the temperature of the distillate, note 3). Again the vacuum is adjusted to maintain a pot temperature of 15-20 °C. The final pot volume should be approximately 8-10 volumes. The solution is cooled to 5-10°C and water (6.5 vol) is added over 30 minutes to precipitate the FmocAA17-26OH. The solid is collected by vacuum filtration and washed with water (2-3 vol). To remove residual pyridine and/or salts, the still damp solid is charged back to the reactor and 80/20 ethanol/water (5 vol), precooled to 0°C, is added. The suspension is stirred at 0°C for 60 minutes, the solids are collected by vacuum filtration, washed with a minimal amount of 80/20 ethanol/water and dried to constant weight to give 0.806 Kg of FmocAA17-26OH in 90.6% yield and 96A% purity (note 4).

**Notes:**

1 To 1 mL of acetone is added 1 drop of a saturated solution of chloranil in toluene, followed by 1 drop of effluent. A blue or violet color indicates the presence of piperidine.

2 Quantitative ninhydrin testing is adequate for monitoring the coupling efficiency. A 2-20 mg sample of the resin is withdrawn and washed clean with methanol. To the sample is added 3 drops of 76% phenol in ethanol, 4 drops of 0.2 mM KCN in pyridine and 3 drops of 0.28 M ninhydrin in ethanol. The solution is diluted to 0.5-1 mL with ethanol and placed in a heat block at 75°C for 5-10 minutes. A blue or violet color indicates free amines (positive). Clear or faint blue is a negative result.

3 The head temperature during the vacuum distillation of DCM was 10-15°C. The head temperature rose to 3500 when the DCM was removed.

4 Vydac, C8, 5µ, 300A

1 mL/min, 262 nm, 30°C

A water/0.1% TFA

B ACN/0.1% TFA

80-99%B/20 min.

retention time: 15.2 minutes

### 11.4 Large Scale Preparation of Fragment Fmoc-AA(27-35)-OH(Fragment 16b)

A total of 4.694 Kg of FmocAA27-35OH was synthesized from 4.45 Kg of FmocTrp(Boc)O-resin. The solid phase synthesis was run in two batches and cleavage from the resin was four batches. FmocAA27-35OH resulting from one batch was about 5% less pure than material from the other batch. This was due to an unidentified, 5A% impurity that was removed in processing to FmocAA17-36NH$_2$. The total amount of FmocTrp(Boc)OH loaded onto the resin was 2.5 moles. The solid phase synthesis proceeded as expected for resin loaded at about 63% of capacity. All of the couplings were complete at the first, 2 hour check point. The reaction and rinse volumes used for the SPPS and cleavage were the same as those used in the synthesis of FmocAA17-26OH. Cleavage from the resin was as described above. Filtration was quick (15 min). Drying the solid after the 90/10 ethanol/water trituration took 3 days (vacuum, no heat). The fragment is stable to drying at 40 °C.

### 11.4.1 Preferred Method of Large Scale Preparation of Fragment Ac-AA(27-35)-OH(Fragment 16b)

To a SPPS chamber containing FmocTrp(Boc)-resin (1 eq, 2.2 Kg, 1.23 moles) is charged DCM (5 vol). The suspension is stirred for 15 minutes and drained. A solution of 20% piperidine in NMP (5 volumes) is added and the suspension stirred for 10-15 minutes to remove the Fmoc protecting group. This process is repeated and the resin is washed with 5-7 x NMP (5 vol) to a negative chloranil test (note 1).

The subsequent amino acid (1.5 eq), HOBT (1.5 eq) and DIEA (1.7 eq) are combined in NMP (6 vol), then cooled to 0-5°C (note 2). HBTU is added and the solution is stirred for 10-15 minutes to dissolve. The solution of activated amino acid is added to the resin. DCM (2 vol) is used to wash the reactor then added to the resin (note 3). The suspension is stirred under an atmosphere of nitrogen for 1-2 hours. Coupling completion is monitored with a qualitative ninhydrin test (note 4). If the ninhydrin test is negative, the vessel is drained, washed with 3 times 5 vol of NMP and the cycle is repeated with the next amino acid in the sequence (the DCM swelling is used only for the first amino acid loaded).

If the test is positive, the suspension is agitated for an additional hour and retested. If the ninhydrin is negative, proceed to the next cycle. If the ninhydrin remains positive, recouple with one eq of the amino acid and reagents. If the ninhydrin is positive after one hour, endcap with 5 eq of acetic anhydride and 5 eq of pyridine in NMP (10 vol) for one hour.

At completion of the fragment synthesis, the resin is drained, washed with 2 X 5 volumes of NMP, 5 X 5 volumes of DCM and dried yielding 4.11 Kg of FmocAA27-35O-resin.

The fragment is cleaved from the resin (2.05 Kg) using 6 x 1.7 volumes of 1% TFA in DCM, 5 minutes each. The 1% TFA/DCM washes are collected in a flask containing pyridine (1:1 volume ratio with the TFA in the wash). The product containing washes are combined and the DCM is removed by distillation (vacuum adjusted to maintain a pot temperature of approximately 15 °C) to approximately one half of the original pot volume. Ethanol (5 vol) is gradually introduced and the distillation (vacuum adjusted to maintain a pot temperature of approximately 20 °C) is continued until the DCM is removed (as determined by the temperature of the distillate, note 5). The pot volume should be 6-7 volumes. The cloudy solution is cooled to 10-15 °C and water (3.5 vol) is added over 30 minutes with rapid agitation to precipitate the FmocAA27-35OH. The solids are collected by vacuum filtration (15 minutes), and washed with water (1 vol). To remove residual pyridine, the damp solid is returned to the reactor and 90/10 ethanol/water precooled to 0-5 °C is added (10 vol). The slurry is stirred at 0-5 °C for 60 minutes, the solid is collected by vacuum filtration, washed with 90/10 ethanol/water (0.5 vol) and dried to constant weight giving 1.19 Kg of FmocAA27-35OH in 89% yield and 89.2A% purity (note 6).

This protocol can be reworked by triturating the solid with 15 volumes of 9:1 ethanol/water with stirring for 12 hours, followed by collection and drying.

**Notes:**

1. To 1 mL of acetone is added 1 drop of a saturated solution of chloranil in toluene, followed by I drop of effluent. A blue or violet color indicates the presence of piperidine.

2 The reagents less HBTU are added at room temperature to assist dissolution. HBTU is added to the cooled solution to minimize racemization.

3 Activation is carried out in NMP because HBTU is not soluble in DCM. DCM is used to wash the reactor and added to the resin to maintain adequate resin swelling.

4 Quantitative ninhydrin testing is adequate for monitoring the coupling efficiency. A 2-20 mg sample of the resin is withdrawn and washed clean with methanol. To the sample is added 3 drops of 76% phenol in ethanol, 4 drops of 0.2 mM KCN in pyridine and 3 drops of 0.28M ninhydrin in ethanol. The solution is diluted to 0.5-1 mL with ethanol and placed in a heat block at 75 °C for 5-10 minutes. A blue or violet color indicates free amines (positive). Clear or faint blue is a negative result.

5 The head temperature during the vacuum distillation of DCM was 10-15 °C. The head temperature rose to 35 °C when the DCM was removed.

6 Vydac, C8, 5 μ, 300A
1 mL/min, 262 nm, 30°C
A water/0.1% TFA
B ACN/0.1% TFA
80-99%B/20 min.
retention time: 15.3 minutes

### 11.5 Large Scale Preparation of Fragment FmocAA(27-36)-OH by Solution Phase coupling of FmocAA(27-35)-OH with HPheNH$_2$

A total of 5.226 Kg of FmocAA27-36NH$_2$ was prepared in four runs from 4.676 Kg of FmocAA27-35OH. Residual coupling reagents or solvent account for the greater then 100% yield. These are removed in the next stage.

Solid sticking to the reactor wall after the water dropout was cited as a significant problem in this stage. Isolation of the crude solid by vacuum filtration took 30 minutes. The average drying time (vacuum, no heat) for the runs was 8 days. The solid needs to be compressed on the filter to remove excess water before it goes into the oven. The fragment is stable to drying at 40 °C and the vacuum ovens need to be heated.

A use test is run on a 0.5 to 1 gram scale with the batch/lot of starting materials to be used to help establish quality and identity. Thin Layer Chromatography (TCL) and HPLC are used to monitor conversion of starting material to product. The primary impurity to look for in fragment FmocAA27-35OH is trifluoroacetic acid (or a salt of it). Activation and reaction of trifluoroacetic acid present in the FmocAA27-35OH with the phenylalanine amide is a rapid process. A small excess of phenylalanine amide can be used to consume any trifluoroacetic acid present. A more significant quality issue is with the phenylalanine amide purchased. Most vendors sell this as the HCl salt. Several lots of the HCl salt of phenylalanine amide have produced an impurity (5-15A%) that co-elutes with the starting material on HPLC. The impurity can be separated from the starting fragment and product by TLC. The impurity is FmocAA27-35NH$_2$ resulting from ammonium chloride present in the HCl salt of phenylalanine amide.

### 11.5.1 Large Scale Preparation of Fragment FmocAA(27-36)-OH by Solution Phase Coupling of FmocAA(27-35) -OH with HPheNH$_2$

A 40L jacketed reactor with a mechanical stirrer is charged with FmocAA27-35OH (1 eq, 1.185 Kg), HOAT (1.1 eq), HCl.HPheNH$_2$ (1.15 eq) and DMF (12.5 vol). DIEA (2.1 eq) is added, the solution is cooled to 0-5 °C and HBTU (1.2 eq) is added. The reaction mixture is stirred for 15 minutes at 0-5 °C then warmed to room temperature and stirred an additional 70 minutes (note 1, HPLC used for in process check). After the reaction is judged complete by HPLC, water (12.5 vol) is added over 15-30 minutes to precipitate the peptide. The slurry is stirred for 15 minutes at ambient temperature. The solids are collected by vacuum filtration, washed with water (3 vol) and dried to give FmocAA27-36NH$_2$ (1.357 Kg in 107% yield and 87.IA% purity by HPLC (note 2).

The reaction may be reworked by trituration of the solid with 15 volumes of 9:1 acetonitrile/water with stirring for 12 hours, collection and drying.

### Notes:

1. In process control, TLC:
   88/12 dichloromethane/methanol
   UV, iodine detection
   Rf: FmocAA27-35OH, 0.49
   Rf: FmocAA27-36NH$_2$, 0.63
   Vydac C8, 5 m, 300A
   30°C, 1 mL/min, 262 nm
   A water/0.1% TFA
   B ACN/0.15 TFA
   80-99%B/20 minutes
   retention time: FmocAA27-35OH, 15.8
   retention time: FmocAA27-36NH$_2$, 17.12
2. A greater then theory yield is generally obtained unless the solid isolated is returned to the reactor and triturated with water.

### 11.6 Large Scale Preparation of Fragment HAA(27-36)-OH from FmocAA(27-36)-OH

A total of 3.897 Kg of HAA27-36NH$_2$ was prepared from 5.221 Kg of FmocAA27-36NH$_2$ in four runs with an average 2 step yield of 86.4%. The range in yield observed for the runs, 74-97%, presumably reflects carryover from one run into the next.

The work-up and product isolation in this stage works very well. The purity of the product is enhanced if the correct amount of DCM is left in the MTBE and the physical properties of the solid lead to rapid filtration and drying. This product isolation process has been incorporated into the new stage described in Section 11.6.2, below.

DCM is the solvent used during the deprotection of FmocAA27-36NH$_2$. At completion of the deprotection, the organic solution is washed with water twice to remove most of the excess piperidine, then concentrated to approximately one third of the original volume. MTBE is added to precipitate the fragment. The distillation is continued to remove the majority of the remaining DCM and complete the precipitation of the fragment. It is important to remove most of the DCM to avoid mass loss in the precipitation. It is also important to leave some DCM in the MTBE to ensure product cleanup. Dibenzofulvene, piperidine/fulvene adduct and residual piperidine are soluble in MTBE. The HAA27-36NH$_2$ is collected and dried. If necessary, a second trituration of the solid (12 hours) with MTBE will remove residual dibenzofulvene and more importantly the piperidine/fulvene adduct that may be present in the HAA27-36NH$_2$. A trituration with hexane will remove dibenzofulvene, but not the piperidine/fulvene adduct. Drying time of the solid isolated from MTBE is 1 day.

Due to the high solubility of HAA27-36NH$_2$ and related impurities in DCM/MTBE, an analytical method was desired to accurately determine the end point of the solvent exchange. A GC method has been developed to assay DCM in MTBE. The distillation is complete when the DCM content in the MTBE is below 6%.

### 11.6.1 Large Scale Preparation of Fragment HAA(27-36)-OH from FmocAA(27-36)-OH

A 40L glass jacketed reactor equipped with a mechanical stirrer and thermometer is charged with FmocAA27-36NH2 (1 eq., 1.356 Kg), DCM (5 vol) and piperidine (0.2 vol). The solution is stirred at ambient temperature for 1.5 hours (note 1). The organic solution is washed with water (2 x 5 vol). The volume of the organic layer is reduced to approximately one third the original volume by distillation (approximately 25 mm Hg, jacket temperature less than 45 °C to avoid melting the solid). MTBE (5 vol) is gradually introduced into the reaction vessel while concentration continues to a point of heavy precipitation and the pot volume is approximately 5 volumes. The solvent exchange is stopped when the DCM content is below 6%. The slurry is cooled to 0-5 °C, stirred for 60 minutes then collected by vacuum filtration (rapid). The solids are washed with MTBE (2 x 0.5 vol) and dried to constant weight to give 1.022Kg of HAA27-36NH2 in 89.2% yield (2 steps) and 91.1A% purity (note 1).

The reaction may be reworked by trituration with MTBE (12.5 vol) at 23 °C for 13 hours, collection by vacuum filtration and drying.

**Notes:**

1. IPC and purity measured by HPLC:
Vydac, C8, 5 , 300A
1 mL/min, 262 nm, 30°C
A water/0.1% TFA
B ACN/0.1% TFA
80-99%B/20 minutes
retention time: FmocAA27-36NH$_2$, 16.5. HAA27-36NH$_2$, 8.4

### 11.6.2 Improved Preparation of Fragment HAA(27-36)-OH by Solution Phase Coupling of FmocAA(27-36)-OH with HPheNH$_2$

A new process that combines Sections 11.5 through 11.6.1, that takes FmocAA27-35OH to HAA27-36NH$_2$ directly has been developed. This new process removes issues associated with isolating FmocAA27-36NH$_2$ and a long drying time. A detailed description is given below. The new process removes a long drying time and a stage from the synthetic route.

Add FmocAA27-35OH (5.0g, I eq), HOAT (0.459, 1.2 eq) and Phe-NH$_2$ (0.389, 1.2 eq) to a 100 mL round bottom flask equipped with a magnetic stirrer and nitrogen inlet. Add 10 volumes of NMP (50 mL) and DIEA (0.45g, 1.5 eq) to the flask and stir under a nitrogen atmosphere until the solids dissolve. Cool the solution to 0 to 5 °C then add HBTU (1 .04g, 1.2 eq). Stir under a nitrogen atmosphere at 0 to 5 °C for 30 minutes. Warm the reaction mixture to 20 °C and continue stirring. Perform an in process check (IPC) 90 minutes after the HBTU was added (note 1).

After the in process check (IPC) shows the reaction is complete, add piperidine (1.379, 7 eq) to the reaction mixture and stir under a nitrogen atmosphere for 1.5 hours. Perform an IPC (note 1). If the Fmoc removal is not complete, stir an additional 30 minutes and run the IPC.

When complete, add the reaction mixture to 5% aqueous acetic acid at a rate as to not exceed a temperature of 35 °C (30 vol). Stir the resulting slurry for I to 2 hours and collect by vacuum filtration (note 2). Wash the solid with 10 volumes (50 mL) of water.

Return the damp solid to the reactor, add 20 volumes of water (100 mL) and stir at ambient temperature for 1 to 2 hours. Collect the solids by vacuum filtration, wash with 10 volumes of water (50 mL) and dry (note 3).

The dried (note 4) solid is triturated with MTBE (20 vol) at ambient temperature for 2 to 5 hours to remove dibenzofulvene and dibenzofulvene piperidine adduct. Collect the solid by vacuum filtration and dry to constant weight yielding 4.55 grams (94.3%) of HAA27-36NH$_2$ with 85-90A% purity (note 1).

Fmoc by products (dibenzofulvene and its piperidine adduct) are generally removed in this protocol, however, if a rework is necessary, stir the solid in 10 volumes of MTBE at 20°C for 2 to 3 hours, collect and dry.

**Notes:**

1. In process check (IPC) by Reverse Phase HPLC:
Column Vydac C8, 300A, 5μ, 30°C
Flow rate 1 mL/min
Detection UV at 262 nm
Mobile phase A. water/0.1 % TFA
        B. acetonitrile/0.1% TFA

Method 80 to 99%B over 20 minutes
Retention Times:
FmocAA27-36NH$_2$ (16.5 min. HAA27-36NH$_2$ (8.4 min)
2. The total filtration time was 10 minutes..
3. The damp filter cake must be returned to the reactor for a water wash immediately to avoid oiling or gumming of the solids.
4. The crude product must dried completely to ensure that the MTBE trituration will remove the dibenzofulvene byproducts.

## 11.7 Large Scale Preparation of Fragment FmocAA(17-36)-OH by Solution Phase synthesis fusion of FmocAA (17-26)-OH with HAA(27-36)-OH

A total of 5.115 Kg of FmocAA17-36NH$_2$ was prepared from 2.993 Kg of HAA27-36NH$_2$ and 3.176 Kg of FmocAA17-26OH in four runs. The average yield was 84.3%. Filtering the crude solid after the water dropout from the reaction mixture took an average of 40-50 minutes.

A use test is run on a 1-2 g scale testing both fragments and the HBTU to be used prior to running the coupling reaction. The stoichiometry of starting materials and reagents is determined from the use test. The solubility of the starting materials is also noted in the use test. A cloudy solution may indicate the presence of salts in FmocAA17-26OH and warrant a water wash on the fragment. It is imperative that all components of the reaction mixture are clearly in solution prior to addition of the HBTU to ensure complete conversion of the starting materials to product with minimal racemization and byproduct formation.

The purity of crude FmocAA17-36NH$_2$ isolated from the water dropout is significantly enhanced by precipitation from IPA. FmocAA17-36NH$_2$ is partially soluble is IPA. Trituration of FmocAA17-36NH$_2$ with approximately 15 volumes of 95/5 IPA/water pre-warmed to 60-70 °C followed by stirring while cooling to room temperature over several hours enhances the purity of the fragment. Both starting materials as well as the piperidine amide of FmocAA17-26OH and enamine urea adduct of HAA27-36NH$_2$ are soluble in the 95% IPA. Trituration of the crude FmocAA17-36NH2 with 95% IPA at room temperature for extended periods of time is not as effective at removing impurities. A stability study at 60-70 °C has been completed. Failure to warm the IPA solution beyond 52 °C appears to have minimal impact on the quality of the product isolated.

## 11.7.1 Preferred Method for Improved Large Scale Preparation of Fragment FmocAA(17-36)-OH by Solution Phase Synthesis fusion of FmocAA(17-26)-OH with HAA(27-36)-OH

A 40L jacketed reactor fitted with a mechanical stirrer and nitrogen inlet is charged with FmocAA17-26OH (1 eq., 0.770 Kg), HAA27-36NH$_2$(1 eq., 0.720 Kg), HOAT (1.5 eq.) and DMF (12.5 volumes relative to FmocAA17-26OH). EtPr$_2$N (1.5 eq.) is added and the suspension is stirred at room temperature until the solids are dissolved (visually). The solution is cooled to 0-5°C under an atmosphere of nitrogen and HBTU (1-1.05 eq) is added. The reaction mixture is stirred at 0-5 °C until the HBTU dissolves (visually, about 15 minutes). The reaction mixture is warmed to 25 °C and stirred for 2 hours (note 1). The DMF solution is cooled to 0-5 °C and cold process water (12.5 vol) is added at such a rate as to not exceed 25 °C. The resulting slurry is stirred for 1-24 hours, the solids are collected by vacuum filtration and washed with water (3 x 4 vol). The solids are dried on the filter for 16-24 hours to approximately 2 times the theoretical mass. The 40 L reactor is charged with 95/5 isopropanol/water (25 vol) and warmed to 45°C. The semi-dry FmocAA17-36NH$_2$ is added to the IPA solution with rapid agitation. The suspension is warmed to 52 °C, then stirred for 12-16 hours as it cools to room temperature (note 2). The solids are collected by vacuum filtration, washed with a minimal amount of IPA and dried to give 1.261 Kg of FmocAA17-36NH$_2$ in 85% yield and 90.SA% purity by HPLC (note 1). The reaction may be reworked by repeating the 95/5 IPA/water trituration.

### Notes:

1. In process control and purity, HPLC
Vydac, C8, 5 μ, 300A
1 mL/min, 262 nm, 30°C
A water/0.1% TFA
B 80/20 IPA/ACN/0.1% TFA
60-95%B/20 minutes
Retention times: HAA27-36NH$_2$, 6.73 minutes

FmocAA17-26OH, 10.67 minutes

FmocAA17-36NH$_2$, 20.2 minutes

2. The FmocAA17-36NH$_2$ will not completely dissolve in this volume at this temperature. Before collecting the solids, stop agitation and allow the solids to settle. Remove a sample of the filtrate and analyze by HPLC. If the filtrate contains a significant amount of FmocAA17-36NH$_2$, cool to 0°C before collecting the solids.

### 11.8 Large Scale Preparation of Fragment HAA(17-36)-OH from FmoCAA(17-36)-OH

A total of 4.965 Kg of HAA17-36NH$_2$ was prepared from 5.112 Kg of FmocAA17-36NH$_2$ in four runs. Both the isolated yield and HPLC traces indicate the presence of dibenzofulvene and perhaps solvent. The dibenzofulvene present will not interfere with the next coupling reaction as it was removed with potassium carbonate, not piperidine, therefore, the piperidine adduct of dibenzofulvene is not an issue chemically.

There are several problems associated with this process. The reaction is run in 12 volumes of DMF. The base removing the Fmoc group is I volume of 1.1 M potassium carbonate, which is incapable of forming a difficult to remove, basic adduct with dibenzofulvene. The deprotection proceeds in about 90 minutes at room temperature.

A 1:1 saturated aqueous sodium chloride:water solution, precooled to 0 °C is added to co-precipitate the fragment and dibenzofulvene. This generates a fine, milky solid which takes hours (5-8) to filter. Once isolated, the damp solid has to be dried completely (less than 1% LOD) to remove the dibenzofulvene impurity. The drying takes 10 days (vacuum, no heat). Once dry, the solid is returned to the reactor and triturated with 3:1 heptane:MTBE (20 vol) at ambient temperature for 18 hours to remove the dibenzofulvene. Shorter trituration times do not remove it completely and if there is any water on the solids, it is not removed. A 3:1 heptane:MTBE (20 vol) rework can be used if dibenzofulvene persists.

To resolve these problems, the following new procedure was developed. Add FmocAA17-36NH$_2$ (2.0 g, 1 eq.) and heptane (8 vol) to a 100 mL round bottom flask equipped with a mechanical stirrer, temperature controller and reflux condenser. Add 2 volumes of MTBE (4 mL) and heat the slurry to 45-50°C (Note 1). Add the piperidine (1 0 eq.). Stir under a nitrogen atmosphere at 45-50°C for 24-36 hours (Note 2). Hot filter the reaction mixture at 45-50°C (Note 3), then wash the cake with 5 volumes (10 mL) of 60:40 heptane:MTBE.

### Notes:

1. Slower reaction will result if MTBE is allowed to escape the reactor thereby changing the heptane:MTBE ratio. Reaction temperatures greater than 50°C can result in gumming of the reaction solids. The Fmoc is largely removed in 5 hours.

2. Reaction completion is monitored by RP-HPLC:

| Column | Vydac C8, 300A, 5 , 30°C |
|---|---|
| Flow rate | 1 mL/min |
| Detection | UV at 262 nm |
| Mobile phase | A. water/0.1% TFA |
| | B. 80:20 IPA:CAN 0.1 %TFA |
| Method | 60 to 95%B over 30 minutes |

3. Hot filtration aids in the removal of the piperidine adduct of dibenzofulvene.

### 11.8.1 Large Scale Preparation of Fragment HAA(17-36)-OH from FmocAA(17-36)-OH

A 40L jacketed reactor is charged with FmocAA17-36NH$_2$ (1 eq, 1.26 Kg) and DMF (12 vol) at room temperature. A 1.11 M aqueous potassium carbonate solution (1 vol, 5 eq) is added at once (note 1). The reaction mixture is stirred at room temperature for 3.5 hours (note 2). The reaction mixture is added slowly to a 1:1 solution of saturated aqueous sodium chloride/water (10 volumes) pre-cooled to 0 °C at such a rate as to not exceed a temperature of 10 °C (approximately 1-2 hr). The solid is collected by vacuum filtration using a rubber dam to compress water from the filter cake (note 3). The wet cake is transferred back to the reaction vessel and triturated (agitated) with water (10 vol) for 1-2 hours to remove residual inorganic salts. The solid is collected by vacuum filtration, compressed with a rubber dam then transferred to a vacuum oven and dried to constant weight. The dry solid (note 4) is triturated (agitated) with 3:1 heptane:MTBE (20 vol) for a minimum of 14 hours at room temperature to remove dibenzofulvene. The solid is collected by vacuum filtration, washed with heptane (2 vol) and dried to give 1.20 Kg HAA17-36NH2 in 99.5% yield and 75A% purity. The HAA17-36NH2 contains 5A% dibenzofulvene. Rework the reaction by repeating the heptane: MTBE trituration.

**Notes :**

1. The solution turns cloudy when the aqueous potassium carbonate is added, but clears with continued stirring. An exotherm of 4-5 °C was noted.
2. HPLC used for IPC and purity.
Vydac C8, 260 nm
1 mL/min, 262 nm, 30 °C
A water/0.1% TFA
B 80:20 IPA/acetonitrile/0.1 % TFA
60-95%B/30 minutes.
3. The product precipitates with a fine particle size resulting in a slow filtration.
4. The material must be free of water for the heptane to effectively remove the dibenzylfulvene.

## 11.9 Large Scale Preparation of Fragment AcAA(1-36)-OH by Solution Phase synthesis from AcAA(1-16)-OH and FmocAA(17-36)-OH

The AcAA1-16OH was dissolved in DMF with HOAT and DIEA, cooled to 0°C and HBTU was added. This was stirred for 15 minutes, or until the HBTU dissolved, then HAA17-36NH$_2$ was added. Pre-activation of AcAA1-16OH in the absence of HAA17-36NH$_2$ was a precaution that later was found not to be necessary. In addition, dissolution of HAA17-36NH$_2$ into the 0°C DMF solution containing the activated AcAAI-160H proved to be slow on larger scale.

A total of 6.972 Kg of AcAA1-36NH$_2$ was prepared from 3.92 Kg of AcAA1-16OH and 4.96 Kg of HAA17-36NH$_2$ in four runs averaging 80.1% yield. Two runs in this stage averaged 87% yield and two runs averaged 73% yield.

A use test is run on a 1-2 g scale testing both fragments and the HBTU to be used prior to running the coupling reaction. The stoichiometry of starting materials and reagents is determined from the use test. The solubility of the starting materials is also noted in the use test. A cloudy solution may indicate the presence of salts in AcAA1-16OH and warrant a water wash on the fragment. It is imperative that all components of the reaction mixture are clearly in solution prior to addition of the HBTU to ensure complete conversion of the starting materials to product with minimal racemization and byproduct formation.

The fragments, AcAA1-16OH and HAA17-36NH$_2$, and reagents HOAT and DIEA were dissolved in DMF, cooled to 0 °C and HBTU is added. One of the runs required an additional equivalent of DIEA to pick up the HCl on HAA17-36NH$_2$. The crude product is isolated from the reaction mixture with a water dropout (filtration time, 10 minutes). The still damp solid was returned to a reactor containing acetonitrile pre-warmed to 55 °C, then stirred rapidly while cooling to 35 °C over 3 hours, then to 20 °C over night. The slurry is cooled to 0-5 °C, stirred an additional 1-2 hours and the solid is collected by filtration. During this process, the AcAA1-36NH$_2$ nearly goes into solution at 55 °C. As the solution cools, the AcAA1-36NH$_2$ precipitates (oils) out of solution. As the solution cools, the oil solidifies and eventually is transformed into a nice white solid. During the course of this transformation, considerable deposition of solid on the reactor wall and stir shaft may occur. Most of this falls off as the slurry is stirred overnight at 20°C. After collecting the solid, the reactor is filled with enough water to cover any solid sticking to the reactor walls or shaft. The slurry is stirred until all remaining solid has fallen off the walls and shaft (approximately 1 hour), then used as a wash on the filter cake. The acetonitrile wash removes unreacted starting materials and any truncated fragments less than 20 amino acids.

## 11.9.1 Preferred Large Scale Preparation of Fragment AcAA(1-36)-OH by Solution Phase Synthesis from AcAA (1-16)-OH and FmocAA(17-36)-OH

A 40 liter reactor is charged with AcAA1-160H (938 g, 1 eq), HAA17-36NH$_2$ (1.19 Kg, 1 eq.), HOAT (1 eq), DMF (19 vol, relative to AcAA1-16OH) and DIEA (1 eq.). The slurry is stirred until the solids dissolve, then cooled to 0-5 °C and HBTU (1.03 eq.) is added. The solution is stirred at 0-5 °C for 15 minutes or until the solid dissolves, warmed to 20 °C and stirred for 2 hours. The reaction mixture is sampled for an IPC (note 1). When the reaction is judged complete, water (19 vol) is added at such a rate as to not exceed 35 °C (note 2).

The slurry is stirred for 1-24 hours then the solid is collected by vacuum filtration (10 minutes) and washed with water (2 x 3 vol.). The filter cake is compressed to remove as much water as possible. Meanwhile, the reactor is charged with 95% acetonitrile/water (30 volumes relative to the AcAAI-160H charge) and warmed to 55 °C with stirring.

The damp solid is added to the reactor in portions to avoid clumping. The slurry is stirred while cooling to 35 °C over 3 hours, then to 20 °C overnight. Cool to 0-5 °C, stir for 2 hours, stop stirring and sample the solution.

Collect the solid by vacuum filtration. Wash the reactor and lines with 90% acetonitrile/water (3.6 vol).

Charge the reactor with a sufficient amount of water to cover any solids sticking to the reactor walls or stir shaft (approximately 30 vol). Stir at ambient temperature until the solids are no longer sticking to the reactor walls and shaft (approximately 1 hour). Collect the solid with the rest and dry to constant weight (1.83 Kg, 86% yield).

Repeat trituration on the dry solid using 90/10 acetonitrile/water.

**Notes:**

1. IPC and purity, HPLC
YMC ODS-A, 150 x 4.6 mm, 5 μ, 120A
1 mL/min, 260 nm, 30 °C
A water/0.1% TFA
B THF/0.1% TFA
60-90%B/30 min., 90-95%B/1 min., 95%B/5 min.
AcAA1-16OH   6.37 minutes
HAA17-36NH$_2$   8.91 minutes
AcAA1-36NH$_2$   18.07 minutes
2. If the temperature exceeds 35 °C during the water addition, the solids that precipitate out can melt leading to clumps and/or deposition on the reactor walls.

**11.10 Deprotection of Side Chains of AcAA(1-36)-OH**

It was the objective of this experiment to change the addition-rate of MTBE in the precipitation to keep the temperature less than 20°C and also isolate and decarboxylate crude T-20 solid (i.e. remove solution decarboxylation).

The crude T20 was isolated by precipitation from ACN/water as a solid prior to loading onto an HPLC column. The percent T20 content of the solid was determined by a weight based HPLC assay.

After the side-chain protecting groups have been removed in the 90/5/5 TFA/water/dithiothreitol, the solution is cooled to 0 °C and MTBE (45 vol. relative to AcAA1-36NH$_2$ charge) is added. This is a minimum amount of MTBE required to ensure complete precipitation of T-20. There is an exotherm on mixing and the first 5 volumes must be added slowly to keep the temperature below 20 °C (approximately 60 minutes). As more MTBE is added, the rate of addition can be increased. Keeping the temperature below 20 °C during the precipitation prevents clumping of the solid as it precipitates out of solution.

The current cleavage/purification protocol involves running the deprotection on scale that accommodates chromatographic purification. The crude T-20 isolated from the deprotection cocktail as a TFA salt was dissolved in acetonitrile/water at pH 5, filtered and allowed to stand for 15 hours to effect decarboxylation of the tryptophan indoles. After the decarboxylation was complete, the solution was diluted and transferred to the purification suite to be loaded onto the column. During the dilution, the solution always became hazy and the turbid solution was pumped onto the column. This resulted in deposition of solid onto the head of the column and gradual erosion of column performance during the purification runs. Allowing T-20 to decarboxylate in acetonitrile/water at pH 5 for several hours leads to formation of highly insoluble aggregates.

A method to avoid decarboxylation in solution has been developed. The crude T-20, isolated as a TFA salt from the MTBE precipitation, will decarboxylate under vacuum at room temperature over about 5-7 days. The decarboxylation was found to be complete in three days at 40°C under vacuum. The TFA content of the solid isolated was 9%. The material can be stored for up to one month at 0°C with 1% wt/wt loss.

A salt exchange in ethanol/water (1:9) with HOAc can be performed after isolating T-20-TFA from MTBE. The acetate salt of crude T-20 is significantly more stable and may be preferred. Either isolation method will allow the side-chain deprotection to be run on larger scale and the T-20 will not be exposed to the acetonitrile/water/HOAc conditions used for decarboxylation that are known to cause aggregation.

**New Method For Deprotection of Side Chains of AcAA(1-36)OH:**

Prepare a 90/5/5 TFA/water/dithiothreitol solution (13 vol) and purge with nitrogen for 3 minutes. AcAA1-36NH$_2$ is added in portions to 90/5/5 TFA/water/dithiothreitol (13 vol) at ambient temperature under an atmosphere of nitrogen. Once in solution, stir at ambient temperature for 4 hours then cool to 0 °C. To precipitate the T-20, MTBE (45 vol), cooled to 0-5 °C, is added drop-wise at a slow rate initially maintaining a temperature below 20 °C (approximately 1-2 hour addition time). The solid is collected by vacuum filtration (note 1). The reactor, lines and filter cake are immediately washed with 3 x 5 volumes of MTBE. The solid is removed, sampled for t = 0 IPC (note 2) and dried under vacuum for 5 days, or until HPLC shows no change.

**Notes:**

1. Avoid pulling air through the solid during this filtration as the deprotection solution is hygroscopic. Pulling air through solid before the TFA solution has been washed out can result in a tacky or oily solid.
2. Use HPLC method TM2-0003-01 (TFA method). The ammonium acetate method (TM2-0006-01) does not separate the various carboxylated intermediates.

**Deprotection of Side Chains of AcAA(1-36)OH**

A total of 7.226 Kg of AcAA1-36NH$_2$ was deprotected in 12 runs. Only 6.972Kg of AcAA1-36NH$_2$ was prepared suggesting that the intermediate may be hygroscopic. The solid isolated from the MTBE dropout was dissolved in 10 volumes of 1:1 ACN/water, filtered and adjusted to pH 3.5 with sodium bicarbonate. Acetic acid (1.5 volume %) was added (pH 4.5 to 5) and the solution was stirred at ambient temperature for 15 hours to effect decarboxylation. The solution was diluted with 25 volumes of water give a total of 40 volumes of an 85/15 water/ACN solution. The solution was turbid in all cases and in some, had a fine solid (aggregated T-20). A sample was taken for IPC and the solution was transferred to the purification suite. Crude T-20 yields for the runs were calculated using a wt/wt HPLC assay.

The deprotection of AcAA1-36NH$_2$ is complete in 90/5/5 TFA/water/DTT at room temperature after 4-5 hours. At 8 hours, noticeable degradation (2%) has occurred. In the same cocktail at 5°C, the deprotection is not complete at 8 hours, but is at 23 hours. There is no difference in the purity of the crude T-20 obtained after 5 hours at room temperature versus 23 hours at 5°C. Because the isolation volume (approximately 55 vol) is much larger then the reaction (deprotection) volume (13 vol), it was necessary to dissolve the AcAA1-36NH$_2$ in the TFA solution in a 20L carboy, then transfer the solution to a 40L reactor.

Isolation of the crude, carboxylated T-20 (as a TFA salt) from the deprotection cocktail is accomplished by precipitation with MTBE. The amount of MTBE needed to precipitate the peptide is 3-4 times the amount of deprotection cocktail. Using MTBE at twice the volume (or less) of the cleavage cocktail leaves peptide behind. Addition of the MTBE to the cleavage cocktail results in an easily filtered solid while adding the cocktail to MTBE produces a fine precipitate which is difficult to filter. There is a temperature rise from 5°C to 40-45°C during the addition of MTBE to the TFA solution. The temperature increase has no effect on the purity of the crude T-20 isolated (187/117,119). There was some clumping of the solids during the MTBE addition. The slurry was stirred for 1-2 hours to break up the clumps. Maintaining a temperature below 20°C during the MTBE addition produces a more uniform solid that filters better (196/31). The addition rate of the MTBE will be controlled in the future. Crude T-20 was collected by filtration under a nitrogen atmosphere. The solid will become tacky if exposed to moist air during the filtration before the TFA has been washed out. After the TFA is washed out, the solid is air stable.

**Method Run:**

A 90/5/5 TFA/water/dithiothreitol solution (13 vol) is prepared in a 20 L carboy then purged with nitrogen for 3 minutes. AcAA1-36NH$_2$ (650 g) is added in portions to prevent clumping. Once the solid is in solution, the solution is transferred to a 70L reactor. The carboy and lines are washed with a minimal amount of 90/5/5/TFA/water/dithiothreitol. The solution is stirred at ambient temperature under an atmosphere of nitrogen for 4 hours, then cooled to 0-5 °C. MTBE (45 vol) is added at a rate such that the internal temperature is less than 30 °C. The solid is collected by vacuum filtration under a stream of nitrogen. The reactor, lines and solid are washed with 2 x 2 volumes of MTBE and dried to constant weight (594 g, 70A%).

The solid (594 g) is dissolved in 50% ACN/water (8 volumes relative to the AcAA1-36H$_2$ charge) and filtered. The vessel and lines are washed with 50% ACN/water (2 vol). The pH of the solution is adjusted to 3.5-4.0 with sodium bicarbonate, then acetic acid (0.18 vol) is added bringing the pH to 4.9. The solution is stirred at ambient temperature for 15 hours (IPC, note 1), then the pH is adjusted to 9-9.5 with 1 M potassium carbonate. The solution is diluted to 85/15 water/ACN by adding water (25 vol). The resulting turbid solution is sampled for wt/wt HPLC analysis and transferred to the purification suite.

**Notes:**

1 Method TM2-0003-01 .
2 Method TM2-0006-01 .

**11.11 Purification of HAA(1-36)-OH**

Purification, lyophilization and packaging are classified in three stages:

$$\%wt/wt = 100 - \% \text{ impurities} - \% \text{ acetate} - \% \text{ water} - \%$$

$$\text{TFA.}$$

The acetate content in the batches was 6-8%.

A total of 2.08 Kg of T-20 (net) was isolated from 6.97 Kg of $AcAA1-36NH_2$. This represents a 49.3% yield from $AcAA1-36NH_2$. The average yield in the chromatographic purification of the was 55%. The yields for individual runs varied from 41-55%. The lowest yields were a result of column or pump failures leading to multiple passes. In general, if the chromatography worked, the yields were in the 50-55% range. The purity of the T-20 isolated was 93-95A%.

Four gradients were examined during purification for a comparison of methods with the objective of minimizing the run time:

1. 15-22% ACN/60 minutes, 22-36% ACN/525 minutes at 330 mL/min.
2. 16-26% ACN/60 minutes, 26-40% ACN/525 minutes at 330 mL/min.
3. Second Trimeris gradient. 15-36% ACN1788 minutes at 330 mL/min.
4. Original Trimeris gradient 20-23% ACN/1 12 minutes, 23-36% ACN/488 minutes at 330 mL/min.

A twenty centimeter (cm) column was packed (axial compression) with the Amberchrom resin (35 cm bed height). Batches of 500-700g AcAAl-36NH2 were deprotected and decarboxylated in solution. This scale produces a column feed stock that contains approximately 400 grams of peptide (approximately 75A% T-20). The stock solution is typically hazy and may contain suspended solid. The turbid solution is loaded onto the column at 500 mL/min using 15% B. There was no pressure build up during the column load for any of the runs. The flow rate is reduced to 330 mL/min and the specified gradient is run for the indicated amount of time. Fractions are collected and those containing over 78% T-20 are pooled (100-110L), diluted with water to bring the acetonitrile content to approximately 15-20% (approximately 140 liters). The column is washed, then equilibrated with 15% B. The T-20 containing solution is loaded back onto the 8 inch column at 900 mL/min. The %B is increased to 50% and T-20 is flushed of the column in approximately 25L.

The solution is frozen in one liter bell jars and lyophilized to a powder. The T20 isolated typically is 92-94A% by HPLC, and contains 6-8% acetate and 3-4% water.

**Preferred Method:**

A solution of T-20 in 85/15 water/acetonitrile (27L) at pH 9.2 was pumped onto an eight inch HPLC column at 500 mL/min. The Flow rate was reduced to 330 mL/min over 30 minutes (in increments of 30-40 mL/min every 5 minutes). A linear gradient going from 20%B to 36%B over 600 minutes is initiated. Fractions are collected until absorbance falls below 0.2 AUFS. The fractions are analyzed by HPLC (note 1) for content. The column is washed with 80%B at 900 mL/min for 10 minutes, then brought back to 15%B and equilibrated at 900 mL/min. The fractions containing over 78A% T-20 are pooled (113 L) and diluted with buffer (28.2 L) to bring the acetonitrile concentration to 15-20%. The solution is pumped onto the column at 900 mL/min. The %B is increased to 50% and T-20 is eluted off the column at 900 mL/min in approximately 25L. The concentration of T-20 is approximately 9-10 mg/mL. The solution is transferred to one liter lyophilization jars, frozen and freeze dried to give 216.29 of T-20 in 54.9% yield and 94.1A% purity.

**Notes:**

1. Fractions are analyzed by HPLC for content and purity.
YMC ODS-A, 150 x 4.6 mm, 5 µm, 120A
1 mL/min, 220 nm, 30°C
A 70/30 50 mM NH$_4$OAc @ pH 7 (adjust with NH$_4$OH):ACN
B 5:95 50 mM NH$_4$OAc @ pH 7 (adjust with NH$_4$OH):ACN
0-15%B/50 min., 15-100%B/2 min. 100%B/3 min.
T-20 retention time, 29.0 minutes.

**11.12 Thermal Stability of Peptides**

A series of experiments were conducted at to characterize the thermal stability of the intermediate T-20 fragments. T-20 fragments were saturated with water, filtered and then placed in enclosed containers and exposed to elevated temperatures. At several time points samples were collected and analyzed by HPLC methods to access the purity changes. Stability of the dry T-20 fragments was also characterized by thermogravimetric analysis (TGA).

All of the fragments can be dried at 40 °C for 3 days but AcAA1-16OH. Only modest degradation is observed after 24 hours at 80 °C in two fragments, FmocAA27-35OH (3%) and FmocAA27-36NH$_2$ (1.4%). Fragment AcAA1-16OH was initially 97A% pure. After 3 days at 40 °C, it was 93.4A% pure and dry. After an additional 24 hours at 80 °C it remained at 93.9A%. The damp AcAA1-16OH should be dried at ambient temperature.

The point of the stability study was to determine if the fragments can be dried at 40 °C rather then ambient temperature. The study indicates that the fragments are stable to drying at 40 °C. This will greatly reduce the drying times.

A study designed to examine the stability of fragments Ac(1-16)-OH, Fmoc(27-35)-OH, Fmoc(17-26)-OH, Fmoc(27-36)-NH$_2$, Fmoc(17-36)-NH$_2$, H(17-36)-NH$_2$, and Ac(1-36)-NH$_2$ under various solvent and temperature conditions that mimic their isolation and purification has been completed. All of the fragments are stable, stability is designated with an X, in the solvent systems from which they are isolated and/or purified:

| | | Time | | | | |
| Intermediate | Storage solution | Initial | 1 hour | 6 hour | 1 day | 3 day |
|---|---|---|---|---|---|---|
| F(27-35)-OH | 9:1 EtOH:H$_2$O, 40°C | X | X | X | X | X |
| F(17-26)-OH | 80:20 EtOH/H$_2$O, 40°C | X | X | X | X | X |
| F(27-36)-NH$_2$ | 1:1 NMP/H$_2$O, 40°C | X | X | X | X | X |
| Ac(1-16)-OH | 0.01 M HCl, 5-10°C | X | X | X | X | X |
| F(17-36)-NH$_2$ | 95% IPA/H$_2$O, 60-65°C | X | X | X | X | X |
| | 50%H$_2$O/NMP, 50°C | X | X | X | X | X |
| | 90:10 EtOH/H$_2$O, 40°C | X | X | X | X | X |
| H(17-36)-NH$_2$ | 1.5:1 H2O/DMF, 40°C | X | X | X | X | X |
| Ac(1-36)-NH$_2$ | 9:1 ACN/H$_2$O, 50°C | X | X | X | X | X |
| | 50%H$_2$O/NMP, 50°C | X | X | X | X | X |
| | 85:15 EtOH:H$_2$O, 70°C | X | X | X | X | X |
| X = HPLC content and impurities, visual appearance check | | | | | | |

**Claims**

**1.** A method for the synthesis of a peptide having the formula

Ac-YTSLIHSLIEESQNQQEKNEQELLELDKWASLWNWF-NH$_2$ (SEQ ID NO:1),

comprising:

(a) reacting a side-chain protected peptide of the formula:

Fmoc-EKNEQELLEL-COOH (SEQ ID NO:11)

with a side chain-protected peptide of the formula:

$NH_2$-DKWASLWNWF-$NH_2$ (SEQ ID NO:18)

to yield a side-chain protected peptide of the formula:

Ac-YTSLIHSLIEESQNQQEKNEQELLELDKWASLWNWF-$NH_2$ (SEQ ID NO:1),

(b) deprotecting the amino terminus of the peptide produced in (a);
(c) reacting the peptide produced in (b) with a side-chain protected peptide of the formula:

Fmoc-YTSLIHSLIEESQNQQ-COOH (SEQ ID NO:4)

to yield a side-chain protected peptide of the formula:

Fmoc-YTSLIHSLIEESQNQQEKNEQELLELDKWASLWNWF-$NH_2$
(SEQ ID NO:1);

(d) modifying the amino terminus of the peptide produced in (c) into an acetyl modification; and
(e) deprotecting the side chains of the side-chain protected peptide of (d) to yield a peptide of the formula:

Ac-YTSLIHSLIEESQNQQEKNEQELLELDKWASLWNWF-$NH_2$
(SEQ ID NO:1).

2. A method for the synthesis of a peptide having the formula

Ac-YTSLIHSLIEESQNQQEKNEQELLELDKWASLWNWF-$NH_2$ (SEQ ID NO:1),

comprising:

(a) reacting a side-chain protected peptide of the formula:

Fmoc-EKNEQELLEL-COOH (SEQ ID NO:11)

with a side chain-protected peptide of the formula:

$NH_2$-DKWASLWNWF-$NH_2$ (SEQ ID NO:18)

to yield a side-chain protected peptide of the formula:

Fmoc-EKNEQELLELDKWASLWNWF-NH$_2$ (SEQ ID NO:12);

(b) deprotecting the amino terminus of the peptide produced in (a);

(c) reacting the peptide produced in (b) with a side-chain protected peptide of the formula:

Ac-YTSLIHSLIEESQNQQ-COOH (SEQ ID NO:4)

to yield a side-chain protected peptide of the formula:

Ac-YTSLIHSLIEESQNQQEKNEQELLELDKWASLWNWF-NH$_2$
(SEQ ID NO:1); and

(d) deprotecting the side chains of the side-chain protected peptide of (c) to yield a peptide of the formula:

Ac-YTSLIHSLIEESQNQQEKNEQELLELDKWASLWNWF-NH$_2$
(SEQ ID NO:1).

3. A method for the synthesis of a peptide having the formula:

Ac-YTSLIHSLIEESQNQQEKNEQELLELDKWASLWNWF-NH$_2$ (SEQ ID NO:1),

comprising:

(a) deprotecting the amino terminus of a side-chain protected peptide of the formula:

Fmoc-EKNEQELLELDKWASLWNWF-NH$_2$ (SEQ ID NO:12);

(b) reacting the peptide produced in (a) with a side-chain protected peptide of the formula:

Fmoc-YTSLIHSLIEESQNQQ-COOH (SEQ ID NO:4),

to yield a side-chain protected peptide of the formula:

Fmoc-YTSLIHSLIEESQNQQEKNEQELLELDKWASLWNWF-NH$_2$
(SEQ ID NO:1);

(c) deprotecting the amino terminus of the peptide produced in (b);

(d) modifying the amino terminus of the peptide produced in (c) into an acetyl modification; and

(e) deprotecting the side chains of the side-chain protected peptide of (c) to yield a peptide of the formula:

Ac-YTSLIHSLIEESQNQQEKNEQELLELDKWASLWNWF-NH$_2$
(SEQ ID NO:1).

4. A method for the synthesis of a peptide having the formula:

Ac-YTSLIHSLIEESQNQQEKNEQELLELDKWASLWNWF-NH$_2$ (SEQ ID NO:1),

comprising:

(a) deprotecting the amino terminus of a side-chain protected peptide of the formula:

Fmoc-EKNEQELLELDKWASLWNWF-NH$_2$ (SEQ ID NO:12);

(b) reacting the peptide produced in (a) with a side chain protected peptide of the formula:

Ac-YTSLIHSLIEESQNQQ-COOH (SEQ ID NO:4),

to yield a side-chain protected peptide of the formula:

Ac-YTSLIHSLIEESQNQQEKNEQELLELDKWASLWNWF-NH$_2$
(SEQ ID NO:1);

(c) deprotecting the side chains of the side-chain protected peptide of (b) to yield a peptide of the formula:

Ac-YTSLIHSLIEESQNQQEKNEQELLELDKWASLWNWF-NH$_2$
(SEQ ID NO:1).

5. The method of claim 3 or 4 wherein the side-chain protected peptide of the formula: Fmoc-EKNEQELLELDK-WASLWNWF-NH$_2$ (SEQ ID NO:12) is synthesized by a method comprising:

(a) reacting a side-chain protected peptide of the formula:

Fmoc-DKWASLWNW-COOH (SEQ ID NO:17)

with HPheNH$_2$ to yield a side-chain protected peptide of the formula:

Fmoc-DKWASLWNWF-NH$_2$ (SEQ ID NO:18);

(b) deprotecting the amino terminus of the peptide produced in (a); and
(c) reacting the peptide produced in (b) with a side-chain protected peptide of the formula:

Fmoc-EKNEQELLEL-COOH (SEQ ID NO:11)

to yield the side-chain protected peptide of the formula:

Fmoc-EKNEQELLELDKWASLWNWF-NH$_2$ (SEQ ID NO:12).

6. The method of claim 3 or 4 wherein the side-chain protected peptide of the formula: Fmoc-EKNEQELLELDK-WASLWNWF-NH$_2$ (SEQ ID NO:12) is synthesized by a method comprising:

(a) reacting a side-chain protected peptide of the formula:

Fmoc-LELDKWASLWNW-COOH (SEQ ID NO:15)

with HPheNH$_2$ to yield a side-chain protected peptide of the formula:

Fmoc-LELDKWASLWNWF-NH$_2$ (SEQ ID NO:16);

(b) deprotecting the amino terminus of the peptide produced in (a); and
(c) reacting the peptide produced in (b) with a side-chain protected peptide of the formula:

Fmoc-EKNEQEL-COOH (SEQ ID NO:10)

to yield the side-chain protected peptide of the formula:

Fmoc-EKNEQELLELDKWASLWNWF-NH$_2$ (SEQ ID NO:12).

7. The method of claim 3 or 4 wherein the side-chain protected peptide of the formula: Fmoc-EKNEQELLELDK-WASLWNWF-NH$_2$ (SEQ ID NO:12) is synthesized by a method comprising:

(a) deprotecting the amino terminus of the peptide:

Fmoc-DKWASLWNWF-NH$_2$ (SEQ ID NO:18);

and
(b) reacting the peptide produced in (a) with a side-chain protected peptide of the formula:

Fmoc-EKNEQELLEL-COOH (SEQ ID NO:11)

to yield the side-chain protected peptide of the formula:

Fmoc-EKNEQELLELDKWASLWNWF-NH$_2$ (SEQ ID NO:12).

8. The method of claim 3 or 4 wherein the side-chain protected peptide of the formula: Fmoc-EKNEQELLELDK-WASLWNWF-NH$_2$ (SEQ ID NO:12) is synthesized by a method comprising:

(a) reacting a side-chain protected peptide of the formula:

Fmoc-EKNEQELLELDKWASLWNW-COOH (SEQ ID NO:19)

with HPheNH$_2$ to yield the side-chain protected peptide of the formula:

Fmoc-EKNEQELLELDKWASLWNWF-NH$_2$ (SEQ ID NO:12).

9. The method of Claim 1 or 3 wherein the side-chain protected peptide of the formula: Fmoc-YTSLIHSLIEESQNQQ-COOH (SEQ ID NO:4) is synthesized by a method comprising:

(a) deprotecting the amino terminus of the side-chain protected peptide of the formula:

Fmoc-IEESQNQQ-COOH (SEQ ID NO:7);

(b) reacting the side-chain protected peptide produced in (a) with a side-chain protected peptide of the formula:

Fmoc-YTSLIHSL-COOH (SEQ ID NO:2)

to yield the side-chain protected peptide of the formula:

Fmoc-YTSLIHSLIEESQNQQ-COOH (SEQ ID NO:4).

**10.** The method of Claim 2 or 4 wherein the side-chain protected peptide of the formula: Ac-YTSLIHSLIEESQNQQ-COOH (SEQ ID NO:4) is synthesized by a method comprising:

(a) reacting a side-chain protected peptide of the formula:

Fmoc-IEESQNQ-COOH (SEQ ID NO:6)

with HGlnOPNB to yield a side-chain protected peptide of the formula:

Fmoc-IEESQNQQ-OPNB (SEQ ID NO:7);

(b) deprotecting the amino terminus of the side-chain protected peptide produced in (a);
(c) reacting the side-chain protected peptide produced in (b) with a side-chain protected peptide of the formula:

Ac-YTSLIHSL-COOH (SEQ ID NO:2)

to yield a side-chain protected peptide of the formula:

Ac-YTSLIHSLIEESQNQQ-OPNB (SEQ ID NO:4);

(d) deprotecting the carboxyl terminus of the side-chain protected peptide produced in (c) to yield the side-chain protected peptide of the formula:

Ac-YTSLIHSLIEESQNQQ-COOH (SEQ ID NO:4).

**11.** The method of claim 1 or 3 wherein the side-chain protected peptide of the formula: Fmoc-YTSLIHSLIEESQNQQ-COOH (SEQ ID NO:4) is synthesized by a method comprising:

(a) reacting a side-chain protected peptide of the formula:

Fmoc-YTSLIHSLIEESQNQ-COOH (SEQ ID NO:3)

with HGlnOPNB to yield the side-chain protected peptide of the formula:

Fmoc-YTSLIHSLIEESQNQQ-OPNB (SEQ ID NO:4);

and
b) deprotecting the carboxyl terminus of the peptide produced in (a) to yield the side-chain protected peptide of the formula:

Fmoc-YTSLIHSLIEESQNQQ-COOH (SEQ ID NO:4).

12. A method for the synthesis of a peptide of the formula:

Ac-YTSLIHSLIEESQNQQEKNEQELLELDKWASLWNWF-NH$_2$ (SEQ ID NO:1),

comprising:

(a) deprotecting the amino terminus of a side-chain protected peptide of the formula: Fmoc-QEKNEQELLELD-KWASLWNWF-NH$_2$ (SEQ ID NO:9);
(b) reacting the peptide produced in (a) with a side-chain protected peptide of the formula: Fmoc-YTSLIHSLIEESQNQ-COOH (SEQ ID NO:3), to yield a side-chain protected peptide of the formula:

Fmoc-YTSLIHSLIEESQNQQEKNEQELLELDKWASLWNWF-NH$_2$
(SEQ ID NO:1);

(c) modifying the amino terminus of the peptide produced in (b) into an acetyl modification; and
(d) deprotecting the side chains of the side-chain protected peptide of (c) to yield the peptide of the formula:

Ac-YTSLIHSLIEESQNQQEKNEQELLELDKWASLWNWF-NH$_2$
(SEQ ID NO:1).

13. The method of claim 12 wherein the side-chain protected peptide of the formula: Fmoc-QEKNEQELLELDK-WASLWNWF-NH$_2$ (SEQ ID NO:9), is synthesized by a method comprising:

(a) reacting a side-chain protected peptide of the formula:

Fmoc-QEKNEQELLELDKWASLWNW-NH$_2$ (SEQ ID NO:8)

with HPheNH$_2$ to yield the side-chain protected peptide of the formula:

Fmoc-QEKNEQELLELDKWASLWNWF-NH$_2$ (SEQ ID NO:9).

**14.** A method for the synthesis of a peptide of the formula:

Ac-YTSLIHSLIEESQNQQEKNEQELLELDKWASLWNWF-NH$_2$ (SEQ ID NO:1),

comprising:

(a) deprotecting the amino terminus of a side-chain protected peptide of the formula: Fmoc-NEQELLELDK-WASLWNWF-NH$_2$ (SEQ ID NO:14);
(b) reacting the peptide produced in (a) with a side-chain protected peptide of the formula: F-moc-YTSLIHSLIEESQNQQEK-COOH (SEQ ID NO:5),
to yield a side-chain protected peptide of the formula:

Fmoc-YTSLIHSLIEESQNQQEKNEQELLELDKWASLWNWF-NH$_2$

(SEQ ID NO:1);

(c) modifying the amino terminus of the peptide produced in (b) into an acetyl modification; and
(d) deprotecting the side chains of the side-chain protected peptide of (c) to yield the peptide of the formula:

Ac-YTSLIHSLIEESQNQQEKNEQELLELDKWASLWNWF-NH$_2$

(SEQ ID NO:1).

**15.** The method of Claim 14 wherein the side-chain protected peptide of the formula: Fmoc-NEQELLELDKWASLWN-WF-NH$_2$ (SEQ ID NO:14), is synthesized by a method comprising:

(a) reacting a side-chain protected peptide of the formula:

Fmoc-NEQELLELDKWASLWNW-NH$_2$ (SEQ ID NO:13)

with HPheNH$_2$ to yield the side-chain protected peptide of the formula:

Fmoc-NEQELLELDKWASLWNWF-NH$_2$ (SEQ ID NO:14).

**16.** The method of claim 1, 2, 3, 4, 12, or 14 wherein the side-chain protected peptide of the formula: Ac-YTSLIHSLIEESQNQQEKNEQELLELDKWASLWNWF-NH$_2$ (SEQ ID NO:1) is produced in an amount of about one or more kilograms.

**17.** The method of claim 1 or 2 wherein the side-chain protected peptide of the formula: Fmoc-EKNEQELLELDK-WASLWNWF-NH$_2$ (SEQ ID NO:12) is produced in an amount of about one or more kilograms.

**18.** The method of claim 5 wherein the side-chain protected peptide of the formula: Fmoc-EKNEQELLELDKWASLWN-WF-NH$_2$ (SEQ ID NO:12) is produced in an amount of about one or more kilograms.

**19.** The method of claim 6 wherein the side-chain protected peptide of the formula: Fmoc-EKNEQELLELDKWASLWN-WF-NH$_2$ (SEQ ID NO:12) is produced in an amount of about one or more kilograms.

**20.** The method of claim 7 wherein the side-chain protected peptide of the formula: Fmoc-EKNEQELLELDKWASLWN-WF-NH$_2$ (SEQ ID NO:12) is produced in an amount of about one or more kilograms.

**21.** The method of claim 8 wherein the side-chain protected peptide of the formula: Fmoc-EKNEQELLELDKWASLWN-

WF-NH$_2$ (SEQ ID NO:12) is produced in an amount of about one or more kilograms.

**22.** The method of claim 9 wherein the side-chain protected peptide of the formula: Fmoc-YTSLIHSLIEESQNQQ-COOH (SEQ ID NO:4) is produced in an amount of about one or more kilograms.

**23.** The method of claim 10 wherein the side-chain protected peptide of the formula: Ac-YTSLIHSLIEESQNQQ-COOH (SEQ ID NO:4) is produced in an amount of about one or more kilograms.

**24.** The method of claim 11 wherein the side-chain protected peptide of the formula: Fmoc-YTSLIHSLIEESQNQQ-COOH (SEQ ID NO:4) is produced in an amount of about one or more kilograms.

**25.** The method of claim 13 wherein the side-chain protected peptide of the formula: Fmoc-QEKNEQELLELDK-WASLWNWF-NH$_2$ (SEQ ID NO:9) is produced in an amount of about one or more kilograms.

**26.** The method of claim 15 wherein the side-chain protected peptide of the formula: Fmoc-NEQELLELDKWASLWN-WF-NH$_2$ (SEQ ID NO:14) is produced in an amount of about one or more kilograms.

**27.** A set of peptide fragments comprising a set selected from the group consisting of:

(a)

YTSLIHSLIEESQNQQ (SEQ ID NO:4),

EKNEQELLELDKWASLWNWF (SEQ ID NO:12);

(b)

YTSLIHSLIEESQNQQ (SEQ ID NO:4),

EKNEQELLEL (SEQ ID NO:11),

DKWASLWNWF (SEQ ID NO:18);

(c)

YTSLIHSLIEESQNQQ (SEQ ID NO:4),

EKNEQELLEL (SEQ ID NO:11),

DKWASLWNW (SEQ ID NO:17);

(d)

YTSLIHSL (SEQ ID NO:2),

IEESQNQ (SEQ ID NO:6),

EKNEQELLELDKWASLWNWF (SEQ ID NO:12);

(e)

YTSLIHSL (SEQ ID NO:2),

IEESQNQ (SEQ ID NO:6),

EKNEQELLEL (SEQ ID NO:11),

DKWASLWNWF (SEQ ID NO:18);

(f)

YTSLIHSL (SEQ ID NO:2),

IEESQNQ (SEQ ID NO:6),

EKNEQELLEL (SEQ ID NO:11),

DKWASLWNW (SEQ ID NO:17);

(g)

YTSLIHSL (SEQ ID NO:2),

IEESQNQQ (SEQ ID NO:7),

EKNEQELLELDKWASLWNWF (SEQ ID NO:12);

(h)

YTSLIHSL (SEQ ID NO:2),

IEESQNQQ (SEQ ID NO:7),

EKNEQELLEL (SEQ ID NO:11),

DKWASLWNWF (SEQ ID NO:18);

(i)

YTSLIHSL (SEQ ID NO:2),

IEESQNQQ (SEQ ID NO:7),

EKNEQELLEL (SEQ ID NO:11),

DKWASLWNW (SEQ ID NO:17);

(j)

YTSLIHSLIEESQNQQ (SEQ ID NO:4),

EKNEQEL (SEQ ID NO:10),

LELDKWASLWNWF (SEQ ID NO:16);

(k)

YTSLIHSLIEESQNQQ (SEQ ID NO:4),

EKNEQEL (SEQ ID NO:10),

LELDKWASLWNW (SEQ ID NO:15);

(l)

YTSLIHSL (SEQ ID NO:2),

IEESQNQ (SEQ ID NO:6),

EKNEQEL (SEQ ID NO:10),

LELDKWASLWNWF (SEQ ID NO:16);

(m)

YTSLIHSL (SEQ ID NO:2),

IEESQNQ (SEQ ID NO:6),

EKNEQEL (SEQ ID NO:10),

LELDKWASLWNW (SEQ ID NO:15);

(n)

YTSLIHSL (SEQ ID NO:2),

IEESQNQQ (SEQ ID NO:7),

EKNEQEL (SEQ ID NO:10),

LELDKWASLWNWF (SEQ ID NO:16);

(o)

YTSLIHSL (SEQ ID NO:2),

IEESQNQQ (SEQ ID NO:7),

EKNEQEL (SEQ ID NO:10),

LELDKWASLWNW (SEQ ID NO:15);

(p)

YTSLIHSLIEESQNQ (SEQ ID NO:3),

QEKNEQELLELDKWASLWNW (SEQ ID NO:8);

(q)

YTSLIHSLIEESQNQ (SEQ ID NO:3),

QEKNEQELLELDKWASLWNWF (SEQ ID NO:9);

(r)

YTSLIHSLIEESQNQQEK (SEQ ID NO:5),

NEQELLELDKWASLWNWF (SEQ ID NO:14);

(s)

YTSLIHSLIEESQNQQEK (SEQ ID NO:5),

NEQELLELDKWASLWNW (SEQ ID NO:13);

and
(t)

YTSLIHSLIEESQNQQ (SEQ ID NO:4),

EKNEQELLELDKWASLWNW (SEQ ID NO:19).

28. The use of a peptide fragment for peptide synthesis according to any of claims 1-26, wherein the peptide fragment is selected from the group consisting of:

YTSLIHL (SEQ ID NO:2);

IEESQNQ (SEQ ID NO:6);

IEESQNQQ (SEQ ID NO:7);

QEKNEQELLELDKWASLWNW (SEQ ID NO:8);

EKNEQEL (SEQ ID NO:10);

EKNEQELLEL (SEQ ID NO:11);

NEQELLELDKWASLWNW (SEQ ID NO:13);

LELDKWASLWNW (SEQ ID NO:15);

LELDKWASLWNWF (SEQ ID NO:16);

DKWASLWNW (SEQ ID NO:17);

DKWASLWNWF (SEQ ID NO:18);

and

EKNEQELLELDKWASLWNW (SEQ ID NO:19).

**29.** A protected peptide selected from the group consisting of:

Ac-YTSLIHSL-COOH (SEQ ID NO:2);

FMOC-YTSLIHSL-COOH (SEQ ID NO:2);

Ac-YTSLIHSLIEESQNQQ-OPNB (SEQ ID NO:4);

FMOC-YTSLIHSLIEESQNQQ-OPNB (SEQ ID NO:4);

Ac-YTSLIHSLIEESQNQQ-COOH (SEQ ID NO:4);

FMOC-YTSLIHSLIEESQNQQ-COOH (SEQ ID NO:4);

Ac-IEESQNQ-COOH (SEQ ID NO:6);

FMOC-IEESQNQ-COOH (SEQ ID NO:6);

$NH_2$-IEESQNQQ-OPNB (SEQ ID NO:7);

FMOC-IEESQNQQ-OPNB (SEQ ID NO:7);

Ac-EKNEQEL-COOH (SEQ ID NO:10);

FMOC-EKNEQEL-COOH (SEQ ID NO:10);

Ac- EKNEQELLEL-COOH (SEQ ID NO:11);

FMOC-EKNEQELLEL-COOH (SEQ ID NO:11);

NH$_2$-EKNEQELLELDKWASLWNWF-NH$_2$ (SEQ ID NO:12);

FMOC-EKNEQELLELDKWASLWNWF-NH$_2$ (SEQ ID NO:12);

Ac-LELDKWASLWNW-COOH (SEQ ID NO:15);

FMOC-LELDKWASLWNW-COOH (SEQ ID NO:15);

NH$_2$-LELDKWASLWNWF-NH$_2$ (SEQ ID NO:16);

FMOC-LELDKWASLWNWF-NH$_2$ (SEQ ID NO:16);

Ac-DKWASLWNW-COOH (SEQ ID NO:17);

FMOC-DKWASLWNW-COOH (SEQ ID NO:17);

NH$_2$-DKWASLWNWF-NH$_2$ (SEQ ID NO:18);

FMOC-DKWASLWNWF-NH$_2$ (SEQ ID NO:18);

and

FMOC-EKNEQELLELDKWASLWNW-COOH (SEQ ID NO:19).

**Patentansprüche**

1. Verfahren zur Herstellung eines Peptids mit der Formel Ac-YTSLIHSLIEESQNQQEKNEQELLELDKWASLWN-WF-NH$_2$ (SEQ ID NO:1), umfassend:

(a) reagieren eines Seitenketten-geschützten Peptids der Formel:
Fmoc-EKNEQELLEL-COOH (SEQ ID NO:11) mit einem Seitenketten-geschützten Peptid der Formel:
NH$_2$-DKWASLWNWF-NH$_2$ (SEQ ID NO:18) um ein Seitenketten-geschütztes Peptid der Formel:
Fmoc-EKNEQELLELDKWASLWNWF-NH$_2$ (SEQ ID NO : 12) zu erhalten;
(b) entfernen der Schutzgruppe vom Aminoterminus des in (a) hergestellten Peptids;
(c) reagieren des in (b) hergestellten Peptids mit einem Seitenketten-geschützten Peptid der Formel:

Fmoc-YTSLIHSLIEESQNQQ-COOH (SEQ ID NO:4)

um ein Seitenketten-geschütztes Peptid der Formel:
Fmoc-YTSLIHLIEESQNQQEKNEQELLELDKWASLWN WF-NH$_2$, (SEQ ID NO:1) zu erhalten;
(d) umwandeln des Aminoterminus des in (c) hergestellten Peptids in eine Acetylmodifikation; und

(e) entfernen der Schutzgruppen der Seitenketten des Seitenketten-geschützten Peptids aus (d), um ein Peptid mit der Formel: Ac-YTSLIHSLIEESQNQQEKNEQELLELDKWASLWNWF -NH$_2$ (SEQ ID NO:1), zu erhalten.

2. Verfahren zur Herstellung eines Peptids der Formel Ac-YTSLIHSLIEESQNQQEKNEQELLELDKWASLWNWF-NH$_2$ (SEQ ID NO:1), umfassend:

(a) reagieren eines Seitenketten-geschützten Peptids der Formel:

$$\text{Fmoc-EKNEQELLEL-COOH (SEQ ID NO:11)}$$

mit einem Seitenketten-geschützten Peptid der Formel:
NH$_2$-DKWASLWNWF-NH$_2$ (SEQ ID NO:18), um ein Seitenketten-geschütztes Peptid der Formel:
Fmoc-EKNEQELLELDKWASLWNWF-NH$_2$ (SEQ ID NO : 12) zu erhalten;
(b) entfernen der Schutzgruppe des Aminoterminus des in (a) erzeugten Peptids;
(c) reagieren des in (b) hergestellten Peptids mit einem Seitenketten-geschützten Peptid der Formel:
Fmoc-YTSLIHSLIEESQNQQ-COOH (SEQ ID NO:4), um ein Seitenketten-geschütztes Peptid der Formel
Ac-YTSLIHSLIEESQNQQEKNEQELLELDKWASLWNWF -NH$_2$ (SEQ ID NO:1) zu erhalten; und
(d) entfernen der Schutzgruppe(n) der Seitenketten des Seitenketten-geschützten Peptids aus (c), um ein Peptid der Formel:
Ac-YTSLIHSLIEESQNQQEKNEQELLELDKWASLWNWF -NH$_2$ (SEQ ID NO:1) zu erhalten;

3. Verfahren zur Herstellung eines Peptids der Formel:
Ac-YTSLIHSLIEESQNQQEKNEQELLELDKWASLWNWF-NH$_2$ (SEQ ID NO:1) umfassend;

(a) entfernen der Schutzgruppen des Aminoterminus des Seitenketten-geschützten Peptids der Formel:

$$\text{Fmoc-EKNEQELLELDKWASLWNWF-NH}_2 \text{ (SEQ ID NO:12);}$$

(b) reagieren des in (a) hergestellten Peptids mit einem Seitenketten-geschützten Peptid der Formel:

$$\text{Fmoc-YTSLIHSLIEESQNQQ-COOH (SEQ ID NO:4),}$$

um ein Seitenketten-geschütztes Peptid der Formel
Ac-YTSLIHSLIEESQNQQEKNEQELLELDKWASLWNWF -NH$_2$ (SEQ ID NO:1) zu erhalten;
(c) entfernen der Schutzgruppen des Aminoterminus des Peptids des (b) hergestellten Peptids;
(d) umwandeln des Aminoterminus des in (c) hergestellten Peptids in eine Acetylmodifikation; und
(e) entfernen der Schutzgruppe(n) der Seitenketten des Seitenketten-geschützten Peptids aus (c), um ein Peptid der Formel:
Ac-YTSLIHSLIEESQNQQEKNEQELLELDKWASLWNWF -NH$_2$ (SEQ ID NO:1) zu erhalten;

4. Verfahren für die Herstellung eines Peptids der Formel Ac-YTSLIHSLIEESQNQQEKNEQELLELDKWASLWNWF-NH$_2$ (SEQ ID NO:1) umfassend:

(a) entfernen der Schutzgruppen des Aminoterminus des Seitenketten-geschützten Peptids der Formel:
Fmoc-EKNEQELLELDKWASLWNWF-NH$_2$ (SEQ ID NO : 12);
(b) reagieren des in (a) hergestellten Peptids mit einem Seitenketten-geschützten Peptids der Formel:
Fmoc-YTSLIHSLIEESQNQQ-COOH (SEQ ID NO:4), um ein Seitenketten-geschütztes Peptid der Formel:
Ac-YTSLIHSLIEESQNQQEKNEQELLELDKWASLWNWF -NH$_2$ (SEQ ID NO:1) zu erhalten;
(c) entfernen der Schutzgruppen der Seitenketten des Seitenketten-geschützten Peptids aus (b), um ein Peptid der Formel:
Ac-YTSLIHSLIEESQNQQEKNEQELLELDKWASLWNWF -NH$_2$ (SEQ ID NO:1) zu erhalten;

**5.** Das Verfahren nach Anspruch 1 oder 4, wobei das Seitenketten-geschützte Peptid der Formel Fmoc-EKNEQEL-LELDKWASLWNWF-NH$_2$ (SEQ ID NO:12) durch ein Verfahren hergestellt wird, umfassend:

(a) reagieren eines Seitenketten-geschützten Peptids der Formel:
Fmoc-DKWASLWNW-COOH (SEQ ID NO:17) mit HPheNH$_2$ um ein Seitenketten-geschütztes Peptid der Formel:
NH$_2$-DKWASLWNWF-NH$_2$ (SEQ ID NO:18), zu erhalten;
(b) entfernen der Schutzgruppen des Aminoterminus des in (a) hergestellten Peptids; und
(c) reagieren des in (b) hergestellten Peptids mit einem Seitenketten-geschützten Peptid der Formel:
Fmoc-EKNEQELLEL-COOH (SEQ ID NO:11), um ein Seitenketten-geschütztes Peptid der Formel:
Fmoc-EKNEQELLELDKWASLWNWF-NH$_2$ (SEQ ID NO:12) zu erhalten.

**6.** Das Verfahren nach Anspruch 3 oder 4, wobei das Seitenketten-geschützte Peptid der Formel Fmoc-EKNEQEL-LELDKWASLWNWF-NH$_2$ (SEQ ID NO:12)
nach einem Verfahren hergestellt wird, umfassend:

(a) reagieren eines Seitenketten-geschützten Peptids der Formel:

$$\text{Fmoc-LELDKWASLWNW-COOH (SEQ ID NO:15)}$$

mit HPheNH$_2$ um ein Seitenketten-geschütztes Peptid der Formel:

$$\text{Fmoc-LELDKWASLWNWF-NH}_2 \text{ (SEQ ID NO:16)}$$

zu erhalten;
(b) entfernen der Schutzgruppen des Aminoterminus des in (a) hergestellten Peptids; und
(c) reagieren des in (b) hergestellten Peptids mit einem Seitenketten-geschützten Peptid der Formel:
Fmoc-EKNEQEL-COOH (SEQ ID NO:10), um ein Seitenketten-geschütztes Peptid der Formel:

$$\text{Fmoc-EKNEQELLELDKWASLWNWF-NH}_2 \text{ (SEQ ID NO:12)}$$

zu erhalten.

**7.** Das Verfahren nach Anspruch 3 oder 4, worin das Seitenketten-geschützte Peptid der Formel Fmoc-EKNEQEL-LELDKWASLWNWF-NH$_2$ (SEQ ID NO:12) nach einem Verfahren hergestellt wird, umfassend:

(a) entfernen der Schutzgruppe des Aminoterminus des Peptids
NH$_2$-DKWASLWNWF-NH$_2$ (SEQ ID NO:18); und
(b) Reagieren des in (a) hergestellten Peptids mit einem Seitenketten-geschützten Peptid der Formel

$$\text{Fmoc-EKNEQELLEL-COOH (SEQ ID NO:11)},$$

um ein Seitenketten-geschütztes Peptid der Formel

$$\text{Fmoc-EKNEQELLELDKWASLWNWF-NH}_2 \text{ (SEQ ID NO:12)}$$

zu erhalten.

**8.** Das Verfahren nach Anspruch 3 oder 4, wobei das Seitenketten-geschützte Peptid der Formel Fmoc-EKNEQEL-LELDKWASLWNWF-NH$_2$ (SEQ ID NO:12) hergestellt wird nach einem Verfahren umfassend:

(a) reagieren eines Seitenketten-geschützten Peptids der Formel

Fmoc-EKNEQELLELDKWASLWNW-COOH (SEY ID NO:19)

mit HPheNH$_2$, um ein Seitenketten-geschütztes Peptid der Formel

Fmoc-EKNEQELLELDKWASLWNWF-NH$_2$ (SEQ ID NO:12)

zu erhalten.

**9.** Das Verfahren nach Anspruch 1 oder 3, wobei das Seitenketten-geschützte Peptid der Formel:

Fmoc-YTSLIHSLIEESQNQQ-COOH (SEQ ID NO:4)

hergestellt wird nach einem Verfahren, umfassend:

(a) entfernen der Schutzgruppe des Aminoterminus des Seitenketten-geschützten Peptids der Formel

Fmoc-IEESQNQQ-COOH (SEQ ID NO:7);

(b) reagieren des in (a) hergestellten Seitenketten-geschützten Peptids mit einem Seitenketten-geschützten Peptid der Formel Fmoc-YTSLIHSL-COOH (SEQ ID NO:2), um ein Seitenketten-geschütztes Peptid der Formel

Fmoc-YTSLIHSLIEESQNQQ-COOH (SEQ ID NO:4)

zu erhalten.

**10.** Das Verfahren nach Anspruch 2 oder 4, wobei das Seitenketten-geschützte Peptid der Formel

Fmoc-YTSLIHSLIEESQNQQ-COOH (SEQ ID NO:4)

hergestellt wird nach einem Verfahren umfassend:

(a) reagieren eines Seitenketten-geschützten Peptids der Formel

Fmoc-IEESQNQ-COOH (SEQ ID NO:6)

mit HGlnOPNB um ein Seitenketten-geschütztes Peptid der Formel

Fmoc-IEESQNQQ-COOH (SEQ ID NO:7)

zu erhalten;

(b) entfernen der Schutzgruppe des Aminoterminus des in (a) hergestellten Seitenketten-geschützten Peptids;

(c) reagieren des Seitenketten-geschützten Peptids, das in (b) hergestellt wurde, mit einem Seitenketten-geschützten Peptid der Formel

$$\text{Fmoc-YTSLIHSL-COOH (SEQ ID NO:2),}$$

um eine Seitenketten-geschütztes Peptid der Formel

$$\text{Fmoc-YTSLIHSLIEESQNQQ-COOH (SEQ ID NO:4)}$$

zu erhalten;

(d) entfernen der Schutzgruppen des Carboxylterminus des in (c) hergestellten Seitenketten-geschützten Peptids, um ein Seitenketten-geschütztes Peptid der Formel

$$\text{Fmoc-YTSLIHSLIEESQNQQ-COOH (SEQ ID NO:4)}$$

zu erhalten.

11. Das Verfahren nach Anspruch 1 oder 3, wobei das Seitenketten-geschützte Peptid der Formel

$$\text{Fmoc-YTSLIHSLIEESQNQQ-COOH (SEQ ID NO:4)}$$

hergestellt wird nach einem Verfahren umfassend:

(a) reagieren eines Seitenketten-geschützten Peptids der Formel

$$\text{Fmoc-YTSLIHSLIEESQNQ-COOH (SEQ ID NO:3)}$$

mit HGlnOPNB, um ein Seitenketten-geschütztes Peptid der Formel

$$\text{Fmoc-YTSLIHSLIEESQNQQ-COOH (SEQ ID NO:4)}$$

zu erhalten; und

(b) entfernen der Schutzgruppe des Carboxylterminus des in (a) hergestellten Peptids, um ein Seitenketten-geschütztes Peptid der Formel

$$\text{Fmoc-YTSLIHSLIEESQNQQ-COOH (SEQ ID NO:4)}$$

zu erhalten.

12. Verfahren zur Herstellung eines Peptids der Formel:
Ac-YTSLIHSLIEESQNQQEKNEQELLELDKWASLWNWF-NH$_2$ (SEQ ID NO:1) umfassend:

(a) entfernen der Schutzgruppe des Aminoterminus eines Seitenketten-geschützten Peptids der Formel

$$\text{Fmoc-QEKNEQELLELDKWASLWNWF-NH}_2 \text{ (SEQ ID NO:9);}$$

(b) reagieren des in (a) hergestellten Peptids mit einem Seitenketten-geschützten Peptid der Formel

Fmoc-YTSLIHSLIEESQNQ-COOH (SEQ ID NO:3)

um ein Seitenketten-geschütztes Peptid der Formel

Ac-YTSLIHSLIEESQNQQEKNEQELLELDKWASLWNWF
-NH$_2$ (SEQ ID NO:1) zu erhalten;
(c) umwandeln des Aminoterminus des in (b) hergestellten Peptids in eine Acetylmodifikation; und
(d) entfernen der Schutzgruppe der Seitenketten des Seitenketten-geschützten Peptids aus (c), um ein Peptid der Formel:
Ac-YTSLIHSLIEESQNQQEKNEQELLELDKWASLWNWF -NH$_2$ (SEQ ID NO:1) zu erhalten.

13. Das Verfahren nach Anspruch 12, wobei das Seitenketten-geschützte Peptid der Formel Fmoc-QEKNEQELLELD-KWASLWNWF-NH$_2$ (SEQ ID NO:9) hergestellt wird nach einem Verfahren umfassend:

   (a) reagieren eines Seitenketten-geschützten Peptids der Formel: Fmoc-QEKNEQELLELDKWASLWNW-NH$_2$ (SEQ ID NO:8) mit HPheNH$_2$, um ein Seitenketten-geschütztes Peptid der Formel Fmoc-QEKNEQELLELDK-WASLWNWF-NH$_2$ (SEQ ID NO:9) zu erhalten.

14. Verfahren zur Herstellung eines Peptids der Formel: Ac-YTSLIHSLIEESQNQQEKNEQELLELDKWASLWN-WF-NH$_2$ (SEQ ID NO:1) umfassend:

   (a) entfernen der Schutzgruppe des Aminoterminus eines Seitenketten-geschützten Peptids der Formel

Fmoc-NEQELLELDKWASLWNWF-NH$_2$ (SEQ ID NO:14);

   (b) reagieren des in (a) hergestellten Peptids mit einem Seitenketten-geschützten Peptid der Formel Fmoc-YTSLIHSLIEESQNQQEK-COOH (SEQ ID NO: 5), um ein Seitenketten-geschütztes Peptid der Formel Ac-YTSLIHSLIEESQNQQEKNEQELLELDKWASLWNWF -NH$_2$ (SEQ ID NO:1) zu erhalten;
   (c) umwandeln des Aminoterminus des in (b) hergestellten Peptids in eine Acetylmodifikation; und
   (d) entfernen der Schutzgruppe(n) der Seitenketten des Seitenketten-geschützten Peptids aus (c), um ein Peptid der Formel
   Ac-YTSLIHSLIEESQNQQEKNEQELLELDKWASLWNWF-NH$_2$ (SEQ ID NO:1) zu erhalten.

15. Das Verfahren nach Anspruch 14, wobei das Seitenketten-geschützte Peptid der Formel: Fmoc-NEQELLELDK-WASLWNWF-NH$_2$ (SEQ ID NO:14) hergestellt wird nach einem Verfahren umfassend:

   (a) reagieren eines Seitenketten-geschützten Peptids der Formel Fmoc-NEQELLELDKWASLWNW-NH$_2$ (SEQ ID NO:13) mit HPheNH$_2$, um ein Seitenketten-geschütztes Peptid der Formel Fmoc-NEQELLELDKWASLWN-WF-NH$_2$ (SEQ ID NO:14) zu erhalten.

16. Das Verfahren nach Anspruch 1, 2, 3, 4, 12 oder 14, wobei das Seitenketten-geschützte Peptid der Formel

Ac-YTSLIHSLIEESQNQQEKNEQELLELDKWASLWNWF-NH$_2$ (SEQ ID NO:1)

in einer Menge von ungefähr ein oder mehr Kilogramm hergestellt wird.

17. Das Verfahren nach Anspruch 1 oder 2, wobei das Seitenketten-geschützte Peptid der Formel

EP 1 071 442 B1

$$\text{Fmoc-EKNEQELLELDKWASLWNWF-NH}_2 \text{ (SEQ ID NO:12)}$$

in einer Menge von ungefähr ein oder mehr Kilogramm hergestellt wird.

18. Das Verfahren nach Anspruch 5, wobei das Seitenketten-geschützte Peptid der Formel

$$\text{Fmoc-EKNEQELLELDKWASLWNWF-NH}_2 \text{ (SEQ ID NO:12)}$$

in einer Menge von ungefähr ein oder mehr Kilogramm hergestellt wird.

19. Das Verfahren nach Anspruch 6, wobei das Seitenketten-geschützte Peptid der Formel

$$\text{Fmoc-EKNEQELLELDKWASLWNWF-NH}_2 \text{ (SEQ ID NO:12)}$$

in einer Menge von ungefähr ein oder mehr Kilogramm hergestellt wird.

20. Das Verfahren nach Anspruch 7, wobei das Seitenketten-geschützte Peptid der Formel

$$\text{Fmoc-EKNEQELLELDKWASLWNWF-NH}_2 \text{ (SEQ ID NO:12)}$$

in einer Menge von ungefähr ein oder mehr Kilogramm hergestellt wird.

21. Das Verfahren nach Anspruch 8, wobei das Seitenketten-geschützte Peptid der Formel

$$\text{Fmoc-EKNEQELLELDKWASLWNWF-NH}_2 \text{ (SEQ ID NO:12)}$$

in einer Menge von ungefähr ein oder mehr Kilogramm hergestellt wird.

22. Das Verfahren nach Anspruch 9, wobei das Seitenketten-geschützte Peptid der Formel

$$\text{Fmoc-YTSLIHSLIEESQNQQ-COOH (SEQ ID NO:4)}$$

in einer Menge von ungefähr ein oder mehr Kilogramm hergestellt wird.

23. Das Verfahren nach Anspruch 10, wobei das Seitenketten-geschützte Peptid der Formel

$$\text{Fmoc-YTSLIHSLIEESQNQQ-COOH (SEQ ID NO:4)}$$

in einer Menge von ungefähr ein oder mehr Kilogramm hergestellt wird.

24. Das Verfahren nach Anspruch 11, wobei das Seitenketten-geschützte Peptid der Formel

$$\text{Fmoc-YTSLIHSLIEESQNQQ-COOH (SEQ ID NO:4)}$$

in einer Menge von ungefähr ein oder mehr Kilogramm hergestellt wird.

25. Das Verfahren nach Anspruch 13, wobei das Seitenketten-geschützte Peptid der Formel

$$\text{Fmoc-QEKNEQELLELDKWASLWNWF-NH}_2 \text{ (SEQ ID NO:9)}$$

66

in einer Menge von ungefähr ein oder mehr Kilogramm hergestellt wird.

26. Das Verfahren nach Anspruch 15, wobei das Seitenketten-geschützte Peptid der Formel

Fmoc-NEQELLELDKWASLWNWF-NH$_2$ (SEQ ID NO:14)

in einer Menge von ungefähr ein oder mehr Kilogramm hergestellt wird.

27. Peptidfragmente umfassend einen Satz Fragmente ausgewählt aus der Gruppe bestehend aus:

(a)

YTSLIHSLIEESQNQQ (SEQ ID NO:4),

EKNEQELLELDKWASLWNWF (SEQ ID NO:12);

(b)

YTSLIHSLIEESQNQQ (SEQ ID NO:4),

EKNEQELLEL (SEQ ID NO:11),

DKWASLWNWF (SEQ ID NO:18);

(c)

YTSLIHSLIEESQNQQ (SEQ ID NO:4),

EKNEQELLEL (SEQ ID NO:11),

DKWASLWNW (SEQ ID NO:17);

(d)

YTSLIHSL (SEQ ID NO:2),

IEESQNQ (SEQ ID NO:6)

EKNEQELLELDKWASLWNWF (SEQ ID NO:12);

(e)

YTSLIHSL (SEQ ID NO:2),

IEESQNQ (SEQ ID NO:6),

EKNEQELLEL (SEQ ID NO:11),

DKWASLWNWF (SEQ ID NO:18);

(f)

YTSLIHSL (SEQ ID NO:2),

IEESQNQ (SEQ ID NO:6),

EKNEQELLEL (SEQ ID NO:11),

DKWASLWNW (SEQ ID NO:17);

(g)

YTSLIHSL (SEQ ID NO:2),

IEESQNQQ (SEQ ID NO:7),

EKNEQELLELDKWASLWNWF (SEQ ID NO:12);

(h)

YTSLIHSL (SEQ ID NO:2),

IEESQNQQ (SEQ ID NO:7),

EKNEQELLEL (SEQ ID NO:11),

DKWASLWNWF (SEQ ID NO:18);

(i)

YTSLIHSL (SEQ ID NO:2),

IEESQNQQ (SEQ ID NO:7),

EKNEQELLEL (SEQ ID NO:11),

DKWASLWNW (SEQ ID NO:17);

(j)

YTSLIHSLIEESQNQQ (SEQ ID NO:4),

EKNEQEL (SEQ ID NO:10),

LELDKWASLWNWF (SEQ ID NO:16);

(k)

YTSLIHSLIEESQNQQ (SEQ ID NO:4),

EKNEQEL (SEQ ID NO:10),

LELDKWASLWNW (SEQ ID NO:15);

(l)

YTSLIHSL (SEQ ID NO:2),

IEESQNQ (SEQ ID NO:6),

EKNEQEL (SEQ ID NO:10),

LELDKWASLWNWF (SEQ ID NO:16);

(m)

YTSLIHSL (SEQ ID NO:2),

IEESQNQ (SEQ ID NO:6),

EKNEQEL (SEQ ID NO:10),

LELDKWASLWNW (SEQ ID NO:15);

(n)

YTSLIHSL (SEQ ID NO:2),

IEESQNQQ (SEQ ID NO:7),

EKNEQEL (SEQ ID NO:10),

LELDKWASLWNWF (SEQ ID NO:16);

(o)

YTSLIHSL (SEQ ID NO:2),

IEESQNQQ (SEQ ID NO:7),

EKNEQEL (SEQ ID NO:10),

LELDKWASLWNW (SEQ ID NO:15);

(p)

YTSLIHSLIEESQNQ (SEQ ID NO:3),

QEKNEQELLELDKWASLWNW (SEQ ID NO:8),

(q)

YTSLIHSLIEESQNQ (SEQ ID NO:3),

QEKNEQELLELDKWASLWNWF (SEQ ID NO:9);

(r)

YTSLIHSLIEESQNQQEK (SEQ ID NO:5),

NEQELLELDKWASLWNWF (SEQ ID NO:14);

(s)

YTSLIHSLIEESQNQQEK (SEQ ID NO:5),

NEQELLELDKWASLWNW (SEQ ID NO:13);

und

(t)

YTSLIHSLIEESQNQQ (SEQ ID NO:4),

EKENEQELLELDKWASLWNW (SEQ ID NO:19).

**28.** Verwendung eines Peptidfragments zur Peptidsynthese gemäß einem beliebigen der Ansprüche 1 - 26, wobei das Peptidfragment ausgewählt wird aus der Gruppe bestehend aus:

YTSLIHSL (SEQ ID NO:2),

IEESQNQ (SEQ ID NO:6),

IEESQNQQ (SEQ ID NO:7),

QEKNEQELLELDKWASLWNW (SEQ ID NO:8),

EKNEQEL (SEQ ID NO:10),

EKNEQELLEL (SEQ ID NO:11),

NEQELLELDKWASLWNW (SEQ ID NO:13),

LELDKWASLWNW (SEQ ID NO:15),

LELDKWASLWNWF (SEQ ID NO:16),

DKWASLWNW (SEQ ID NO:17),

DKWASLWNWF (SEQ ID NO:18);

und

EKENEQELLELDKWASLWNW (SEQ ID NO:19).

**29.** Ein geschütztes Peptid ausgewählt aus der Gruppe bestehend aus:

Ac-YTSLIHSL-COOH (SEQ ID NO:2);

FMOC-YTSLIHSL-COOH (SEQ ID NO:2);

Ac-YTSLIHSLIEESQNQQ-OPNB (SEQ ID NO:4);

FMOC-YTSLIHSLIEESQNQQ-OPNB (SEQ ID NO:4);

Ac-YTSLIHSLIEESQNQQ-COOH (SEQ ID NO:4);

FMOC-YTSLIHSLIEESQNQQ-COOH (SEQ ID NO:4);

Ac-IEESQNQ-COOH (SEQ ID NO:6),

FMOC-IEESQNQ-COOH (SEQ ID NO:6),

$NH_2$-IEESQNQQ-OPNB (SEQ ID NO:7),

FMOC-IEESQNQQ-OPNB (SEQ ID NO:7),

Ac-EKNEQEL-COOH (SEQ ID NO:10),

FMOC-EKNEQEL-COOH (SEQ ID NO:10),

Ac-EKNEQELLEL-COOH (SEQ ID NO:11),

FMOC-EKNEQELLEL-COOH (SEQ ID NO:11),

$NH_2$-EKNEQELLELDKWASLWNWF-$NH_2$ (SEQ ID NO:12),

FMOC-EKNEQELLELDKWASLWNWF-$NH_2$ (SEQ ID NO:12),

Ac-LELDKWASLWNW-COOH (SEQ ID NO:15),

FMOC-LELDKWASLWNW-COOH (SEQ ID NO:15),

$NH_2$-LELDKWASLWNWF-$NH_2$ (SEQ ID NO:16),

FMOC-LELDKWASLWNWF-NH$_2$ (SEQ ID NO:16),

Ac-DKWASLWNW-COOH (SEQ ID NO:17),

FMOC-DKWASLWNW-COOH (SEQ ID NO:17),

NH$_2$-DKWASLWNWF-NH$_2$ (SEQ ID NO:18);

FMOC-DKWASLWNWF-NH$_2$ (SEQ ID NO:18);

und

FMOC-EKENEQELLELDKWASLWNW-COOH (SEQ ID NO:19).

**Revendications**

1. Procédé de synthèse d'un peptide répondant à la formule Ac-YTSLIHSLIEESQNQQEKNEQELLELD-KWASZWNWF-NH$_2$ (SEQ ID NO : 1), comprenant :

(a) la réaction d'un peptide ayant des chaînes latérales protégées de formule :
Fmoc-EKNEQELLEL-COOH (SEQ ID NO:11) avec un peptide ayant des chaînes latérales protégées de formule :

NH$_2$-DKWASLWNWF-NH$_2$ (SEQ ID NO : 18)

pour donner un peptide ayant des chaînes latérales protégées de formule :

Fmoc-EKNEQELLELDKWASLWNWF-NH$_2$ (SEQ ID NO : 12);

(b) la déprotection de l'extrémité amino-terminale du peptide produit en (a) ;
(c) la réaction du peptide produit en (b) avec un peptide ayant des chaînes latérales protégées de formule :

Fmoc-YTSLIHSLIEESQNQQ-COOH (SEQ ID NO : 4)

pour donner un peptide ayant des chaînes latérales protégées de formule :

YTSLIHSLIEESQNQQEKNEQELLELDKWASLWNWF-NH$_2$ (SEQ ID NO : 1) ;

(d) la modification de l'extrémité amino-terminale du peptide produit en (c) en une modification d'acétyle ; et
(e) la déprotection des chaînes latérales du peptide ayant des chaînes latérales protégées de (d) pour donner un peptide de formule :

`Ac-YTSLIHSLIEESQNQQEKNEQELLELDKWASLWNWF-NH₂` (SEQ ID NO : 1).

2. Procédé de synthèse d'un peptide répondant à la formule :
Ac-YTSLIHSLIEESQNQQEKNEQELLELDKWASLWNWF-NH$_2$ (SEQ ID NO : 1), comprenant :

(a) la réaction d'un peptide ayant des chaînes latérales protégées de formule :

`Fmoc-EKNEQELLEL-COOH (SEQ ID NO : 11)`

avec un peptide ayant des chaînes latérales protégées de formule :

`NH₂-DKWASLWNWF-NH₂ (SEQ ID NO : 18)`

pour donner un peptide ayant des chaînes latérales protégées de formule :

`Fmoc-EKNEQELLELDKWASLWNWF-NH₂ (SEQ ID NO : 12);`

(b) la déprotection de l'extrémité amino-terminale du peptide produit en (a) ;
(c) la réaction du peptide produit en (b) avec un peptide ayant des chaînes latérales protégées de formule :

`Ac-YTSLIHSLIEESQNQQ-COOH (SEQ ID NO : 4)`

pour donner un peptide ayant des chaînes latérales protégées de formule :
Ac-YTSLIHSLIEESQNQQEKNEQELLELDKWASZWNWF-NH$_2$ (SEQ ID NO : 1), et
(d) la déprotection des chaînes latérales du peptide ayant des chaînes latérales protégées de (c) pour donner un peptide de formule :

`Ac-YTSLIHSLIEESQNQQEKNEQELLELDKWASLWNWF-NH₂` (SEQ ID NO : 1).

3. Procédé de synthèse d'un peptide répondant à la formule :
Ac-YTSLIHSLIEESQNQQEKNEQELLELDKWASZWNWF-NH$_2$ (SEQ ID NO : 1), comprenant :

(a) la déprotection de l'extrémité amino-terminale d'un peptide ayant des chaînes latérales protégées de formule :

`Fmoc-EKNEQELLELDKWASLWNWF-NH₂ (SEQ ID NO : 12);`

(b) la réaction du peptide produit en (a) avec un peptide ayant des chaînes latérales protégées de formule :

`Fmoc-YTSLIHSLIEESQNQQ-COOH (SEQ ID NO : 4),`

pour donner un peptide ayant des chaînes latérales protégées de formule :

`Fmoc-YTSLIHSLIEESQNQQEKNEQELLELDKWASLWNWF-NH₂` `(SEQ ID NO : 1) ;`

(c) la déprotection de l'extrémité amino-terminale du peptide produit en (b) ;
(d) la modification de l'extrémité amino-terminale du peptide produit en (c) en une modification d'acétyle ; et
(e) la déprotection des chaînes latérales du peptide ayant des chaînes latérales protégées de (c) pour donner un peptide de formule :

`Ac-YTSLIHSLIEESQNQQEKNEQELLELDKWASLWNWF-NH₂` `(SEQ ID NO : 1).`

4.  Procédé de synthèse d'un peptide répondant à la formule :
    Ac-YTSLIHSLIEESQNQQEKNEQELLELDKWASZWNWF-NH$_2$ (SEQ ID NO : 1), comprenant :

    (a) la déprotection de l'extrémité amino-terminale d'un peptide ayant des chaînes latérales protégées de formule :

    `Fmoc- EKNEQELLELDKWASLWNWF-NH₂ (SEQ ID NO : 12);`

    (b) la réaction du peptide produit en (a) avec un peptide ayant des chaînes latérales protégées de formule :

    `Ac-YTSLIHSLIEESQNQQ-COOH (SEQ ID NO : 4),`

    pour donner un peptide ayant des chaînes latérales protégées de formule :

    `Ac-YTSLIHSLIEESQNQQEKNEQELLELDKWASLWNWF-NH₂` `(SEQ ID NO : 1)`

    (c) la déprotection des chaînes latérales du peptide ayant des chaînes latérales protégées de (b) pour donner un peptide de formule :

    `Ac-YTSLIHSLIEESQNQQEKNEQELLELDKWASLWNWF-NH₂` `(SEQ ID NO : 1).`

5.  Procédé selon la revendication 3 ou 4, dans lequel le peptide ayant des chaînes latérales protégées de formule :
    Fmoc- EKNEQELLELDKWASLWNWF-NH$_2$ (SEQ ID NO : 12) est synthétisé par un procédé comprenant :

    (a) la réaction d'un peptide ayant des chaînes latérales protégées de formule :

    `Fmoc-DKWASLWNW-COOH (SEQ ID NO : 17)`

    avec du HPHeNH$_2$ pour donner un peptide ayant des chaînes latérales protégées de formule :

    `Fmoc-DKWASLWNWF-NH₂ (SEQ ID NO : 18);`

(b) la déprotection de l'extrémité amino-terminale du peptide produit en (a) ; et

(c) la réaction du peptide produit en (b) avec un peptide ayant des chaînes latérales protégées de formule :

Fmoc-EKNEQELLEL-COOH (SEQ ID NO : 11)

pour donner le peptide ayant des chaînes latérales protégées de formule :

Fmoc-EKNEQELLELDKWASLWNWF-NH$_2$ (SEQ ID NO : 12).

6. Procédé selon la revendication 3 ou 4, dans lequel le peptide ayant des chaînes latérales protégées de formule : Fmoc-EKNEQELLELDKWASLWNWF-NH$_2$ (SEQ ID NO:12) est synthétisé par un procédé comprenant :

(a) la réaction d'un peptide ayant des chaînes latérales protégées de formule :

Fmoc-LELDKWASLWNW-COOH (SEQ ID NO : 15)

avec du HPHeNH$_2$ pour donner un peptide ayant des chaînes latérales protégées de formule :

Fmoc- LELDKWASLWNWF-NH$_2$ (SEQ ID NO : 16);

(b) la déprotection de l'extrémité amino-terminale du peptide produit en (a) ; et

(c) la réaction du peptide produit en (b) avec un peptide ayant des chaînes latérales protégées de formule :

Fmoc-EKNEQEL-COOH (SEQ ID NO : 10)

pour donner le peptide ayant des chaînes latérales protégées de formule :

Fmoc-EKNEQELLELDKWASLWNWF-NH$_2$ (SEQ ID NO : 12).

7. Procédé selon la revendication 3 ou 4, dans lequel le peptide ayant des chaînes latérales protégées de formule : Fmoc-EKNEQELLELDKWASLWNWF-NH$_2$ (SEQ ID NO:12) est synthétisé par un procédé comprenant :

(a) la déprotection de l'extrémité amino-terminale du peptide :
Fmoc-DKWASLWNWF-NH$_2$ (SEQ ID NO : 18); et
(b) la réaction du peptide produit en (a) avec un peptide ayant des chaînes latérales protégées de formule :

Fmoc-EKNEQELLEL-COOH (SEQ ID NO : 11)

pour donner le peptide ayant des chaînes latérales protégées de formule :

Fmoc-EKNEQELLELDKWASLWNWF-NH$_2$ (SEQ ID NO : 12).

8. Procédé selon la revendication 3 ou 4, dans lequel le peptide ayant des chaînes latérales protégées de formule : Fmoc-EKNEQELLELDKWASLWNWF-NH$_2$ (SEQ ID NO:12) est synthétisé par un procédé comprenant :

(a) la réaction d'un peptide ayant des chaînes latérales protégées de formule :

`Fmoc-EKNEQELLELDKWASLWNW-COOH (SEQ ID NO : 19)`

avec du HPHeNH$_2$ pour donner le peptide ayant des chaînes latérales protégées de formule :

`Fmoc- EKNEQELLELDKWASLWNWF-NH₂ (SEQ ID NO : 12).`

9.  Procédé selon la revendication 1 ou 3, dans lequel le peptide ayant des chaînes latérales protégées de formule : Fmoc-YTSLIHSLIEESQNQQ-COOH (SEQ ID NO : 4) est synthétisé par un procédé comprenant :

    (a) la déprotection de l'extrémité amino-terminale du peptide ayant des chaînes latérales protégées de formule :

    `Fmoc-IEESQNQQ-COOH (SEQ ID NO : 7);`

    (b) la réaction du peptide ayant des chaînes latérales protégées produit en (a) avec un peptide ayant des chaînes latérales protégées de formule :

    `Fmoc-YTSLIHSL-COOH (SEQ ID NO : 2)`

    pour donner le peptide ayant des chaînes latérales protégées de formule :

    `Fmoc-YTSLIHSLIEESQNQQ-COOH (SEQ ID NO : 4).`

10. Procédé selon la revendication 2 ou 4, dans lequel le peptide ayant des chaînes latérales protégées de formule : Ac-YTSLIHSLIEESQNQQ-COOH (SEQ ID NO : 4) est synthétisé par un procédé comprenant :

    (a) la réaction d'un peptide ayant des chaînes latérales protégées de formule :

    `Fmoc-IEESQNQ-COOH (SEQ ID NO : 6)`

    avec du HGlnOPNB pour donner un peptide ayant des chaînes latérales protégées de formule :

    `Fmoc-IEESQNQQ-OPNB (SEQ ID NO : 7);`

    (b) la déprotection de l'extrémité amino-terminale du peptide ayant des chaînes latérales protégées produit en (a) ;
    (c) la réaction du peptide ayant des chaînes latérales protégées produit en (b) avec un peptide ayant des chaînes latérales protégées de formule :

    `Ac-YTSLIHSL-COOH (SEQ ID NO : 2)`

    pour donner un peptide ayant des chaînes latérales protégées de formule :

    `Ac-YTSLIHSLIEESQNQQ-OPNB (SEQ ID NO : 4);`

(d) la déprotection de l'extrémité carboxy-terminale du peptide ayant des chaînes latérales protégées produit en (c) pour donner le peptide ayant des chaînes latérales protégées de formule :

    `Ac-YTSLIHSLIEESQNQQ-COOH (SEQ ID NO : 4).`

**11.** Procédé selon la revendication 1 ou 3, dans lequel le peptide ayant des chaînes latérales protégées de formule : Fmoc-YTSLIHSLIEESQNQQ-COOH (SEQ ID NO : 4) est synthétisé par un procédé comprenant :

(a) la réaction du peptide ayant des chaînes latérales protégées de formule :

    `Fmoc-YTSLIHSLIEESQNQ-COOH (SEQ ID NO : 3)`

avec du HGlnOPNB pour donner le peptide ayant des chaînes latérales protégées de formule : Fmoc-YTSLIHSLIEESQNQQ-COOH (SEQ ID NO : 4); et
(b) la déprotection de l'extrémité carboxy-terminale du peptide produit en (a) pour donner le peptide ayant des chaînes latérales protégées de formule :

    `Fmoc-YTSLIHSLIEESQNQQ-COOH (SEQ ID NO : 4).`

**12.** Procédé de synthèse d'un peptide de formule :
Ac-YTSLIHSLIEESQNQQEKNEQELLELDKWASZWNWF-NH$_2$ (SEQ ID NO : 1), comprenant :

(a) la déprotection de l'extrémité amino-términale d'un peptide ayant des chaînes latérales protégées de formule :

    `Fmoc-QEKNEQELLELDKWASLWNWF-NH₂ (SEQ ID NO : 9);`

(b) la réaction du peptide produit en (a) avec un peptide ayant des chaînes latérales protégées de formule :

    `Fmoc-YTSLIHSLIEESQNQ-COOH (SEQ ID NO : 3),`

pour donner un peptide ayant des chaînes latérales protégées de formule :

    `Fmoc-YTSLIHSLIEESQNQQEKNEQELLELDKWASLWNWF-NH₂ (SEQ ID`
    `NO : 1) ;`

(c) la modification de l'extrémité amino-terminale du peptide produit en (b) en une modification d'acétyle ; et
(d) la déprotection des chaînes latérales du peptide ayant des chaînes latérales protégées de (c) pour donner le peptide de formule :

    `Ac-YTSLIHSLIEESQNQQEKNEQELLELDKWASLWNWF-NH₂ (SEQ ID`
    `NO : 1).`

**13.** Procédé selon la revendication 12, dans lequel le peptide ayant des chaînes latérales protégées de formule : Fmoc-QEKNEQELLELDKWASLWNWF-NH$_2$ (SEQ ID NO : 9), est synthétisé par un procédé comprenant :

(a) la réaction d'un peptide ayant des chaînes latérales protégées de formule :

`Fmoc-QEKNEQELLELDKWASLWNW-NH`$_2$ `(SEQ ID NO : 8)`

avec du HPHeNH$_2$ pour donner le peptide ayant des chaînes latérales protégées de formule :

`Fmoc-QEKNEQELLELDKWASLWNWF-NH`$_2$ `(SEQ ID NO : 9).`

**14.** Procédé de synthèse d'un peptide de formule :
Ac-YTSLIHSLIEESQNQQEKNEQELLELDKWASZWNWF-NH$_2$ (SEQ ID NO : 1), comprenant :

(a) la déprotection de l'extrémité amino-terminale d'un peptide ayant des chaînes latérales protégées de formule :

`Fmoc-NEQELLELDKWASLWNWF-NH`$_2$ `(SEQ ID NO : 14);`

(b) la réaction du peptide produit en (a) avec un peptide ayant des chaînes latérales protégées de formule :

`Fmoc-YTSLIHSLIEESQNQQEK-COOH (SEQ ID NO : 5),`

pour donner un peptide ayant des chaînes latérales protégées de formule :

`Fmoc-YTSLIHSLIEESQNQQEKNEQELLELDKWASLWNWF-NH`$_2$ `(SEQ ID NO : 1) ;`

(c) la modification de l'extrémité amino-terminale du peptide produit en (b) en une modification d'acétyle ; et
(d) la déprotection des chaînes latérales du peptide ayant des chaînes latérales protégées de (c) pour donner le peptide de formule :

`Ac-YTSLIHSLIEESQNQQEKNEQELLELDKWASLWNWF-NH`$_2$ `(SEQ ID NO : 1).`

**15.** Procédé selon la revendication 14, dans lequel le peptide ayant des chaînes latérales protégées de formule : Fmoc-NEQELLELDKWASLWNWF-NH$_2$ (SEQ ID NO:14); est synthétisé par un procédé comprenant :

(a) la réaction d'un peptide ayant des chaînes latérales protégées de formule :

`Fmoc-NEQELLELDKWASLWNW-NH`$_2$ `(SEQ ID NO : 13)`

avec du HPHeNH$_2$ pour donner le peptide ayant des chaînes latérales protégées de formule :

`Fmoc-NEQELLELDKWASLWNWF-NH`$_2$ `(SEQ ID NO : 14).`

**16.** Procédé selon la revendication 1, 2, 3, 4, 12 ou 14, dans lequel le peptide ayant des chaînes latérales protégées de formule : Ac-YTSLIHSLIEESQNQQEKNEQELLELDKWASLWNWF-NH$_2$ (SEQ ID NO : 1) est produit en une quantité d'environ un kilogramme ou plus.

**17.** Procédé selon la revendication 1 ou 2, dans lequel le peptide ayant des chaînes latérales protégées de formule : Fmoc- EKNEQELLELDKWASLWNWF-NH$_2$ (SEQ ID NO : 12) est produit en une quantité d'environ un kilogramme ou plus.

**18.** Procédé selon la revendication 5, dans lequel le peptide ayant des chaînes latérales protégées de formule : Fmoc-EKNEQELLELDKWASLWNWF-NH$_2$ (SEQ ID NO : 12) est produit en une quantité d'environ un kilogramme ou plus.

**19.** Procédé selon la revendication 6, dans lequel le peptide ayant des chaînes latérales protégées de formule : Fmoc-EKNEQELLELDKWASLWNWF-NH$_2$ (SEQ ID NO : 12) est produit en une quantité d'environ un kilogramme ou plus.

**20.** Procédé selon la revendication 7, dans lequel le peptide ayant des chaînes latérales protégées de formule : Fmoc-EKNEQELLELDKWASLWNWF-NH$_2$ (SEQ ID NO : 12) est produit en une quantité d'environ un kilogramme ou plus.

**21.** Procédé selon la revendication 8, dans lequel le peptide ayant des chaînes latérales protégées de formule : Fmoc-EKNEQELLELDKWASLWNWF-NH$_2$ (SEQ ID NO : 12) est produit en une quantité d'environ un kilogramme ou plus.

**22.** Procédé selon la revendication 9, dans lequel le peptide ayant des chaînes latérales protégées de formule : Fmoc-YTSLIHSLIEESQNQQ-COOH (SEQ ID NO : 4) est produit en une quantité d'environ un kilogramme ou plus.

**23.** Procédé selon la revendication 10, dans lequel le peptide ayant des chaînes latérales protégées de formule : Ac-YTSLIHSLIEESQNQQ-COOH (SEQ ID NO : 4) est produit en une quantité d'environ un kilogramme ou plus.

**24.** Procédé selon la revendication 11, dans lequel le peptide ayant des chaînes latérales protégées de formule : Fmoc-YTSLIHSLIEESQNQQ-COOH (SEQ ID NO : 4) est produit en une quantité d'environ un kilogramme ou plus.

**25.** Procédé selon la revendication 13, dans lequel le peptide ayant des chaînes latérales protégées de formule : Fmoc- Fmoc-QEKNEQELLELDKWASLWNMF-NH$_2$ (SEQ ID NO : 9) est produit en une quantité d'environ un kilogramme ou plus.

**26.** Procédé selon la revendication 15, dans lequel le peptide ayant des chaînes latérales protégées de formule : Fmoc- Fmoc-NEQELLELDKWASLWNWF-NH$_2$ (SEQ ID NO : 14) est produit en une quantité d'environ un kilogramme ou plus.

**27.** Ensemble de fragments peptidiques comprenant un ensemble choisi dans le groupe constitué de :

(a)

        YTSLIHSLIEESQNQQ (SEQ ID NO : 4),

        EKNEQELLELDKWASLWNWF (SEQ ID NO : 12);

(b)

        YTSLIHSLIEESQNQQ (SEQ ID NO : 4),

        EKNEQELLEL (SEQ ID NO : 11),

        DKWASLWNWF (SEQ ID NO : 18);

(c)

YTSLIHSLIEESQNQQ (SEQ ID NO : 4),

EKNEQELLEL (SEQ ID NO : 11),

DKWASLWNW (SEQ ID NO : 17);

(d)

YTSLIHSL (SEQ ID NO : 2),

IEESQNQ (SEQ ID NO : 6);

EKNEQELLELDKWASLWNWF (SEQ ID NO : 12);

(e)

YTSLIHSL (SEQ ID NO : 2),

IEESQNQ (SEQ ID NO : 6),

EKNEQELLEL (SEQ ID NO : 11);

DKWASLWNWF (SEQ ID NO : 18);

(f)

YTSLIHSL (SEQ ID NO : 2),

IEESQNQ (SEQ ID NO : 6),

EKNEQELLEL (SEQ ID NO : 11);

DKWASLWNW (SEQ ID NO : 17);

(g)

YTSLIHSL (SEQ ID NO : 2),

IEESQNQQ (SEQ ID NO : 7),

EKNEQELLELDKWASLWNWF (SEQ ID NO : 12);

(h)

YTSLIHSL (SEQ ID NO :2),

IEESQNQQ (SEQ ID NO : 7),

EKNEQELLEL (SEQ ID NO : 11),

DKWASLWNWF (SEQ ID NO : 18);

(i)

YTSLIHSL (SEQ ID NO : 2),

IEESQNQQ (SEQ ID NO : 7),

EKNEQELLEL (SEQ ID NO : 11),

DKWASLWNW (SEQ ID NO : 17);

(j)

YTSLIHSLIEESQNQQ (SEQ ID NO : 4),

EKNEQEL (SEQ ID NO : 10),

LELDKWASLWNWF (SEQ ID NO : 16);

(k)

YTSLIHSLIEESQNQQ (SEQ ID NO : 4),

EKNEQEL (SEQ ID NO : 10),

LELDKWASLWNW (SEQ ID NO : 15);

(1)

YTSLIHSL (SEQ ID NO : 2),

IEESQNQ (SEQ ID NO : 6),

EKNEQEL (SEQ ID NO : 10),

LELDKWASLWNWF (SEQ ID NO : 16);

(m)

YTSLIHSL (SEQ ID NO : 2),

IEESQNQ (SEQ ID NO : 6),

EKNEQEL (SEQ ID NO : 10),

LELDKWASLWNW (SEQ ID NO : 15);

(n)

YTSLIHSL (SEQ ID NO : 2),

IEESQNQQ (SEQ ID NO : 7),

EKNEQEL (SEQ ID NO : 10),

LELDKWASLWNWF (SEQ ID NO : 16);

(o)

YTSLIHSL (SEQ ID NO :2),

IEESQNQQ (SEQ ID NO : 7),

EKNEQEL (SEQ ID NO : 10),

LELDKWASLWNW (SEQ ID NO : 15);

(p)

YTSLIHSLIEESQNQ (SEQ ID NO : 3),

QEKNEQELLELDKWASLWNW (SEQ ID NO : 8);

(q)

YTSLIHSLIEESQNQ (SEQ ID NO : 3),

QEKNEQELLELDKWASLWNWF (SEQ ID NO : 9);

(r)

YTSLIHSLIEESQNQQEK (SEQ ID NO : 5),

NEQELLELDKWALWNWF (SEQ ID NO : 14);

(s)

YTSLIHSLIEESQNQQEK (SEQ ID NO : 5),

NEQELLELDKWASLWNW (SEQ ID NO : 13);

et
(t)

YTSLIHSLIEESQNQQ (SEQ ID NO : 4),

EKNEQELLELDKWASLWNW (SEQ ID NO : 19).

**28.** Utilisation d'un fragment peptidique pour une synthèse de peptide selon l'une quelconque des revendications 1 à 26, dans laquelle le fragment peptidique est choisi dans le groupe constitué par :

YTSLIHSL (SEQ ID NO : 2);

IEESQNQ (SEQ ID NO : 6);

IEESQNQQ (SEQ ID NO : 7);

QEKNEQELLELDKWASLWNW (SEQ ID NO : 8);

EKNEQEL (SEQ ID NO : 10);

EKNEQELLEL (SEQ ID NO : 11);

NEQELLELDKWASLWNW (SEQ ID NO : 13);

LELDKWASLWNW (SEQ ID NO : 15);

LELDKWASLWNWF (SEQ ID NO : 16);

DKWASLWNW (SEQ ID NO : 17);

DKWASLWNWF (SEQ ID NO : 18);

et

EKNEQELLELDKWASLWNW (SEQ ID NO : 19).

**29.** Peptide protégé choisi dans le groupe constitué par :

Ac-YTSLIHSL-COOH (SEQ ID NO : 2);

FMOC-YTSLIHSL-COOH (SEQ ID NO : 2);

Ac-YTSLIHSLIEESQNQQ-OPNB (SEQ ID NO : 4);

FMOC-YTSLIHSLIEESQNQQ-OPNB (SEQ ID NO : 4);

Ac-YTSLIHSLIEESQNQQ-COOH (SEQ ID NO : 4);

FMOC-YTSLIHSLIEESQNQQ-COOH (SEQ ID NO : 4);

Ac-IEESQNQ-COOH (SEQ ID NO : 6);

FMOC-IEESQNQ-COOH (SEQ ID NO : 6);

$NH_2$-IEESQNQQ-OPNB (SEQ ID NO : 7);

FMOC-IEESQNQQ-OPNB (SEQ ID NO : 7);

Ac-EKNEQEL-COOH (SEQ ID NO : 10);

FMOC-EKNEQEL-COOH (SEQ ID NO : 10);

Ac-EKNEQELLEL-COOH (SEQ ID NO : 11);

FMOC-EKNEQELLEL-COOH (SEQ ID NO : 11);

$NH_2$-EKNEQELLELDKWASLWNWF-$NH_2$ (SEQ ID NO : 12);

FMOC-EKNEQELLELDKWASLWNWF-$NH_2$ (SEQ ID NO : 12);

Ac-LEKDKWASLWNW-COOH (SEQ ID NO : 15);

FMOC-LEKDKWASLWNW-COOH (SEQ ID NO : 15);

$NH_2$-LEKDKWASLWNWF-$NH_2$ (SEQ ID NO : 16);

FMOC-LEKDKWASLWNWF-$NH_2$ (SEQ ID NO : 16);

Ac-DKWASLWNW-COOH (SEQ ID NO : 17);

FMOC-DKWASLWNW-COOH (SEQ ID NO : 17);

$NH_2$-DKWASLWNWF-$NH_2$ (SEQ ID NO : 18);

FMOC-DKWASLWNWF-$NH_2$ (SEQ ID NO : 18);

et

FMOC-EKNEQELLELDKWASLWNW-COOH (SEQ ID NO : 19).

FIG.1

EP 1 071 442 B1

Fmoc-AA1-8-OH
(SPPS)

FmocAA9-15-OH
(SPPS)

FmocAA17-26-OH
(SPPS)

FmocAA27-35-OH
(SPPS)

HGlnOPNB

HPheNH$_2$

FmocAA9-16-OPNB

FmocAA27-36-NH$_2$

H-AA9-16-OPNB

H-AA27-36-NH$_2$

Fmoc-AA1-16-OPNB

FmocAA17-36-NH$_2$

H-AA1-16-OPNB

H-AA17-36-NH$_2$

Ac-AA1-16-OPNB

Ac-AA1-16-OH

AcAA1-36NH$_2$

FIG.2

FIG.3

EP 1 071 442 B1

Fmoc–AA1–16–OH
(SPPS)

FmocAA17–26–OH
(SPPS)

FmocAA27–35–OH
(SPPS)

$\downarrow$ HPheNH$_2$

FmocAA27–36–NH$_2$

$\downarrow$

H–AA27–36–NH$_2$

FmocAA17–36–NH$_2$

$\downarrow$

H–AA17–36–NH$_2$

Fmoc–AA1–36–NH$_2$

$\downarrow$

H–AA1–36–NH$_2$

$\downarrow$

Ac–AA1–36–NH$_2$

FIG.4

Ac−AA1−16−OH
(SPPS)

FmocAA17−35−OH
(SPPS)

HPheNH$_2$

FmocAA17−36−NH$_2$

H−AA17−36−NH$_2$

AcAA1−36−NH$_2$

# FIG.5